# EUROPEAN PATENT SPECIFICATION

(11) **EP 2 236 629 B1**
(45) Date of publication and mention of the grant of the patent: **18.01.2012**
(21) Application number: 10160953.5
(22) Date of filing: 15.05.2000
(51) Int. Cl.: C12Q 1/68

(54) **Nucleic acid probe-based diagnostic assays for prokaryotic and eukaryotic organisms**
Diagnosetests auf Basis einer Nukleinsäuresonde für prokaryotische und eukaryotische Organismen
Réseaux de diagnostic à base de sondes d'acide nucléique pour organismes procaryotes et eucaryotes

(30) Priority: 14.05.1999 WO PCT/IE99/00043
(43) Date of publication of application: 06.10.2010
(62) Divisional of application: 00925552.2
(73) Proprietor: Enterprise Ireland (trading as BioResearch Ireland), Dublin 9 (IE); National University of Ireland, Galway, Galway (IE)
(72) Inventor: Barry, Thomas Gerard, County Galway (IE); Smith, Terence James, Galway (IE)
(74) Representative: Gates, Marie Christina Esther

(56) References cited:
- EP-A1- 0 915 170
- WO-A1-98/10101
- WILLIAMS K P ET AL: "The tmRNA Website." NUCLEIC ACIDS RESEARCH 1 JAN 1998 LNKD- PUBMED:9399824, vol. 26, no. 1, 1 January 1998 (1998-01-01), pages 163-165, XP002594155 ISSN: 0305-1048

### SEQUENCE LISTING

<110> ENTERPRISE IRELAND (t/a BIORESEARCH IRELAND) et al
<120> Nucleic acid probe-based diagnostic assays for prokaryotic and eukaryotic organisms.
<130> P00-37-PCT
<140>
   <141>
<150> PCT/IE 99/00043
   <151> 1999-05-14
<160> 228
<170> PatentIn Ver. 2.0
<210> 1
   <211> 366
   <212> DNA
   <213> Actinobacillus actinomycetemcomitans
<400> 1
   ggggctgatt ctggattcga cgggattagc gaagcccgaa gtgcacgtcg aggtgcggta 60
   ggcctcgtaa ataaaccgca aaaaaatagt cgcaaacgac gaacaatacg ctttagcagc 120
   ttaataacct gcctttagcc ttcgctcccc agcttccgct cgtaagacgg ggataaagcg 180
   gagtcaaacc aaaacgagat cgtgtggaag ccaccgtttg aggatcgaag cattaaatta 240
   aatcaaagta gcttaattgt cgcgtgtccg tcagcaggat taagtgaatt taaagaccgg 300
   actaaacgtg tagtgctaac ggcagaggaa tttcggacgg gggttcaact ccccccagct 360
   ccacca                                                            366
<210> 2
   <211> 366
   <212> RNA
   <213> Actinobacillus actinomycetemcomitans
<400> 2
   ggggcugauu cuggauucga cgggauuagc gaagcccgaa gugcacgucg aggugcggua 60
   ggccucguaa auaaaccgca aaaaaauagu cgcaaacgac gaacaauacg cuuuagcagc 120
   uuaauaaccu gccuuuagcc uucgcucccc agcuuccgcu cguaagacgg ggauaaagcg 180
   gagucaaacc aaaacgagau cguguggaag ccaccguuug aggaucgaag cauuaaauua 240
   aaucaaagua gcuuaauugu cgcguguccg ucagcaggau uaagugaauu uaaagaccgg 300
   acuaaacgug uagugcuaac ggcagaggaa uuucggacgg ggguucaacu ccccccagcu 360
   ccacca 366
<210> 3
   <211> 315
   <212> DNA
   <213> Aeromonas salmonicida
<400> 3
   aagattcacg aaacccaagg tgcatgccga ggtgcggtag gcctcgttaa caaaccgcaa 60
   aaaaatagtc gcaaacgacg aaaactacgc actagcagct taataacctg catagagccc 120
   ttctacccta gcttgcctgt gtcctaggga atcggaaggt catccttcac aggatcgtgt 180
   ggaagtcctg ctcggggcgg aagcattaaa accaatcgag ctagtcaatt cgtggcgtgt 240
   ctctccgcag cgggttggcg aatgtaaaga gtgactaagc atgtagtacc gaggatgtag 300
   taattttgga cgggg 315
<210> 4
   <211> 315
   <212> RNA
   <213> Aeromonas salmonicida
<400> 4
   aagauucacg aaacccaagg ugcaugccga ggugcgguag gccucguuaa caaaccgcaa 60
   aaaaauaguc gcaaacgacg aaaacuacgc acuagcagcu uaauaaccug cauagagccc 120
   uucuacccua gcuugccugu guccuaggga aucggaaggu cauccuucac aggaucgugu 180
   ggaaguccug cucggggcgg aagcauuaaa accaaucgag cuagucaauu cguggcgugu 240
   cucuccgcag cggguuggcg aauguaaaga gugacuaagc auguaguacc gaggauguag 300
   uaauuuugga cgggg 315
<210> 5
   <211> 349
   <212> DNA
   <213> Alcaligenes eutrophus
<400> 5
   tgggccgacc tggtttcgac gtggttacaa agcagtgagg cataccgagg acccgtcacc 60
   tcgttaatca atggaatgca ataactgcta acgacgaacg ttacgcactc gcttaattgc 120
   ggccgtcctc gcactggctc gctgacgggc tagggtcgca agaccacgcg aggtatttac 180
   gtcagataag ctccggaagg gtcacgaagc cggggacgaa aacctagtga ctcgccgtcg 240
   tagagcgtgt tcgtccgatg cgccggttaa atcaaatgac agaactaagt atgtagaact 300
   ctctgtggag ggcttacgga cgcgggttcg attcccgccg gctccacca 349
<210> 6
   <211>349
   <212> RNA
   <213> Alcaligenes eutrophus
<400> 6
   ugggccgacc ugguuucgac gugguuacaa agcagugagg cauaccgagg acccgucacc 60
   ucguuaauca auggaaugca auaacugcua acgacgaacg uuacgcacuc gcuuaauugc 120
   ggccguccuc gcacuggcuc gcugacgggc uagggucgca agaccacgcg agguauuuac 180
   gucagauaag cuccggaagg gucacgaagc cggggacgaa aaccuaguga cucgccgucg 240
   uagagcgugu ucguccgaug cgccgguuaa aucaaaugac agaacuaagu auguagaacu 300
   cucuguggag ggcuuacgga cgcggguucg auucccgccg gcuccacca 349
<210> 7
   <211> 347
   <212> DNA
   <213> Aquifex aeolicus
<400> 7
   gggggcggaa aggattcgac ggggacaggc ggtccccgag gagcaggccg ggtggctccc 60
   gtaacagccg ctaaaacagc tcccgaagct gaactcgctc tcgctgccta attaaacggc 120
   agcgcgtccc cggtaggttt gcgggtggcc taccggaggg cgtcagagac acccgctcgg 180
   gctactcggt cgcacggggc tgagtagctg acacctaacc cgtgctaccc tcggggagct 240
   tgcccgtggg cgacccgagg ggaaatcctg aacacgggct aagcctgtag agcctcggat 300
   gtggccgccg tcctcggacg cgggttcgat tcccgccgcc tccacca 347
<210> 8
   <211> 347
   <212> RNA
   <213> Aquifex aeolicus
<400> 8
   gggggcggaa aggauucgac ggggacaggc gguccccgag gagcaggccg gguggcuccc 60
   guaacagccg cuaaaacagc ucccgaagcu gaacucgcuc ucgcugccua auuaaacggc 120
   agcgcguccc cgguagguuu gcggguggcc uaccggaggg cgucagagac acccgcucgg 180
   gcuacucggu cgcacggggc ugaguagcug acaccuaacc cgugcuaccc ucggggagcu 240
   ugcccguggg cgacccgagg ggaaauccug aacacgggcu aagccuguag agccucggau 300
   guggccgccg uccucggacg cggguucgau ucccgccgcc uccacca 347
<210> 9
   <211> 316
   <212> DNA
   <213> Bacillus megaterium
<400> 9
   agggtagttc gagcttaggt tgcgagtcga ggagatggcc tcgttaaaac atcaacgcca 60
   ataataactg gcaaatctaa caataacttc gctttagctg cataatagta gcttagcgtt 120
   cctccctcca tcgcccatgt ggtagggtaa gggactcact ttaagtgggc tacgccggag 180
   ttcgccgtct gaggacgaag gaagagaata atcagactag cgactgggac gcctgttggt 240
   aggcagaaca gctcgcgaat gatcaatatg ccaactacac tcgtagacgc ttaagtggcc 300
   atatttctgg acgtgg                                                 316
<210> 10
   <211> 316
   <212> RNA
   <213> Bacillus megaterium
<400> 10
   aggguaguuc gagcuuaggu ugcgagucga ggagauggcc ucguuaaaac aucaacgcca 60
   auaauaacug gcaaaucuaa caauaacuuc gcuuuagcug cauaauagua gcuuagcguu 120
   ccucccucca ucgcccaugu gguaggguaa gggacucacu uuaagugggc uacgccggag 180
   uucgccgucu gaggacgaag gaagagaaua aucagacuag cgacugggac gccuguuggu 240
   aggcagaaca gcucgcgaau gaucaauaug ccaacuacac ucguagacgc uuaaguggcc 300
   auauuucugg acgugg                                                 316
<210> 11
   <211> 363
   <212> DNA
   <213> Bacillus subtilis
<400> 11
   ggggacgtta cggattcgac agggatggat cgagcttgag ctgcgagccg agaggcgatc 60
   tcgtaaacac gcacttaaat ataactggca aaactaacag ttttaaccaa aacgtagcat 120
   tagctgccta ataagcgcag cgagctcttc ctgacattgc ctatgtgtct gtgaagagca 180
   catccaagta ggctacgctt gcgttcccgt ctgagaacgt aagaagagat gaacagacta 240
   gctctcggaa ggcccgcccg caggcaagaa gatgagtgaa accataaata tgcaggctac 300
   gctcgtagac gcttaagtaa tcgatgtttc tggacgtggg ttcgactccc accgtctcca 360
   cca                                                               363
<210> 12
   <211> 363
   <212> RNA
   <213> Bacillus subtilis
<400> 12
   ggggacguua cggauucgac agggauggau cgagcuugag cugcgagccg agaggcgauc 60
   ucguaaacac gcacuuaaau auaacuggca aaacuaacag uuuuaaccaa aacguagcau 120
   uagcugccua auaagcgcag cgagcucuuc cugacauugc cuaugugucu gugaagagca 180
   cauccaagua ggcuacgcuu gcguucccgu cugagaacgu aagaagagau gaacagacua 240
   gcucucggaa ggcccgcccg caggcaagaa gaugagugaa accauaaaua ugcaggcuac 300
   gcucguagac gcuuaaguaa ucgauguuuc uggacguggg uucgacuccc accgucucca 360
   cca                                                               363
<210> 13
   <211> 387
   <212> DNA
   <213> Bordetella pertussis
<400> 13
   ggggccgatc cggattcgac gtgggtcatg aaacagctca gggcatgccg agcaccagta 60
   agctcgttaa tccactggaa cactacaaac gccaacgacg agcgtctcgc tctcgccgct 120
   taagcggtga gccgctgcac tgatctgtcc ttgggtcagg cgggggaagg caacttcaca 180
   gggggcaacc ccgaaccgca gcagcgacat tcacaaggaa tcggccaccg ctggggtcac 240
   acggcgttgg tttaaattac gtgaatcgcc ctggtccggc ccgtcgatcg gctaagtcca 300
   gggttaaatc caaatagatc gactaagcat gtagaactgg ttgcggaggg cttgcggacg 360
   ggggttcaat tccccccggc tccacca                                     387
<210> 14
   <211> 387
   <212> RNA
   <213> Bordetella pertussis
<400> 14
   ggggccgauc cggauucgac gugggucaug aaacagcuca gggcaugccg agcaccagua 60
   agcucguuaa uccacuggaa cacuacaaac gccaacgacg agcgucucgc ucucgccgcu 120
   uaagcgguga gccgcugcac ugaucugucc uugggucagg cgggggaagg caacuucaca 180
   gggggcaacc ccgaaccgca gcagcgacau ucacaaggaa ucggccaccg cuggggucac 240
   acggcguugg uuuaaauuac gugaaucgcc cugguccggc ccgucgaucg gcuaagucca 300
   ggguuaaauc caaauagauc gacuaagcau guagaacugg uugcggaggg cuugcggacg 360
   gggguucaau uccccccggc uccacca                                     387
<210> 15
   <211> 362
   <212> DNA
   <213> Borrelia burgdorferi
<400> 15
   ggggatgttt tggatttgac tgaaaatgtt aatattgtaa gttgcaggca gagggaatct 60
   cttaaaactt ctaaaataaa tgcaaaaaat aataacttta caagctcaaa tcttgtaatg 120
   gctgcttaag ttagcagagg gttttgttga atttggcttt gaggttcact tatactcttt 180
   tcgacatcaa agcttgctta aaaatgtttt caagttgatt tttagggact tttatacttg 240
   agagcaattt ggtggtttgc tagtatttcc aaaccatatt gcttaataaa atactagata 300
   agcttgtaga agcttatagt attattttta ggacgcgggt tcaattcccg ccatctccac 360
   ca                                                                362
<210> 16
   <211> 362
   <212> RNA
   <213> Borrelia burgdorferi
<400> 16
   ggggauguuu uggauuugac ugaaaauguu aauauuguaa guugcaggca gagggaaucu 60
   cuuaaaacuu cuaaaauaaa ugcaaaaaau aauaacuuua caagcucaaa ucuuguaaug 120
   gcugcuuaag uuagcagagg guuuuguuga auuuggcuuu gagguucacu uauacucuuu 180
   ucgacaucaa agcuugcuua aaaauguuuu caaguugauu uuuagggacu uuuauacuug 240
   agagcaauuu ggugguuugc uaguauuucc aaaccauauu gcuuaauaaa auacuagaua 300
   agcuuguaga agcuuauagu auuauuuuua ggacgcgggu ucaauucccg ccaucuccac 360
   ca                                                                362
<210> 17
   <211> 359
   <212> DNA
   <213> Campylobacter jejuni
<400> 17
   gggagcgact tggcttcgac aggagtaagt ctgcttagat ggcatgtcgc tttgggcaaa 60
   gcgtaaaaag cccaaataaa attaaacgca aacaacgtta aattcgctcc tgcttacgct 120
   aaagctgcgt aagttcagtt gagcctgaaa tttaagtcat actatctagc ttaattttcg 180
   gtcatttttg atagtgtagc cttgcgtttg acaagcgttg aggtgaaata aagtcttagc 240
   cttgcttttg agttttggaa gatgagcgaa gtagggtgaa gtagtcatct ttgctaagca 300
   tgtagaggtc tttgtgggat tatttttgga caggggttcg attcccctcg cttccacca  359
<210> 18
   <211> 359
   <212> RNA
   <213> Campylobacter jejuni
<400> 18
   gggagcgacu uggcuucgac aggaguaagu cugcuuagau ggcaugucgc uuugggcaaa 60
   gcguaaaaag cccaaauaaa auuaaacgca aacaacguua aauucgcucc ugcuuacgcu 120
   aaagcugcgu aaguucaguu gagccugaaa uuuaagucau acuaucuagc uuaauuuucg 180
   gucauuuuug auaguguagc cuugcguuug acaagcguug aggugaaaua aagucuuagc 240
   cuugcuuuug aguuuuggaa gaugagcgaa guagggugaa guagucaucu uugcuaagca 300
   uguagagguc uuugugggau uauuuuugga cagggguucg auuccccucg cuuccacca  359
<210> 19
   <211> 420
   <212> DNA
   <213> Chlamydia trachomatis (D/UW-3/CX)
<400> 19
   gggggtgtaa aggtttcgac ttagaaatga agcgttaatt gcatgcggag ggcgttggct 60
   ggcctcctaa aaagccgaca aaacaataaa tgccgaacct aaggctgaat gcgaaattat 120
   cagcttcgct gatctcgaag atctaagagt agctgcttaa ttagcaaagt tgttacctaa 180
   atacgggtga cccggtgttc gcgagctcca ccagaggttt tcgaaacacc gtcatgtatc 240
   tggttagaac ttaggtcctt taattctcga ggaaatgagt ttgaaattta atgagagtcg 300
   ttagtctcta taggggtttc tagctgagga gacataacgt atagtaccta ggaactaagc 360
   atgtagaggt tagcggggag tttactaagg acgagagttc gactctctcc acctccacca 420
<210> 20
   <211> 420
   <212> RNA
   <213> Chlamydia trachomatis (D/UW-3/CX)
<400> 20
   ggggguguaa agguuucgac uuagaaauga agcguuaauu gcaugcggag ggcguuggcu 60
   ggccuccuaa aaagccgaca aaacaauaaa ugccgaaccu aaggcugaau gcgaaauuau 120
   cagcuucgcu gaucucgaag aucuaagagu agcugcuuaa uuagcaaagu uguuaccuaa 180
   auacggguga cccgguguuc gcgagcucca ccagagguuu ucgaaacacc gucauguauc 240
   ugguuagaac uuagguccuu uaauucucga ggaaaugagu uugaaauuua augagagucg 300
   uuagucucua uagggguuuc uagcugagga gacauaacgu auaguaccua ggaacuaagc 360
   auguagaggu uagcggggag uuuacuaagg acgagaguuc gacucucucc accuccacca 420
<210> 21
   <211> 421
   <212> DNA
   <213> Chlamydia trachomatis (mouse pneumonitis)
<400> 21
   gggggtgtaa aggtttcgac ttagaaatga agcgttaatt gcatgcggag ggcgttggct 60
   ggcctcctaa aaagccgaca aaacaataaa tgccgaacct aaggctgaat gcgaaattat 120
   cagcttcgct gatcttaatg atctaagagt tgctgcttaa ttagcaaagt tgttacctaa 180
   gtactggtaa cccggtgttc gcgagctcca ccagaggttt tcgaaacgcc gtcatttatc 240
   tggttagaat tagggccttt taactctcaa gggaactaat ttgaatttta atgagagtcg 300
   ttggtctcta tagaggtttc tagctgagga gatataacgt aaaatattct agaaactaag 360
   catgtagagg ttagcgggga gtttactaag gacgagagtt cgaatctctc cacctccacc 420
   a                                                                 421
<210> 22
   <211> 421
   <212> RNA
   <213> Chlamydia trachomatis (mouse pneumonitis)
<400> 22
   ggggguguaa agguuucgac uuagaaauga agcguuaauu gcaugcggag ggcguuggcu 60
   ggccuccuaa aaagccgaca aaacaauaaa ugccgaaccu aaggcugaau gcgaaauuau 120
   cagcuucgcu gaucuuaaug aucuaagagu ugcugcuuaa uuagcaaagu uguuaccuaa 180
   guacugguaa cccgguguuc gcgagcucca ccagagguuu ucgaaacgcc gucauuuauc 240
   ugguuagaau uagggccuuu uaacucucaa gggaacuaau uugaauuuua augagagucg 300
   uuggucucua uagagguuuc uagcugagga gauauaacgu aaaauauucu agaaacuaag 360
   cauguagagg uuagcgggga guuuacuaag gacgagaguu cgaaucucuc caccuccacc 420
   a                                                                 421
<210> 23
   <211> 404
   <212> DNA
   <213> Chlorobium tepidum
<400> 23
   ggggatgaca ggctatcgac aggataggtg tgagatgtcg ttgcactccg agtttcagca 60
   tggacggact cgttaaacaa gtctatgtac caatagatgc agacgattat tcgtatgcaa 120
   tggctgcctg attagcacaa gttaattcag aagccatcgt cctgcggtga atgcgcttac 180
   tctgaagccg ccggatggca taacccgcgc ttgagcctac gggttcgcgc aagtaagctc 240
   cgtacattca tgcccgaggg ggtgtgcggg taaccaatcg ggataagggg acgaacgctg 300
   ctggcggtgt aatcggacca cgaaaaacca accaccagag atgagtgtgg taactgcatc 360
   gagcagtgtc ctggacgcgg gttcaagtcc cgccatctcc acca                  404
<210> 24
   <211> 404
   <212> RNA
   <213> Chlorobium tepidum
<400> 24
   ggggaugaca ggcuaucgac aggauaggug ugagaugucg uugcacuccg aguuucagca 60
   uggacggacu cguuaaacaa gucuauguac caauagaugc agacgauuau ucguaugcaa 120
   uggcugccug auuagcacaa guuaauucag aagccaucgu ccugcgguga augcgcuuac 180
   ucugaagccg ccggauggca uaacccgcgc uugagccuac ggguucgcgc aaguaagcuc 240
   cguacauuca ugcccgaggg ggugugcggg uaaccaaucg ggauaagggg acgaacgcug 300
   cuggcggugu aaucggacca cgaaaaacca accaccagag augagugugg uaacugcauc 360
   gagcaguguc cuggacgcgg guucaagucc cgccaucucc acca                  404
<210> 25
   <211> 294
   <212> DNA
   <213> Cyanophora paradoxa (alga) cyanelle
<400> 25
   ggggctgttt aggtttcgac gtttttttct aattatgttt gttaagcaag tcgaggattt 60
   gttctatctc gaaaatcaag aactctcaaa atttaaacgc aactaatatt gtacgtttta 120
   accgtaaagc agctttcgct gtttaataat tacttttaat ttaaaaacct aattttttta 180
   ggaatttatt tatttattgt ttatcctgct taatgaatta aaaaaagcta tacttgtgaa 240
   taaacgcata atttaaaaaa acggacgtgg gttcaaatcc caccagctcc acca       294
<210> 26
   <211> 294
   <212> RNA
   <213> Cyanophora paradoxa (alga) cyanelle
<400> 26
   ggggcuguuu agguuucgac guuuuuuucu aauuauguuu guuaagcaag ucgaggauuu 60
   guucuaucuc gaaaaucaag aacucucaaa auuuaaacgc aacuaauauu guacguuuua 120
   accguaaagc agcuuucgcu guuuaauaau uacuuuuaau uuaaaaaccu aauuuuuuua 180
   ggaauuuauu uauuuauugu uuauccugcu uaaugaauua aaaaaagcua uacuugugaa 240
   uaaacgcaua auuuaaaaaa acggacgugg guucaaaucc caccagcucc acca       294
<210> 27
   <211> 189
   <212> DNA
   <213> Clostridium acetobutylicum
<400> 27
   aatctggcgt cgagagcggg gaaacgagcc ttacaaagct ttgagtaagg aacggaattt 60
   atgaagctac tgaagtgaaa agcttgtttg taggcgtttc atggagggaa tgttaaaata 120
   caaactgcac tcggagatgc ttaatgaaac cattttcgga caggggttcg attcccctcg 180
   cctccacca 189
<210> 28
   <211> 189
   <212> RNA
   <213> Clostridium acetobutylicum
<400> 28
   aaucuggcgu cgagagcggg gaaacgagcc uuacaaagcu uugaguaagg aacggaauuu 60
   augaagcuac ugaagugaaa agcuuguuug uaggcguuuc auggagggaa uguuaaaaua 120
   caaacugcac ucggagaugc uuaaugaaac cauuuucgga cagggguucg auuccccucg 180
   ccuccacca 189
<210> 29
   <211> 349
   <212> DNA
   <213> Deinococcus radiodurans
<400> 29
   gggggtgacc cggtttcgac aggggaactg aaggtgatgt tgcgtgtcga ggtgccgttg 60
   gcctcgtaaa caaacggcaa agccatttaa ctggcaacca gaactacgct ctcgctgctt 120
   aagtgagatg acgaccgtgc agcccggcct ttggcgtcgc ggaagtcact aaaaaagaag 180
   gctagcccag gcgattctcc atagccgacg gcgaaacttt atggagctac ggcctgcgag 240
   aacctgccca ctggtgagcg ccggcccgac aatcaaacag tgggatacac acgtagacgc 300
   acgctggacg gacctttgga cggcggttcg actccgccca cctccacca 349
<210> 30
   <211> 349
   <212> RNA
   <213> Deinococcus radiodurans
<400> 30
   gggggugacc cgguuucgac aggggaacug aaggugaugu ugcgugucga ggugccguug 60
   gccucguaaa caaacggcaa agccauuuaa cuggcaacca gaacuacgcu cucgcugcuu 120
   aagugagaug acgaccgugc agcccggccu uuggcgucgc ggaagucacu aaaaaagaag 180
   gcuagcccag gcgauucucc auagccgacg gcgaaacuuu auggagcuac ggccugcgag 240
   aaccugccca cuggugagcg ccggcccgac aaucaaacag ugggauacac acguagacgc 300
   acgcuggacg gaccuuugga cggcgguucg acuccgccca ccuccacca 349
<210> 31
   <211> 330
   <212> DNA
   <213> Desulfovibrio desulfuricans
<400> 31
   gggactggaa ccgtagcggc aggtcgaggc gccgctggcc tcgtaaaaag cggcacaaaa 60
   gtaattgcca acaacgatta cgactacgct tacgctgcct aataacagcg aggcaatgac 120
   cgtttaacgg tcgcgccgat cagggccatg cctgataacc ctgattggcg acacttatca 180
   ggctggcgaa aaccggctct cgccggggtt tttcgcgagg agtttaccgg cgggattgct 240
   gcgttgtgcc tggtcagggg ccaacagcgc ggtgaaatac atacttgacc taaacctgta 300
   atgcttcgtg tggaatgttc tcggacgggg                                  330
<210> 32
   <211> 330
   <212> RNA
   <213> Desulfovibrio desulfuricans
<400> 32
   gggacuggaa ccguagcggc aggucgaggc gccgcuggcc ucguaaaaag cggcacaaaa 60
   guaauugcca acaacgauua cgacuacgcu uacgcugccu aauaacagcg aggcaaugac 120
   cguuuaacgg ucgcgccgau cagggccaug ccugauaacc cugauuggcg acacuuauca 180
   ggcuggcgaa aaccggcucu cgccgggguu uuucgcgagg aguuuaccgg cgggauugcu 240
   gcguugugcc uggucagggg ccaacagcgc ggugaaauac auacuugacc uaaaccugua 300
   augcuucgug uggaauguuc ucggacgggg                                  330
<210> 33
   <211> 318
   <212> DNA
   <213> Dichelobacter nodosus
<400> 33
   ctcgaggtgc atgtcgagaa tgagagaatc tcgttaaata ctttcaaaac ttatagttgc 60
   aaacgacgac aactacgctt tagcggctta attcccgctt tcgcttacct agatttgtct 120
   gtgggtttac cgtaagcgac attaacacag aatcgctggt taacgcgtcc gctgttaatc 180
   ggttaaatta agcggaatcg cttgtaaaat gcctgagcgt tggctgttta tgagttaaac 240
   ctaattaact gctctaaaca tgtagtacca aaagttaagg attcgcggac gggggttcaa 300
   atccccccgc ctccacca                                                318
<210> 34
   <211> 318
   <212> RNA
   <213> Dichelobacter nodosus
<400> 34
   cucgaggugc augucgagaa ugagagaauc ucguuaaaua cuuucaaaac uuauaguugc 60
   aaacgacgac aacuacgcuu uagcggcuua auucccgcuu ucgcuuaccu agauuugucu 120
   guggguuuac cguaagcgac auuaacacag aaucgcuggu uaacgcgucc gcuguuaauc 180
   gguuaaauua agcggaaucg cuuguaaaau gccugagcgu uggcuguuua ugaguuaaac 240
   cuaauuaacu gcucuaaaca uguaguacca aaaguuaagg auucgcggac ggggguucaa 300
   auccccccgc cuccacca                                                318
<210> 35
   <211> 367
   <212> DNA
   <213> Enterococcus faecalis
<400> 35
   gggggcgtta cggattcgac aggcatagtt gagcttgaat tgcgtttcgt aggttacggc 60
   tacgttaaaa cgttacagtt aaatataact gctaaaaacg aaaacaattc tttcgcttta 120
   gctgcctaaa aaccagctag cgaagatcct cccggcatcg cccatgtgct cgggtcaggg 180
   tcctaatcga agtgggatac gctaaatttt tccgtctgta aaatttagag gagcttacca 240
   gactagcaat acagaatgcc tgtcactcgg cacgctgtaa agcgaacctt taaatgagtg 300
   tctatgaacg tagagattta agtggcaata tgtttggacg cgggttcgac tcccgccgtc 360
   tccacca                                                           367
<210> 36
   <211> 367
   <212> RNA
   <213> Enterococcus faecalis
<400> 36
   gggggcguua cggauucgac aggcauaguu gagcuugaau ugcguuucgu agguuacggc 60
   uacguuaaaa cguuacaguu aaauauaacu gcuaaaaacg aaaacaauuc uuucgcuuua 120
   gcugccuaaa aaccagcuag cgaagauccu cccggcaucg cccaugugcu cgggucaggg 180
   uccuaaucga agugggauac gcuaaauuuu uccgucugua aaauuuagag gagcuuacca 240
   gacuagcaau acagaaugcc ugucacucgg cacgcuguaa agcgaaccuu uaaaugagug 300
   ucuaugaacg uagagauuua aguggcaaua uguuuggacg cggguucgac ucccgccguc 360
   uccacca                                                           367
<210> 37
   <211> 363
   <212> DNA
   <213> Escherichia coli
<400> 37
   ggggctgatt ctggattcga cgggatttgc gaaacccaag gtgcatgccg aggggcggtt 60
   ggcctcgtaa aaagccgcaa aaaatagtcg caaacgacga aaactacgct ttagcagctt 120
   aataacctgc ttagagccct ctctccctag cctccgctct taggacgggg atcaagagag 180
   gtcaaaccca aaagagatcg cgtggaagcc ctgcctgggg ttgaagcgtt aaaacttaat 240
   caggctagtt tgttagtggc gtgtccgtcc gcagctggca agcgaatgta aagactgact 300
   aagcatgtag taccgaggat gtaggaattt cggacgcggg ttcaactccc gccagctcca 360
   cca                                                               363
<210> 38
   <211> 363
   <212> RNA
   <213> Escherichia coli
<400> 38
   ggggcugauu cuggauucga cgggauuugc gaaacccaag gugcaugccg aggggcgguu 60
   ggccucguaa aaagccgcaa aaaauagucg caaacgacga aaacuacgcu uuagcagcuu 120
   aauaaccugc uuagagcccu cucucccuag ccuccgcucu uaggacgggg aucaagagag 180
   gucaaaccca aaagagaucg cguggaagcc cugccugggg uugaagcguu aaaacuuaau 240
   caggcuaguu uguuaguggc guguccgucc gcagcuggca agcgaaugua aagacugacu 300
   aagcauguag uaccgaggau guaggaauuu cggacgcggg uucaacuccc gccagcucca 360
   cca 363
<210> 39
   <211> 366
   <212> DNA
   <213> Haemophilus influenzae
<400> 39
   ggggctgatt ctggattcga cgggattagc gaagcccaag gtgcacgtcg aggtgcggta 60
   ggcctcgtaa ataaaccgca aaaaaatagt cgcaaacgac gaacaatacg ctttagcagc 120
   ttaataacct gcatttagcc ttcgcgctcc agcttccgct cgtaagacgg ggataacgcg 180
   gagtcaaacc aaaacgagat cgtgtggaag ccaccgtttg aggatcgaag cactaaattg 240
   aatcaaacta gcttaagttt agcgtgtctg tccgcatgct taagtgaaat taaagacgag 300
   actaaacgtg tagtactgaa ggtagagtaa tttcggacgg gggttcaact ccccccagct 360
   ccacca 366
<210> 40
   <211> 366
   <212> RNA
   <213> Haemophilus influenzae
<400> 40
   ggggcugauu cuggauucga cgggauuagc gaagcccaag gugcacgucg aggugcggua 60
   ggccucguaa auaaaccgca aaaaaauagu cgcaaacgac gaacaauacg cuuuagcagc 120
   uuaauaaccu gcauuuagcc uucgcgcucc agcuuccgcu cguaagacgg ggauaacgcg 180
   gagucaaacc aaaacgagau cguguggaag ccaccguuug aggaucgaag cacuaaauug 240
   aaucaaacua gcuuaaguuu agcgugucug uccgcaugcu uaagugaaau uaaagacgag 300
   acuaaacgug uaguacugaa gguagaguaa uuucggacgg ggguucaacu ccccccagcu 360
   ccacca 366
<210> 41
   <211> 340
   <212> DNA
   <213> Helicobacter pylori (ATC 43504)
<400> 41
   agatttcttg tcgcgcagat agcatgccaa gcgctgcttg taaaacagca acaaaaataa 60
   ctgtaaacaa cacagattac gctccagctt acgctaaagc tgcgtgagtt aatctccttt 120
   tggagctgga ctgattagaa tttctagcgt tttaatcgct ccataacctt aagctagacg 180
   cttttaaaag gtggttcgcc ttttaaacta agaaacaaga actcttgaaa ctatcttaag 240
   gttttagaaa gttggaccag agctagtttt aaggctaaaa actaaccaat tttctaagca 300
   ttgtagaagt ttgtgtttag ggcaagattt ttggactggg 340
<210> 42
   <211> 340
   <212> RNA
   <213> Helicobacter pylori (ATC 43504)
<400> 42
   agauuucuug ucgcgcagau agcaugccaa gcgcugcuug uaaaacagca acaaaaauaa 60
   cuguaaacaa cacagauuac gcuccagcuu acgcuaaagc ugcgugaguu aaucuccuuu 120
   uggagcugga cugauuagaa uuucuagcgu uuuaaucgcu ccauaaccuu aagcuagacg 180
   cuuuuaaaag gugguucgcc uuuuaaacua agaaacaaga acucuugaaa cuaucuuaag 240
   guuuuagaaa guuggaccag agcuaguuuu aaggcuaaaa acuaaccaau uuucuaagca 300
   uuguagaagu uuguguuuag ggcaagauuu uuggacuggg                       340
<210> 43
   <211> 386
   <212> DNA
   <213> Helicobacter pylori (strain 26695)
<400> 43
   ggggctgact tggatttcga cagatttctt gtcgcacaga tagcatgcca agcgctgctt 60
   gtaaaacagc aacaaaaata actgtaaaca acacagatta cgctccagct tacgctaaag 120
   ctgcgtgagt taatctcctt ttggagctgg actgattaga atttctagcg ttttaatcgc 180
   tccataacct taagctagac gcttttaaaa ggtggttcgc cttttaaact aagaaacaag 240
   aactcttgaa actatctcaa ggttttagaa agttggacca gagctagttt taaggctaaa 300
   aaaccaacca attttctaag cattgtagaa gtttgtgttt agggcaagat tttggactgg 360
   gggttcgatt ccccacagct ccacca                                      386
<210> 44
   <211> 386
   <212> RNA
   <213> Helicobacter pylori (strain 26695)
<400> 44 .
   ggggcugacu uggauuucga cagauuucuu gucgcacaga uagcaugcca agcgcugcuu 60
   guaaaacagc aacaaaaaua acuguaaaca acacagauua cgcuccagcu uacgcuaaag 120
   cugcgugagu uaaucuccuu uuggagcugg acugauuaga auuucuagcg uuuuaaucgc 180
   uccauaaccu uaagcuagac gcuuuuaaaa ggugguucgc cuuuuaaacu aagaaacaag 240
   aacucuugaa acuaucucaa gguuuuagaa aguuggacca gagcuaguuu uaaggcuaaa 300
   aaaccaacca auuuucuaag cauuguagaa guuuguguuu agggcaagau uuuggacugg 360
   ggguucgauu ccccacagcu ccacca                                      386
<210> 45
   <211> 312
   <212> DNA
   <213> Klebsiella aerogenes (NCTC 9528)
<400> 45
   gggattcgcg aaacccaagg tgcatgccga ggggcggttg gcctcgtaaa aagccgcaaa 60
   aaaatagtcg caaacgacga aaactacgct ttagcagctt aataacctgc taagagccct 120
   ctctccctag cttccgctcc taagacgggg aataaagaga ggtcaaaccc aaaagagatc 180
   gcgtggaagc cctgcctggg gttgaagcgt taaaactaat caggctagtt tgtcagtggc 240
   gtgtccgtcc gcagctggcc agcgaatgta aagactggac taagcatgta gtgccgagga 300
   tgtaggaatt tc 312
<210> 46
   <211> 312
   <212> RNA
   <213> Klebsiella aerogenes (NCTC 9528)
<400> 46
   gggauucgcg aaacccaagg ugcaugccga ggggcgguug gccucguaaa aagccgcaaa 60
   aaaauagucg caaacgacga aaacuacgcu uuagcagcuu aauaaccugc uaagagcccu 120
   cucucccuag cuuccgcucc uaagacgggg aauaaagaga ggucaaaccc aaaagagauc 180
   gcguggaagc ccugccuggg guugaagcgu uaaaacuaau caggcuaguu ugucaguggc 240
   guguccgucc gcagcuggcc agcgaaugua aagacuggac uaagcaugua gugccgagga 300
   uguaggaauu uc 312
<210> 47
   <211> 316
   <212> DNA
   <213> Lactobacillus lactis (NCTC 662)
<400> 47
   aagcacagtt cgagcttgaa ttgcgtttcg taggttacgt ctacgttaaa acgttacagt 60
   taaatataac tgctaaaaac gaaaacaact cttacgcttt agctgcctaa aaacagttag 120
   cgtagatcct ctcggcatcg cccatgtgct cgagtaaggg tctcaaattt agtgggatac 180
   gttaaacttt tccgtctgta aagtttaaaa gagatcatca gactagcgat acagaatgcc 240
   tgtcactcgg caagctgtaa agcgaaacct caaatgagtt gactatgaac gtagattttt 300
   aagtgtcgat gtgttt 316
<210> 48
   <211> 316
   <212> RNA
   <213> Lactobacillus lactis (NCTC 662)
<400> 48
   aagcacaguu cgagcuugaa uugcguuucg uagguuacgu cuacguuaaa acguuacagu 60
   uaaauauaac ugcuaaaaac gaaaacaacu cuuacgcuuu agcugccuaa aaacaguuag 120
   cguagauccu cucggcaucg cccaugugcu cgaguaaggg ucucaaauuu agugggauac 180
   guuaaacuuu uccgucugua aaguuuaaaa gagaucauca gacuagcgau acagaaugcc 240
   ugucacucgg caagcuguaa agcgaaaccu caaaugaguu gacuaugaac guagauuuuu 300
   aagugucgau guguuu 316
<210> 49
   <211> 362
   <212> DNA
   <213> Legionella pneumophila
<400> 49
   gtgggttgca aaaccggaag tgcatgccga gaaggagatc tctcgtaaat aagactcaat 60
   taaatataaa tgcaaacgat gaaaactttg ctggtgggga agctatcgct gcctaataag 120
   cactttagtt aaaccatcac tgtgtactgg ccaataaacc cagtatcccg ttcgaccgag 180
   cccgcttatc ggtatcgaat caacggtcat aagagataag ctagcgtcct aatctatccc 240
   gggttatggc gcgaaactca gggaatcgct gtgtatcatc ctgcccgtcg gaggagccac 300
   agttaaattc aaaagacaag gctatgcatg tagagctaaa ggcagaggac ttgcggacgc 360
   gg 362
<210> 50
   <211> 362
   <212> RNA
   <213> Legionella pneumophila
<400> 50
   guggguugca aaaccggaag ugcaugccga gaaggagauc ucucguaaau aagacucaau 60
   uaaauauaaa ugcaaacgau gaaaacuuug cuggugggga agcuaucgcu gccuaauaag 120
   cacuuuaguu aaaccaucac uguguacugg ccaauaaacc caguaucccg uucgaccgag 180
   cccgcuuauc gguaucgaau caacggucau aagagauaag cuagcguccu aaucuauccc 240
   ggguuauggc gcgaaacuca gggaaucgcu guguaucauc cugcccgucg gaggagccac 300
   aguuaaauuc aaaagacaag gcuaugcaug uagagcuaaa ggcagaggac uugcggacgc 360
   gg 362
<210> 51
   <211> 322
   <212> DNA
   <213> Listeria grayi
<400> 51
   acagggatag gtcgagcttg agttgcgagc cggggggatc ggcccgtcat caacgtcaaa 60
   gccaataata actggcaaac aaaacaacaa tttagctttc gctgcctaat agcagtctga 120
   atagctgatc ctccgtgcat cacccatgtg ctacggtaag ggtctcactt ttaagtgggt 180
   tacgctggct tatctccgtc tggggcaaac gagaagagca taatcagact agctagatag 240
   agccctgacg ccgggcagac atctatgcga aatccaaata cggcaactac gctcgtagat 300
   gctcaagtgc cgatatttct gg 322
<210> 52
   <211> 322
   <212> RNA
   <213> Listeria grayi
<400> 52
   acagggauag gucgagcuug aguugcgagc cggggggauc ggcccgucau caacgucaaa 60
   gccaauaaua acuggcaaac aaaacaacaa uuuagcuuuc gcugccuaau agcagucuga 120
   auagcugauc cuccgugcau cacccaugug cuacgguaag ggucucacuu uuaagugggu 180
   uacgcuggcu uaucuccguc uggggcaaac gagaagagca uaaucagacu agcuagauag 240
   agcccugacg ccgggcagac aucuaugcga aauccaaaua cggcaacuac gcucguagau 300
   gcucaagugc cgauauuucu gg 322
<210> 53
   <211> 322
   <212> DNA
   <213> Listeria innocua
<400> 53
   acagggatag ttcgagcttg agttgcgagt cggggggatc gtcctcgtta tcaacgtcaa 60
   agccaataat aactggcaaa gaaaaacaaa acctagcttt cgctgcctaa taagcagtag 120
   catagctgat cctccgtgca tcgcccatgt gctacggtaa gggtctcact ctaagtgggc 180
   tacactagtt aatctccgtc tgaggttaaa tagaagagct taatcagact agctgaatgg 240
   aagcctgtta ccgggctgat gtttatgcga aatgctaata cggtgactac gctcgtagat 300
   attcaagtgc cgatatttct gg 322
<210> 54
   <211> 322
   <212> RNA
   <213> Listeria innocua
<400> 54
   acagggauag uucgagcuug aguugcgagu cggggggauc guccucguua ucaacgucaa 60
   agccaauaau aacuggcaaa gaaaaacaaa accuagcuuu cgcugccuaa uaagcaguag 120
   cauagcugau ccuccgugca ucgcccaugu gcuacgguaa gggucucacu cuaagugggc 180
   uacacuaguu aaucuccguc ugagguuaaa uagaagagcu uaaucagacu agcugaaugg 240
   aagccuguua ccgggcugau guuuaugcga aaugcuaaua cggugacuac gcucguagau 300
   auucaagugc cgauauuucu gg 322
<210> 55
   <211> 322
   <212> DNA
   <213> Listeria monocytogenes (NCTC 7973)
<400> 55
   acagggatag ttcgagcttg agttgcgagt cggggggatc gtcctcgtta tcaacgtcaa 60
   agccaataat aactggcaaa gaaaaacaaa acctagcttt cgctgcctaa taagcagtag 120
   catagctgat cctccgtgca tcgcccatgt gctacggtaa gggtctcact ctaagtgggc 180
   tacactagtt aatctccgtc tggggttaaa tagaagagct taatcagact agctgaatgg 240
   aagcctgtta ccgggccgat gtttatgcga aatgctaata cggtgactac gctcgtagat 300
   atttaagtgc cgatatttct gg 322
<210> 56
   <211> 322
   <212> RNA
   <213> Listeria monocytogenes (NCTC 7973)
<400> 56
   acagggauag uucgagcuug aguugcgagu cggggggauc guccucguua ucaacgucaa 60
   agccaauaau aacuggcaaa gaaaaacaaa accuagcuuu cgcugccuaa uaagcaguag 120
   cauagcugau ccuccgugca ucgcccaugu gcuacgguaa gggucucacu cuaagugggc 180
   uacacuaguu aaucuccguc ugggguuaaa uagaagagcu uaaucagacu agcugaaugg 240
   aagccuguua ccgggccgau guuuaugcga aaugcuaaua cggugacuac gcucguagau 300
   auuuaagugc cgauauuucu gg 322
<210> 57
   <211> 247
   <212> DNA
   <213> Listeria monocytogenes (NCTC 11994)
<400> 57
   caaagccaat aataactggc aaagaaaaac aaaacctagc tttcgctgcc taataagcag 60
   tagcatagct gatcctccgt gcatcgccca tgtgctacgg taagggtctc actctaagtg 120
   ggctacacta gttaatctcc gtctggggtt aaatagaaga gcttaatcag actagctgaa 180
   tggaagcctg ttaccgggcc gatgtttatg cgaaatgcta atacggtgac tacgctcgta 240
   gatattt 247
<210> 58
   <211> 247
   <212> RNA
   <213> Listeria monocytogenes (NCTC 11994)
<400> 58
   caaagccaau aauaacuggc aaagaaaaac aaaaccuagc uuucgcugcc uaauaagcag 60
   uagcauagcu gauccuccgu gcaucgccca ugugcuacgg uaagggucuc acucuaagug 120
   ggcuacacua guuaaucucc gucugggguu aaauagaaga gcuuaaucag acuagcugaa 180
   uggaagccug uuaccgggcc gauguuuaug cgaaaugcua auacggugac uacgcucgua 240
   gauauuu 247
<210> 59
   <211> 322
   <212> DNA
   <213> Listeria murrayi
<400> 59
   acagggatag ttcgagcttg agttgcgagt cggggggatc gtcctcgtta tcaacgtcaa 60
   agccaataat aactggcaaa gaaaaacaaa acctagcttt cgctgcctaa taagcagtag 120
   catagctgat cctccgtgca tcgcccatgt gctacggtaa gggtctcact ctaagtgggc 180
   tacactagtt aatctccgtc tgaggttaaa tagaagagct taatgagact agctgaatgg 240
   aagcctgtta ccgggctgat gtttatgcga aatgctaata cggtgactac gctcgtagat 300
   attcaagtgc cgatatttct gg 322
<210> 60
   <211> 322
   <212> RNA
   <213> Listeria murrayi
<400> 60
   acagggauag uucgagcuug aguugcgagu cggggggauc guccucguua ucaacgucaa 60
   agccaauaau aacuggcaaa gaaaaacaaa accuagcuuu cgcugccuaa uaagcaguag 120
   cauagcugau ccuccgugca ucgcccaugu gcuacgguaa gggucucacu cuaagugggc 180
   uacacuaguu aaucuccguc ugagguuaaa uagaagagcu uaaugagacu agcugaaugg 240
   aagccuguua ccgggcugau guuuaugcga aaugcuaaua cggugacuac gcucguagau 300
   auucaagugc cgauauuucu gg                                          322
<210> 61
   <211> 322
   <212> DNA
   <213> Listeria welshimeri
<400> 61
   acagggatag ttcgagcttg agttgcgagt cggggggatc gtcctcgtta tcaacgtcaa 60
   agccaataat aactggcaaa gaaaaacaaa acctagcttt cgctgcctaa taagcagtag 120
   catagctgat cctccgtgca tcgcccatgt gctacggtaa gggtctcact ctaagtgggc 180
   tacactggct aatctccgtc tgaggttagt tggaagagct taatcagact agctgaatgg 240
   aagcctgtta ccgggccgat gtttatgcga aatgctaata cggtgactac gctcgtagat 300
   atttaagtgc cgatatttct gg                                          322
<210> 62
   <211> 322
   <212> RNA
   <213> Listeria welshimeri
<400> 62
   acagggauag uucgagcuug aguugcgagu cggggggauc guccucguua ucaacgucaa 60
   agccaauaau aacuggcaaa gaaaaacaaa accuagcuuu cgcugccuaa uaagcaguag 120
   cauagcugau ccuccgugca ucgcccaugu gcuacgguaa gggucucacu cuaagugggc 180
   uacacuggcu aaucuccguc ugagguuagu uggaagagcu uaaucagacu agcugaaugg 240
   aagccuguua ccgggccgau guuuaugcga aaugcuaaua cggugacuac gcucguagau 300
   auuuaagugc cgauauuucu gg                                          322
<210> 63
   <211> 322
   <212> DNA
   <213> Marinobacter hydrocarbonoclasticus
<400> 63
   gccggtgacg aacccttggg tgcatgccga gatggcagcg aatctcgtaa atccaaagct 60
   gcaacgtaat agtcgcaaac gacgaaaact acgcactggc ggcgtaagcc gttccagtcg 120
   tcctggctga ggcgcctata actcagtagc aacatcccag gacgtcatcg cttataggct 180
   gctccgttca ccagagctca ctggtgttcg gctaagatta aagagctcgc ctcttgcacc 240
   ctgaccttcg ggtcgcttga ggttaaatca atagaaggac actaagcatg tagacctcaa 300
   ggcctagtgc tggcggacgc gg                                          322
<210> 64
   <211> 322
   <212> RNA
   <213> Marinobacter hydrocarbonoclasticus
<400> 64
   gccggugacg aacccuuggg ugcaugccga gauggcagcg aaucucguaa auccaaagcu 60
   gcaacguaau agucgcaaac gacgaaaacu acgcacuggc ggcguaagcc guuccagucg 120
   uccuggcuga ggcgccuaua acucaguagc aacaucccag gacgucaucg cuuauaggcu 180
   gcuccguuca ccagagcuca cugguguucg gcuaagauua aagagcucgc cucuugcacc 240
   cugaccuucg ggucgcuuga gguuaaauca auagaaggac acuaagcaug uagaccucaa 300
   ggccuagugc uggcggacgc gg 322
<210> 65
   <211> 338
   <212> DNA
   <213> Mycobacterium avium
<400> 65
   ttcgcgcatc gaatcaaggg aagcgtgccg gtgcaggcaa ctgaccaccg taagcgtcgt 60
   tgcaaataga taagcgccga ttcacatcag cgcgacttac ctctcgctgc ctaagcgaca 120
   gctagtccgt cagcccggga acgccctcga cccggagcct ggcgtcagct agagggatcc 180
   accgatgagt tcggtcgcgg gactcatcgg gacaccaaca gcgactggga tcgtcatcct 240
   ggcttgttcg cgtgaccagg agatccgagt agaggcatag cgaactgcgc acggagaagc 300
   cttgagggaa tgccgtagaa cccgggttcg attcccaa 338
<210> 66
   <211> 338
   <212> RNA
   <213> Mycobacterium avium
<400> 66
   uucgcgcauc gaaucaaggg aagcgugccg gugcaggcaa cugaccaccg uaagcgucgu 60
   ugcaaauaga uaagcgccga uucacaucag cgcgacuuac cucucgcugc cuaagcgaca 120
   gcuaguccgu cagcccggga acgcccucga cccggagccu ggcgucagcu agagggaucc 180
   accgaugagu ucggucgcgg gacucaucgg gacaccaaca gcgacuggga ucgucauccu 240
   ggcuuguucg cgugaccagg agauccgagu agaggcauag cgaacugcgc acggagaagc 300
   cuugagggaa ugccguagaa cccggguucg auucccaa 338
<210> 67
   <211> 318
   <212> DNA
   <213> Mycobacterium bovis
<400> 67
   ttcgcgcatc gaatcaaggg aagcgtgccg gtgcaggcaa gagaccaccg taagcgtcgt 60
   tgcgaccaaa taagcgccga ttcacatcag cgcgactacg tctcgctgcc taagcgacgg 120
   ctagtctgtc agaccgggaa cgccctcggc ccggaccctg gcatcagcta gagggatcca 180
   ccgatgagtc cggtcgcggg actcctcggg acaaccacag cgactgggat cgtcatctcg 240
   gctagttcgc gtgaccggga gatccgagca gaggcatagc gaactgcgca cggagaagcc 300
   ttgagggaat gccgtagg 318
<210> 68
   <211> 318
   <212> RNA
   <213> Mycobacterium bovis
<400> 68
   uucgcgcauc gaaucaaggg aagcgugccg gugcaggcaa gagaccaccg uaagcgucgu 60
   ugcgaccaaa uaagcgccga uucacaucag cgcgacuacg ucucgcugcc uaagcgacgg 120
   cuagucuguc agaccgggaa cgcccucggc ccggacccug gcaucagcua gagggaucca 180
   ccgaugaguc cggucgcggg acuccucggg acaaccacag cgacugggau cgucaucucg 240
   gcuaguucgc gugaccggga gauccgagca gaggcauagc gaacugcgca cggagaagcc 300
   uugagggaau gccguagg 318
<210> 69
   <211> 369
   <212> DNA
   <213> Mycobacterium leprae
<400> 69
   ggggctgaaa ggtttcgact tcgcgcatcg aatcaaggga agcgtgccgg tgcaggcaag 60
   agaccaccgt aagcgtcgtt gcagcaatat aagcgccgat tcatatcagc gcgactatgc 120
   tctcgctgcc taagcgatgg ctagtctgtc agaccgggaa cgccctcgtc ccggagcctg 180
   gcatcagcta gagggatcta ccgatgggtt cggtcgcggg actcgtcggg acaccaaccg 240
   cgactgggat cgtcatcctg gctagttcgc gtgatcagga gatccgagta gaggcatagc 300
   gaactacgca cggagaagcc ttgagggaaa tgccgtagga cccgggttcg attcccggca 360
   gctccacca 369
<210> 70
   <211> 369
   <212> RNA
   <213> Mycobacterium leprae
<400> 70
   ggggcugaaa gguuucgacu ucgcgcaucg aaucaaggga agcgugccgg ugcaggcaag 60
   agaccaccgu aagcgucguu gcagcaauau aagcgccgau ucauaucagc gcgacuaugc 120
   ucucgcugcc uaagcgaugg cuagucuguc agaccgggaa cgcccucguc ccggagccug 180
   gcaucagcua gagggaucua ccgauggguu cggucgcggg acucgucggg acaccaaccg 240
   cgacugggau cgucauccug gcuaguucgc gugaucagga gauccgagua gaggcauagc 300
   gaacuacgca cggagaagcc uugagggaaa ugccguagga cccggguucg auucccggca 360
   gcuccacca 369
<210> 71
   <211> 338
   <212> DNA
   <213> Mycobacterium paratuberculosis
<400> 71
   ttcgcgcatc gaatcaaggg aagcgtgccg gtgcaggcaa ctgaccaccg taagcgtcgt 60
   tgcaaataga taagcgccga ttcacatcag cgcgacttac ctctcgctgc ctaagcgaca 120
   gctagtccgt cagcccggga acgccctcga cccggagcct ggcgtcagct agagggatcc 180
   accgatgagt tcggtcgcgg gactcatcgg gacaccaaca gcgactggga tcgtcatcct 240
   ggcttgttcg cgtgaccagg agatccgagt agaggcatag cgaactgcgc acggagaagc 300
   cttgagggaa tgccgtagaa cccgggttcg attcccaa 338
<210> 72
   <211> 338
   <212> RNA
   <213> Mycobacterium paratuberculosis
<400> 72
   uucgcgcauc gaaucaaggg aagcgugccg gugcaggcaa cugaccaccg uaagcgucgu 60
   ugcaaauaga uaagcgccga uucacaucag cgcgacuuac cucucgcugc cuaagcgaca 120
   gcuaguccgu cagcccggga acgcccucga cccggagccu ggcgucagcu agagggaucc 180
   accgaugagu ucggucgcgg gacucaucgg gacaccaaca gcgacuggga ucgucauccu 240
   ggcuuguucg cgugaccagg agauccgagu agaggcauag cgaacugcgc acggagaagc 300
   cuugagggaa ugccguagaa cccggguucg auucccaa 338
<210> 73
   <211> 368
   <212> DNA
   <213> Mycobacterium tuberculosis
<400> 73
   ggggctgaac ggtttcgact tcgcgcatcg aatcaaggga agcgtgccgg tgcaggcaag 60
   agaccaccgt aagcgtcgtt gcgaccaaat aagcgccgat tcacatcagc gcgactacgc 120
   tctcgctgcc taagcgacgg ctagtctgtc agaccgggaa cgccctcggc ccggaccctg 180
   gcatcagcta gagggatcca ccgatgagtc cggtcgcggg actcctcggg acaaccacag 240
   cgactgggat cgtcatctcg gctagttcgc gtgaccggga gatccgagca gaggcatagc 300
   gaactgcgca cggagaagcc ttgagggaat gccgtaggac ccgggttcga ttcccggcag 360
   ctccacca 368
<210> 74
   <211> 368
   <212> RNA
   <213> Mycobacterium tuberculosis
<400> 74
   ggggcugaac gguuucgacu ucgcgcaucg aaucaaggga agcgugccgg ugcaggcaag 60
   agaccaccgu aagcgucguu gcgaccaaau aagcgccgau ucacaucagc gcgacuacgc 120
   ucucgcugcc uaagcgacgg cuagucuguc agaccgggaa cgcccucggc ccggacccug 180
   gcaucagcua gagggaucca ccgaugaguc cggucgcggg acuccucggg acaaccacag 240
   cgacugggau cgucaucucg gcuaguucgc gugaccggga gauccgagca gaggcauagc 300
   gaacugcgca cggagaagcc uugagggaau gccguaggac ccggguucga uucccggcag 360
   cuccacca                                                          368
<210> 75
   <211> 411
   <212> DNA
   <213> Mycoplasma capricolum
<400> 75
   ggggatgtca tggatttgac aggatatctt tagtacatat aagcagtagt gttgtagact 60
   ataaatacta ctaggtttaa aaaaacgcaa ataaaaacga agaaactttt gaaatgccag 120
   catttatgat gaataatgca tcagctggag caaactttat gtttgcttaa taactactag 180
   tttagttata gtatttcacg aattatagat attttaagct ttatttataa ccgtattacc 240
   caagcttaat agaatatatg attgcaataa atatatttga aatctaattg caaatgatat 300
   ttaaccttta gttaatttta gttaaatatt ttaattagaa aattaactaa actgtagaaa 360
   gtatgtatta atatatcttg gacgcgagtt cgattctcgc catctccacc a          411
<210> 76
   <211> 411
   <212> RNA
   <213> Mycoplasma capricolum
<400> 76
   ggggauguca uggauuugac aggauaucuu uaguacauau aagcaguagu guuguagacu 60
   auaaauacua cuagguuuaa aaaaacgcaa auaaaaacga agaaacuuuu gaaaugccag 120
   cauuuaugau gaauaaugca ucagcuggag caaacuuuau guuugcuuaa uaacuacuag 180
   uuuaguuaua guauuucacg aauuauagau auuuuaagcu uuauuuauaa ccguauuacc 240
   caagcuuaau agaauauaug auugcaauaa auauauuuga aaucuaauug caaaugauau 300
   uuaaccuuua guuaauuuua guuaaauauu uuaauuagaa aauuaacuaa acuguagaaa 360
   guauguauua auauaucuug gacgcgaguu cgauucucgc caucuccacc a          411
<210> 77
   <211> 388
   <212> DNA
   <213> Mycoplasma genitalium (ATTC 33530, #1)
<400> 77
   ggggatgttt tgggtttgac ataatgctga tagacaaaca gtagcattgg ggtatgcccc 60
   ttacagcgct aggttcaata accgacaaag aaaataacga agtgttggta gaaccaaatt 120
   tgatcattaa ccaacaagca agtgttaact ttgcttttgc ataagtagat actaaagcta 180
   cagctggtga atagtcatag tttgctagct gtcatagttt atgactcgag gttaaatcgt 240
   tcaatttaac ctttaaaaat agaacttgtt gtttccatga ttgttttgtg atcaattgga 300
   aacaagacaa aaatccacaa aactaaaatg tagaagctgt ttgttgtgtc ctttatggaa 360
   acgggttcga ttcccgtcat ctccacca                                    388
<210> 78
   <211> 388
   <212> RNA
   <213> Mycoplasma genitalium (ATTC 33530, #1)
<400> 78
   ggggauguuu uggguuugac auaaugcuga uagacaaaca guagcauugg gguaugcccc 60
   uuacagcgcu agguucaaua accgacaaag aaaauaacga aguguuggua gaaccaaauu 120
   ugaucauuaa ccaacaagca aguguuaacu uugcuuuugc auaaguagau acuaaagcua 180
   cagcugguga auagucauag uuugcuagcu gucauaguuu augacucgag guuaaaucgu 240
   ucaauuuaac cuuuaaaaau agaacuuguu guuuccauga uuguuuugug aucaauugga 300
   aacaagacaa aaauccacaa aacuaaaaug uagaagcugu uuguuguguc cuuuauggaa 360
   acggguucga uucccgucau cuccacca                                   388
<210> 79
   <211> 243
   <212> RNA
   <213> Mycoplasma genitalium (ATTC 33530, #2)
<400> 79
   acaaagcgaa gacaaacaga gcaggggagc cccacagcgc aggcaaaacc gacaaagaaa 60
   aaacgaaggg gagaccaaag acaaaccaac aagcaaggaa cgcgcaaaga gaacaaagca 120
   cagcgggaaa gcaaggcagc gcaagagacc gaggaaacgc aaaaccaaaa aagaacggcc 180
   agagggacaa ggaaacaaga caaaaaccac aaaacaaaag agaagcgggg ccaggaaacg 240
   ggc                                                               243
<210> 80
   <211> 350
   <212> RNA
   <213> Mycoplasma genitalium (ATTC 33530, #2)
<400> 80
   acauaaugcu gauagacaaa caguagcauu gggguaugcc ccuuacagcg cuagguucaa 60
   uaaccgacaa agaaaauaac gaaguguugg uagauccaaa uuugaucauu aaccaacaag 120
   caaguguuaa cuuugcuuuu gcauaaguag auacuaaagc uacagcuggu gaauagucau 180
   aguuugcuag cugucauagu uuaugacucg agguuaaauc guucaauuua accuuuaaaa 240
   auagaacuug uuguuuccau gauuguuuug ugaucaauug gaaacaagac aaaaauccac 300
   aaaacuaaaa uguagaagcu guuuguugug uccuuuaugg aaacggguuc            350
<210> 81
   <211> 387
   <212> DNA
   <213> Mycoplasma pneumophila
<400> 81
   ggggatgtag aggttttgac ataatgttga aaggaaaaca gttgcagtgg ggtatgcccc 60
   ttacagctct aggtataata accgacaaaa ataacgacga agttttggta gatccaatgt 120
   tgatcgctaa ccaacaagca agtatcaact acgctttcgc ttagaacata ctaaagctac 180
   acgaattgaa tcgccatagt ttggttcgtg tcacagttta tggctcgggg ttaactggtt 240
   caacttaatc cttaaattat gaacttatcg tttacttgtt tgtcttatga tctaaagtaa 300
   gcgagacatt aaaacataag actaaactgt agaagctgtt ttaccaatcc tttatggaaa 360
   cgggttcgat tcccgtcatc tccacca                                     387
<210> 82
   <211> 387
   <212> RNA
   <213> Mycoplasma pneumophila
<400> 82
   ggggauguag agguuuugac auaauguuga aaggaaaaca guugcagugg gguaugcccc 60
   uuacagcucu agguauaaua accgacaaaa auaacgacga aguuuuggua gauccaaugu 120
   ugaucgcuaa ccaacaagca aguaucaacu acgcuuucgc uuagaacaua cuaaagcuac 180
   acgaauugaa ucgccauagu uugguucgug ucacaguuua uggcucgggg uuaacugguu 240
   caacuuaauc cuuaaauuau gaacuuaucg uuuacuuguu ugucuuauga ucuaaaguaa 300
   gcgagacauu aaaacauaag acuaaacugu agaagcuguu uuaccaaucc uuuauggaaa 360
   cggguucgau ucccgucauc uccacca                                     387
<210> 83
   <211> 318
   <212> DNA
   <213> Neisseria gonorrhoeae (ATCC 19424)
<400> 83
   gggggttgcg aagcagatgc gggcataccg gggtctcaga ttcccgtaaa acactgaatt 60
   caaatagtcg caaacgacga aacttacgct ttagccgctt aaggctagcc gttgcagcag 120
   tcggtcaatg ggctgtgtgg cgaaagccac cgcaacgtca tcttacattg actggtttcc 180
   agccgggtta cttggcagga aataagactt aaggtaactg gtttccaaaa ggcctgttgg 240
   tcggcatgat ggaaataaga ttttcaaata gacacaacta agtatgtaga acgctttgta 300
   gaggactttc ggacgggg                                               318
<210> 84
   <211> 318
   <212> RNA
   <213> Neisseria gonorrhoeae (ATCC 19424)
<400> 84
   ggggguugcg aagcagaugc gggcauaccg gggucucaga uucccguaaa acacugaauu 60
   caaauagucg caaacgacga aacuuacgcu uuagccgcuu aaggcuagcc guugcagcag 120
   ucggucaaug ggcugugugg cgaaagccac cgcaacguca ucuuacauug acugguuucc 180
   agccggguua cuuggcagga aauaagacuu aagguaacug guuuccaaaa ggccuguugg 240
   ucggcaugau ggaaauaaga uuuucaaaua gacacaacua aguauguaga acgcuuugua 300
   gaggacuuuc ggacgggg                                               318
<210> 85
   <211> 363
   <212> DNA
   <213> Neisseria gonorrhoeae (FA 1090)
<400> 85
   gggggcgacc ttggtttcga cgggggttgc gaagcagatg cgggcatacc ggggtctcag 60
   attcccgtaa aacactgaat tcaaatagtc gcaaacgacg aaacttacgc tttagccgct 120
   taaggctagc cgttgcagca gtcggtcaat gggctgtgtg gtgaaagcca ccgcaacgtc 180
   atcttacatt gactggtttc cagccgggtt acttggcagg aaataagact taaggtaact 240
   ggtttccaaa aggcctgttg gtcggcatga tggaaataag attttcaaat agacacaact 300
   aagtatgtag aacgctttgt agaggacttt cggacggggg ttcgattccc cccgcctcca 360
   cca                                                               363
<210> 86
   <211> 363
   <212> RNA
   <213> Neisseria gonorrhoeae (FA 1090)
<400> 86
   gggggcgacc uugguuucga cggggguugc gaagcagaug cgggcauacc ggggucucag 60
   auucccguaa aacacugaau ucaaauaguc gcaaacgacg aaacuuacgc uuuagccgcu 120
   uaaggcuagc cguugcagca gucggucaau gggcugugug gugaaagcca ccgcaacguc 180
   aucuuacauu gacugguuuc cagccggguu acuuggcagg aaauaagacu uaagguaacu 240
   gguuuccaaa aggccuguug gucggcauga uggaaauaag auuuucaaau agacacaacu 300
   aaguauguag aacgcuuugu agaggacuuu cggacggggg uucgauuccc cccgccucca 360
   cca                                                               363
<210> 87
   <211> 363
   <212> DNA
   <213> Neisseria meningitidis
<400> 87
   gggggcgacc ttggtttcga cgggggttgc gaagcagatg cgggcatacc ggggtctcag 60
   attcccgtaa aacactgaat tcaaatagtc gcaaacgacg aaacttacgc tttagccgct 120
   taaggctagc cgttgcagca gtcggtcaat gggctgtgtg gcgaaagcca ccgcaacgtc 180
   atcttacatt gactggtttc ctgccgggtt atttggcagg aaatgagatt taaggtaact 240
   ggtttccaaa aggcctgttg gtcggcatga tggaaataag attttcaaat agacacaact 300
   aagtatgtag aacgctttgt agaggacttt cggacggggg ttcgattccc cccgcctcca 360
   cca                                                               363
<210> 88
   <211> 363
   <212> RNA
   <213> Neisseria meningitidis
<400> 88
   gggggcgacc uugguuucga cggggguugc gaagcagaug cgggcauacc ggggucucag 60
   auucccguaa aacacugaau ucaaauaguc gcaaacgacg aaacuuacgc uuuagccgcu 120
   uaaggcuagc cguugcagca gucggucaau gggcugugug gcgaaagcca ccgcaacguc 180
   aucuuacauu gacugguuuc cugccggguu auuuggcagg aaaugagauu uaagguaacu 240
   gguuuccaaa aggccuguug gucggcauga uggaaauaag auuuucaaau agacacaacu 300
   aaguauguag aacgcuuugu agaggacuuu cggacggggg uucgauuccc cccgccucca 360
   cca 363
<210> 89
   <211> 385
   <212> DNA
   <213> Nostoc muscorum PCC7120
<400> 89
   gggtccgtcg gtttcgacag gttggcgaac gctactctgt gattcaggtc gagagtgagt 60
   ctcctctgca aatcaaggct caaaacaaaa gtaaatgcga ataacatcgt taaatttgct 120
   cgtaaggacg ctctagtagc tgcctaaata gcctctttca ggttcgagcg tcttcggttt 180
   gactccgtta aggactgaag accaaccccc aacggatgct ctagcaatgt tctctggttg 240
   gcttgctagc taagatttaa tcagagcatc ctacgttcgg gataatgaac gattcccgcc 300
   ttgagggtca gaaaggctaa acctgtgaat gagcgggggg tcaataccca atttggacag 360
   cagttcgact ctgctcgatc cacca 385
<210> 90
   <211> 385
   <212> RNA
   <213> Nostoc muscorum PCC7120
<400> 90
   ggguccgucg guuucgacag guuggcgaac gcuacucugu gauucagguc gagagugagu 60
   cuccucugca aaucaaggcu caaaacaaaa guaaaugcga auaacaucgu uaaauuugcu 120
   cguaaggacg cucuaguagc ugccuaaaua gccucuuuca gguucgagcg ucuucgguuu 180
   gacuccguua aggacugaag accaaccccc aacggaugcu cuagcaaugu ucucugguug 240
   gcuugcuagc uaagauuuaa ucagagcauc cuacguucgg gauaaugaac gauucccgcc 300
   uugaggguca gaaaggcuaa accugugaau gagcgggggg ucaauaccca auuuggacag 360
   caguucgacu cugcucgauc cacca 385
<210> 91
   <211> 371
   <212> DNA
   <213> Odontella sinensis (diatom) chloroplast
<400> 91
   ggggctgact tggtttcgac atttaaaaat tgttacagta tgatgcaggt cgaagtttct 60
   aatcttcgta aaaaaagaga aatttataat aaatgctaat aatttaattt cttctgtgtt 120
   taaaagttta tcaactaagc aaaatagttt aaatttaagt tttgctgttt aagttttatg 180
   cacatttaat gatctagtaa ataactttgt tcgctataat ttatatttat aactagactt 240
   ttgtcttttt tatagtttag aataacttta tcatttcaaa cctcgttcca tctagttgaa 300
   ctaaacctgt gaacgaatac tataataaaa tttttagatg gacgtgggtt cgactcccat 360
   cagctccacc a 371
<210> 92
   <211> 371
   <212> RNA
   <213> Odontella sinensis (diatom) chloroplast
<400> 92
   ggggcugacu ugguuucgac auuuaaaaau uguuacagua ugaugcaggu cgaaguuucu 60
   aaucuucgua aaaaaagaga aauuuauaau aaaugcuaau aauuuaauuu cuucuguguu 120
   uaaaaguuua ucaacuaagc aaaauaguuu aaauuuaagu uuugcuguuu aaguuuuaug 180
   cacauuuaau gaucuaguaa auaacuuugu ucgcuauaau uuauauuuau aacuagacuu 240
   uugucuuuuu uauaguuuag aauaacuuua ucauuucaaa ccucguucca ucuaguugaa 300
   cuaaaccugu gaacgaauac uauaauaaaa uuuuuagaug gacgugggUU cgacucccau 360
   cagcuccacc a                                                      371
<210> 93
   <211> 323
   <212> DNA
   <213> Porphyra purpureum (red alga) chloroplast
<400> 93
   ggggctgcaa ggtttctaca ttgtgaaaaa acaaatatat gaaagtaaaa cgagctcatt 60
   attagagctt ttagttaaat aaatgcagaa aataatatta ttgctttttc tcgaaaatta 120
   gctgttgcat aaatagtctc aatttttgta attcgaagtg atagactctt atacactacg 180
   aatattctgt tagagttgct cttaataaaa gaaaagtaaa aaaatacaaa ttcttatgtt 240
   ttttacctga attgattcaa tttaaggtta gtattttttg atttttacaa tggacgtggg 300
   ttcaagtccc accagctcca cca                                         323
<210> 94
   <211> 323
   <212> RNA
   <213> Porphyra purpureum (red alga) chloroplast
<400> 94
   ggggcugcaa gguuucuaca uugugaaaaa acaaauauau gaaaguaaaa cgagcucauu 60
   auuagagcuu uuaguuaaau aaaugcagaa aauaauauua uugcuuuuuc ucgaaaauua 120
   gcuguugcau aaauagucuc aauuuuugua auucgaagug auagacucuu auacacuacg 180
   aauauucugu uagaguugcu cuuaauaaaa gaaaaguaaa aaaauacaaa uucuuauguu 240
   uuuuaccuga auugauucaa uuuaagguua guauuuuuug auuuuuacaa uggacguggg 300
   uucaaguccc accagcucca cca                                         323
<210> 95
   <211>407
   <212> DNA
   <213> Porphyromonas gingivalis
<400> 95
   ggggctgacc ggctttgaca gcgtgatgaa gcggtatgta agcatgtagt gcgtgggtgg 60
   cttgcactat aatctcagac atcaaaagtt taattggcga aaataactac gctctcgctg 120
   cgtaatcgaa gaatagtaga ttagacgctt catcgccgcc aaagtggcag cgacgagaca 180
   tcgcccgagc agctttttcc cgaagtagct cgatggtgcg gtgctgacaa atcgggaacc 240
   gctacaggat gcttcctgcc tgtggtcaga tcgaacggaa gataaggatc gtgcattggg 300
   tcgtttcagc ctccgctcgc tcacgaaaat tccaactgaa actaaacatg tagaaagcat 360
   attgattcca tgtttggacg agggttcaat tccctccagc tccacca 407
<210> 96
   <211>407
   <212> RNA
   <213> Porphyromonas gingivalis
<400> 96
   ggggcugacc ggcuuugaca gcgugaugaa gcgguaugua agcauguagu gcgugggugg 60
   cuugcacuau aaucucagac aucaaaaguu uaauuggcga aaauaacuac gcucucgcug 120
   cguaaucgaa gaauaguaga uuagacgcuu caucgccgcc aaaguggcag cgacgagaca 180
   ucgcccgagc agcuuuuucc cgaaguagcu cgauggugcg gugcugacaa aucgggaacc 240
   gcuacaggau gcuuccugcc uguggucaga ucgaacggaa gauaaggauc gugcauuggg 300
   ucguuucagc cuccgcucgc ucacgaaaau uccaacugaa acuaaacaug uagaaagcau 360
   auugauucca uguuuggacg aggguucaau ucccuccagc uccacca 407
<210> 97
   <211> 310
   <212> DNA
   <213> Proteus rettgeri (NCTC 10975)
<400> 97
   gggatttgcg aaacccaagg tgcatgccga ggggcggttg gcctcgtaaa aagccgcaaa 60
   aaaatagtcg caaacgacga aaactacgct ttagcagctt aataacctgc ttagagccct 120
   ctctccctag cctccgctct tggacgggga tcaagagagg tcaaacccaa aagagatcgc 180
   gtggatgcct tgcctggggt tgaagcgtta aacttaatca ggatagtttg ttggtggcgt 240
   gtctgtccgc agctggcaaa tgaattcaaa gactagacta agcatgtagt accgaggatg 300
   tagaaatttc 310
<210> 98
   <211> 310
   <212> RNA
   <213> Proteus rettgeri (NCTC 10975)
<400> 98
   gggauuugcg aaacccaagg ugcaugccga ggggcgguug gccucguaaa aagccgcaaa 60
   aaaauagucg caaacgacga aaacuacgcu uuagcagcuu aauaaccugc uuagagcccu 120
   cucucccuag ccuccgcucu uggacgggga ucaagagagg ucaaacccaa aagagaucgc 180
   guggaugccu ugccuggggu ugaagcguua aacuuaauca ggauaguuug uugguggcgu 240
   gucuguccgc agcuggcaaa ugaauucaaa gacuagacua agcauguagu accgaggaug 300
   uagaaauuuc 310
<210> 99
   <211> 241
   <212> DNA
   <213> Pseudoalteromonas haloplanktis
<400> 99
   gggagcgaaa cccaagggca gccgaggggc ggggcccgaa aaagccgcaa aaaaaagcgc 60
   aaacgacgaa aacacgcagc agcaaaaccg cagagccccc cccagccccg ccggacgggg 120
   acaagagagg caaacccaaa agagacgcgg gagccgccgg gggaagcgaa acaacaggaa 180
   ggggggcggc gccgcagcgg caaagaacaa agacagacaa gcagagaccg aggagagaaa 240
   c                                                                 241
<210> 100
   <211> 313
   <212> RNA
   <213> Pseudoalteromonas haloplanktis
<400> 100
   ggaauucaag aagcccgagg ugcaugucga ggugcgguuu gccucguaaa aaagccgcaa 60
   uuuaaaguaa ucgcaaacga cgauaacuac ucucuagcag cuuaggcugg cuagcgcucc 120
   uuccauguau ucuuguggac uggauuuugg agugucaccc uaacaccuga ucgcgacgga 180
   aacccuggcc gggguugaag cguuaaaacu aagcggccuc gccuuuaucu accguguuug 240
   uccgggauuu aaagguuaau uaaaugacaa uacuaaacau guaguaccga cggucgaggc 300
   uuuucggacg ggg                                                    313
<210> 101
   <211> 353
   <212> DNA
   <213> Pseudomonas aeruginosa
<400> 101
   ggggccgatt aggattcgac gccggtaaca aaagttgagg ggcatgccga gttggtagca 60
   gaactcgtaa attcgctgct gcaaacttat agttgccaac gacgacaact acgctctagc 120
   tgcttaatgc ggctagcagt cgctagggga tgcctgtaaa cccgaaacga ctgtcagata 180
   gaacaggatc gccgccaagt tcgctgtaga cgtaacggct aaaactcata cagctcgctc 240
   caagcaccct gccactcggg cggcgcggag ttaactcagt agagctggct aagcatgtaa 300
   aaccgatagc ggaaagctgg cggacggggg ttcaaatccc cccggttcca cca        353
<210> 102
   <211> 353
   <212> RNA
   <213> Pseudomonas aeruginosa
<400> 102
   ggggccgauu aggauucgac gccgguaaca aaaguugagg ggcaugccga guugguagca 60
   gaacucguaa auucgcugcu gcaaacuuau aguugccaac gacgacaacu acgcucuagc 120
   ugcuuaaugc ggcuagcagu cgcuagggga ugccuguaaa cccgaaacga cugucagaua 180
   gaacaggauc gccgccaagu ucgcuguaga cguaacggcu aaaacucaua cagcucgcuc 240
   caagcacccu gccacucggg cggcgcggag uuaacucagu agagcuggcu aagcauguaa 300
   aaccgauagc ggaaagcugg cggacggggg uucaaauccc cccgguucca cca        353
<210> 103
   <211> 363
   <212> DNA
   <213> Salmonella typhimurium
<400> 103
   ggggctgatt ctggattcga cgggatttgc gaaacccaag gtgcatgccg aggggcggtt 60
   ggcctcgtaa aaagccgcaa aaaaatagtc gcaaacgacg aaacctacgc tttagcagct 120
   taataacctg cttagagccc tctctcccta gcctccgctc ttaggacggg gatcaagaga 180
   ggtcaaaccc aaaagagatc gcgcggatgc cctgcctggg gttgaagcgt taaaacgaat 240
   caggctagtc tggtagtggc gtgtccgtcc gcaggtgcca ggcgaatgta aagactgact 300
   aagcatgtag taccgaggat gtaggaattt cggacgcggg ttcaactccc gccagctcca 360
   cca 363
<210> 104
   <211> 363
   <212> RNA
   <213> Salmonella typhimurium
<400> 104
   ggggcugauu cuggauucga cgggauuugc gaaacccaag gugcaugccg aggggcgguu 60
   ggccucguaa aaagccgcaa aaaaauaguc gcaaacgacg aaaccuacgc uuuagcagcu 120
   uaauaaccug cuuagagccc ucucucccua gccuccgcuc uuaggacggg gaucaagaga 180
   ggucaaaccc aaaagagauc gcgcggaugc ccugccuggg guugaagcgu uaaaacgaau 240
   caggcuaguc ugguaguggc guguccgucc gcaggugcca ggcgaaugua aagacugacu 300
   aagcauguag uaccgaggau guaggaauuu cggacgcggg uucaacuccc gccagcucca 360
   cca 363
<210> 105
   <211> 355
   <212> DNA
   <213> Shewanella putrefaciens
<400> 105
   gggggcgatt ctggattcga caggattcac gaaaccctgg gagcatgccg aggggcggtt 60
   ggcctcgtaa aaagccgcaa agttatagtt gcaaacgacg ataactacgc tctagccgct 120
   taatgccgct agccatctac cacacgcttt gcacatgggc agtggatttg atggtcatct 180
   cacatcgtgc tagcgaggga accctgtctg ggggtgaacc gcgaaacagt accggactca 240
   ccgtgtggga tcctgtcttt cggagttcaa acggttaaac aatagaaaga ctaagcatgt 300
   agcgccttgg atgtaggttt tctggacgcg ggttcaagtc ccgccgcctc cacca 355
<210> 106
   <211> 355
   <212> RNA
   <213> Shewanella putrefaciens
<400> 106
   gggggcgauu cuggauucga caggauucac gaaacccugg gagcaugccg aggggcgguu 60
   ggccucguaa aaagccgcaa aguuauaguu gcaaacgacg auaacuacgc ucuagccgcu 120
   uaaugccgcu agccaucuac cacacgcuuu gcacaugggc aguggauuug auggucaucu 180
   cacaucgugc uagcgaggga acccugucug ggggugaacc gcgaaacagu accggacuca 240
   ccguguggga uccugucuuu cggaguucaa acgguuaaac aauagaaaga cuaagcaugu 300
   agcgccuugg auguagguuu ucuggacgcg gguucaaguc ccgccgccuc cacca 355
<210> 107
   <211> 362
   <212> DNA
   <213> Staphylococcus aureus
<400> 107
   ggggacgttc atggattcga caggggtccc ccgagctcat taagcgtgtc ggagggttgt 60
   cttcgtcatc aacacacaca gtttataata actggcaaat caaacaataa tttcgcagta 120
   gctgcctaat cgcactctgc atcgcctaac agcatttcct atgtgctgtt aacgcgattc 180
   aaccttaata ggatatgcta aacactgccg tttgaagtct gtttagaaga aacttaatca 240
   aactagcatc atgttggttg tttatcactt ttcatgatgc gaaacctatc gataaactac 300
   acacgtagaa agatgtgtat caggaccttt ggacgcgggt tcaaatcccg ccgtctccac 360
   ca 362
<210> 108
   <211> 362
   <212> RNA
   <213> Staphylococcus aureus
<400> 108
   ggggacguuc auggauucga cagggguccc ccgagcucau uaagcguguc ggaggguugu 60
   cuucgucauc aacacacaca guuuauaaua acuggcaaau caaacaauaa uuucgcagua 120
   gcugccuaau cgcacucugc aucgccuaac agcauuuccu augugcuguu aacgcgauuc 180
   aaccuuaaua ggauaugcua aacacugccg uuugaagucu guuuagaaga aacuuaauca 240
   aacuagcauc auguugguug uuuaucacuu uucaugaugc gaaaccuauc gauaaacuac 300
   acacguagaa agauguguau caggaccuuu ggacgcgggu ucaaaucccg ccgucuccac 360
   ca 362
<210> 109
   <211> 349
   <212> DNA
   <213> Streptococcus gordonii
<400> 109
   ggggtcgtta cggattcgac aggcattatg aggcatattt tgcgactcat ctagcggatg 60
   taaaacgcca gttaaatata actgcaaaaa ataatacttc ttacgcttta gctgcctaaa 120
   aaccagcggg cgtgacccga ttcggattgc ttgtgtctga tgacaggtct tattattagc 180
   aagctacggt agaatcttgt ctagtgattt tacaagagat tgatagactc gcttgatttg 240
   ggcttgagtt atgtgtcaaa atcaagttaa aacaatacat agcctatggt tgtagacaaa 300
   tgtgttggca gatgtttgga cgtgggttcg actcccaccg gctccacca 349
<210> 110
   <211> 349
   <212> RNA
   <213> Streptococcus gordonii
<400> 110
   ggggucguua cggauucgac aggcauuaug aggcauauuu ugcgacucau cuagcggaug 60
   uaaaacgcca guuaaauaua acugcaaaaa auaauacuuc uuacgcuuua gcugccuaaa 120
   aaccagcggg cgugacccga uucggauugc uugugucuga ugacaggucu uauuauuagc 180
   aagcuacggu agaaucuugu cuagugauuu uacaagagau ugauagacuc gcuugauuug 240
   ggcuugaguu augugucaaa aucaaguuaa aacaauacau agccuauggu uguagacaaa 300
   uguguuggca gauguuugga cguggguucg acucccaccg gcuccacca 349
<210> 111
   <211> 349
   <212> DNA
   <213> Streptococcus mutans
<400> 111
   ggggtcgtta cggattcgac aggcattatg agacctattt tgcgactcat ctagcggatg 60
   taaaacgcca gttaaatata actgcaaaaa atacaaattc ttacgcagta gctgcctaaa 120
   aaccagcctg tgtgatcaat aacaaattgc ttgtgtttgt tgattggtct tattgttaac 180
   aagctacgtt agaactgagt caggctgttc taaaagagtt ctactgactc gcatcgttag 240
   agtttgagtt atgtattgta acggtgttaa ataaacacat aacctatagt tgtagacaaa 300
   tgggttagca gatgtttgga cgtgggttcg actcccaccg gctccacca 349
<210> 112
   <211> 349
   <212> RNA
   <213> Streptococcus mutans
<400> 112
   ggggucguua cggauucgac aggcauuaug agaccuauuu ugcgacucau cuagcggaug 60
   uaaaacgcca guuaaauaua acugcaaaaa auacaaauuc uuacgcagua gcugccuaaa 120
   aaccagccug ugugaucaau aacaaauugc uuguguuugu ugauuggucu uauuguuaac 180
   aagcuacguu agaacugagu caggcuguuc uaaaagaguu cuacugacuc gcaucguuag 240
   aguuugaguu auguauugua acgguguuaa auaaacacau aaccuauagu uguagacaaa 300
   uggguuagca gauguuugga cguggguucg acucccaccg gcuccacca 349
<210> 113
   <211> 348
   <212> DNA
   <213> Streptococcus pneumoniae
<400> 113
   ggggtcgtta cggattcgac aggcattatg aggcatattt tgcgactcgt gtggcgacgt 60
   aaacgctcag ttaaatataa ctgcaaaaaa taacacttct tacgctctag ctgcctaaaa 120
   accagcaggc gtgacccgat ttggattgct cgtgttcaat gacaggtctt attattagcg 180
   agatacgatt aagccttgtc tagcggtttg ataagagatt gatagactcg cagtttctag 240
   acttgagtta tgtgtcgagg ggctgttaaa ataatacata acctatggtt gtagacaaat 300
   atgttggcag gtgtttggac gtgggttcga ctcccaccgg ctccacca             348
<210> 114
   <211> 348
   <212> RNA
   <213> Streptococcus pneumoniae
<400> 114
   ggggucguua cggauucgac aggcauuaug aggcauauuu ugcgacucgu guggcgacgu 60
   aaacgcucag uuaaauauaa cugcaaaaaa uaacacuucu uacgcucuag cugccuaaaa 120
   accagcaggc gugacccgau uuggauugcu cguguucaau gacaggucuu auuauuagcg 180
   agauacgauu aagccuuguc uagcgguuug auaagagauu gauagacucg caguuucuag 240
   acuugaguua ugugucgagg ggcuguuaaa auaauacaua accuaugguu guagacaaau 300
   auguuggcag guguuuggac guggguucga cucccaccgg cuccacca             348
<210> 115
   <211> 348
   <212> DNA
   <213> Streptococcus pyogenes
<400> 115
   ggggttgtta cggattcgac aggcattatg aggcatgttt tgcgtcccat cggcagatgt 60
   aaattgccag ttaaatataa ctgcaaaaaa tacaaactct tacgctttag ctgcctaaaa 120
   accagctagc gtgacttcta caagattgct tgtgtcctgt tagaagtctc aaaatagcaa 180
   gctacggtta cgaaattgtc tagtttcgtg acaagagatt gatagactcg caaactaatg 240
   gcttgagtta tgtgtcttta gtttgttaaa tgaagacata acctatggac gtagacaaat 300
   atgttggcag gtgtttggac gtgggttcga ctcccaccag ctccacca             348
<210> 116
   <211> 348
   <212> RNA
   <213> Streptococcus pyogenes
<400> 116
   gggguuguua cggauucgac aggcauuaug aggcauguuu ugcgucccau cggcagaugu 60
   aaauugccag uuaaauauaa cugcaaaaaa uacaaacucu uacgcuuuag cugccuaaaa 120
   accagcuagc gugacuucua caagauugcu uguguccugu uagaagucuc aaaauagcaa 180
   gcuacgguua cgaaauuguc uaguuucgug acaagagauu gauagacucg caaacuaaug 240
   gcuugaguua ugugucuuua guuuguuaaa ugaagacaua accuauggac guagacaaau 300
   auguuggcag guguuuggac guggguucga cucccaccag cuccacca             348
<210> 117
   <211> 394
   <212> DNA
   <213> Synechococcus sp. PCC6301
<400> 117
   ggggctgtaa tggtttcgac gtgttggtga atccttcacc gtgattcagg ccgagaggga 60
   gtccactctc gtaaatccag gctcaaccaa aagtaactgc gaacaacatc gttcctttcg 120
   ctcgtaaggc tgctcctgta gctgcttaaa cgccacaaac tttctggctc gagcgtctag 180
   tcgtagactc cgttaatacg cctagactta aacccccaac ggatgctcga gtggcggcct 240
   caggtccgtc ctctcgctaa gcaaaaacct gagcatcccg ccaacgggga taatcgttgg 300
   ctcccgcaca gtgggtcaac cgtgctaagc ctgtgaacga gcggaaagtt actagtcaat 360
   gcggacagcg gttcgattcc gctcagctcc acca 394
<210> 118
   <211> 394
   <212> RNA
   <213> Synechococcus sp. PCC6301
<400> 118
   ggggcuguaa ugguuucgac guguugguga auccuucacc gugauucagg ccgagaggga 60
   guccacucuc guaaauccag gcucaaccaa aaguaacugc gaacaacauc guuccuuucg 120
   cucguaaggc ugcuccugua gcugcuuaaa cgccacaaac uuucuggcuc gagcgucuag 180
   ucguagacuc cguuaauacg ccuagacuua aacccccaac ggaugcucga guggcggccu 240
   cagguccguc cucucgcuaa gcaaaaaccu gagcaucccg ccaacgggga uaaucguugg 300
   cucccgcaca gugggucaac cgugcuaagc cugugaacga gcggaaaguu acuagucaau 360
   gcggacagcg guucgauucc gcucagcucc acca 394
<210> 119
   <211> 399
   <212> DNA
   <213> Synechocystis PCC6803
<400> 119
   ggggccgcaa tggtttcgac aggttggcga aagcttgccc gtgatacagg tcgagagtga 60
   gtctcctctc gcaaatcaaa ggctcaaaaa aaagtaactg cgaataacat cgtcagcttc 120
   aaacgggtag ccatagcagc ctagtctgta aaagctacat tttcttgtca aagaccgttt 180
   acttcttttc tgactccgtt aaggattaga ggttaacccc aacggatgct ttgtttggct 240
   cttctctagt tagctaaaca atcaagactc agactagagc atcccaccat cagggataat 300
   cgatggtccc cgtcctaggg ctagaaggac taaacctgtg aatgagcgga aagttaatac 360
   ccagtttgga cagcagttca attctgctcg gctccacca 399
<210> 120
   <211> 399
   <212> RNA
   <213> Synechocystis PCC6803
<400> 120
   ggggccgcaa ugguuucgac agguuggcga aagcuugccc gugauacagg ucgagaguga 60
   gucuccucuc gcaaaucaaa ggcucaaaaa aaaguaacug cgaauaacau cgucagcuuc 120
   aaacggguag ccauagcagc cuagucugua aaagcuacau uuucuuguca aagaccguuu 180
   acuucuuuuc ugacuccguu aaggauuaga gguuaacccc aacggaugcu uuguuuggcu 240
   cuucucuagu uagcuaaaca aucaagacuc agacuagagc aucccaccau cagggauaau 300
   cgaugguccc cguccuaggg cuagaaggac uaaaccugug aaugagcgga aaguuaauac 360
   ccaguuugga cagcaguuca auucugcucg gcuccacca 399
<210> 121
   <211> 356
   <212> DNA
   <213> Thermotoga maritima
<400> 121
   gggggcgaac gggttcgacg gggatggagt cccctgggaa gcgagccgag gtccccacct 60
   cctcgtaaaa aaggtgggac aaagaataag tgccaacgaa cctgttgctg ttgccgctta 120
   atagataagc ggccgtcctc tccgaagttg gctgggcttc ggaagagggc gtgagagatc 180
   cagcctaccg attcagcttc gccttccggc ctgaatcggg aaaactcagg aaggctgtgg 240
   gagaggacac cctgcccgtg ggaggtccct cccgagagcg aaaacacggg ctgcgctcgg 300
   agaagcccag gggcctccat cttcggacgg gggttcgaat ccccccgcct ccacca 356
<210> 122
   <211> 356
   <212> RNA
   <213> Thermotoga maritima
<400> 122
   gggggcgaac ggguucgacg gggauggagu ccccugggaa gcgagccgag guccccaccu 60
   ccucguaaaa aaggugggac aaagaauaag ugccaacgaa ccuguugcug uugccgcuua 120
   auagauaagc ggccguccuc uccgaaguug gcugggcuuc ggaagagggc gugagagauc 180
   cagccuaccg auucagcuuc gccuuccggc cugaaucggg aaaacucagg aaggcugugg 240
   gagaggacac ccugcccgug ggaggucccu cccgagagcg aaaacacggg cugcgcucgg 300
   agaagcccag gggccuccau cuucggacgg ggguucgaau ccccccgccu ccacca 356
<210> 123
   <211> 349
   <212> DNA
   <213> Thermus thermophilus
<400> 123
   gggggtgaaa cggtctcgac gggggtcgcc gagggcgtgg ctgcgcgccg aggtgcgggt 60
   ggcctcgtaa aaacccgcaa cggcataact gccaacacca actacgctct cgcggcttaa 120
   tgaccgcgac ctcgcccggt agccctgccg ggggctcacc ggaagcgggg acacaaaccc 180
   ggctagcccg gggccacgcc ctctaacccc gggcgaagct tgaagggggc tcgctcctgg 240
   ccgcccgtcc gcgggccaag ccaggaggac acgcgaaacg cggactacgc gcgtagaggc 300
   ccgccgtaga gaccttcgga cgggggttcg actcccccca cctccacca 349
<210> 124
   <211> 349
   <212> RNA
   <213> Thermus thermophilus
<400> 124
   gggggugaaa cggucucgac gggggucgcc gagggcgugg cugcgcgccg aggugcgggu 60
   ggccucguaa aaacccgcaa cggcauaacu gccaacacca acuacgcucu cgcggcuuaa 120
   ugaccgcgac cucgcccggu agcccugccg ggggcucacc ggaagcgggg acacaaaccc 180
   ggcuagcccg gggccacgcc cucuaacccc gggcgaagcu ugaagggggc ucgcuccugg 240
   ccgcccgucc gcgggccaag ccaggaggac acgcgaaacg cggacuacgc gcguagaggc 300
   ccgccguaga gaccuucgga cggggguucg acucccccca ccuccacca 349
<210> 125
   <211> 354
   <212> DNA
   <213> Treponema pallidum
<400> 125
   ggggatgact aggtttcgac tagggatgtg gggtgttgcg ctgcaggtgg agtgtcgatc 60
   tcctgattcg gcgcctttat aactgccaat tctgacagtt tcgactacgc gctcgccgcg 120
   taatcgcggg cctgtgtttg cgctgctctg agcgaacata tcggcccgac gccaaacgga 180
   gcttgctctt acgttgtgca cggcggacgt agggggactt ttgtctgtgc taagactctg 240
   gcgcgtgcgg tgcaggccta gcagagtccg acaaacgcag tacgcaccgc taaacctgta 300
   ggcgcgcagc actcgctctt taggacgggg gttcgattcc ccccatctcc acca 354
<210> 126
   <211>354
   <212> RNA
   <213> Treponema pallidum
<400> 126
   ggggaugacu agguuucgac uagggaugug ggguguugcg cugcaggugg agugucgauc 60
   uccugauucg gcgccuuuau aacugccaau ucugacaguu ucgacuacgc gcucgccgcg 120
   uaaucgcggg ccuguguuug cgcugcucug agcgaacaua ucggcccgac gccaaacgga 180
   gcuugcucuu acguugugca cggcggacgu agggggacuu uugucugugc uaagacucug 240
   gcgcgugcgg ugcaggccua gcagaguccg acaaacgcag uacgcaccgc uaaaccugua 300
   ggcgcgcagc acucgcucuu uaggacgggg guucgauucc ccccaucucc acca 354
<210> 127
   <211> 367
   <212> DNA
   <213> Vibrio cholerae
<900> 127
<210> 128
   <211> 367
   <212> RNA
   <213> Vibrio cholerae
<400> 128
   ggggcugauu caggauucga cgggaauuuu gcagucugag gugcaugccg aggugcggua 60
   ggccucguua acaaaccgca aaaaaauagu cgcaaacgac gaaaacuacg cacuagcagc 120
   uuaauacccu gcucagagcc cuuccucccu agcuuccgcu uguaagacgg ggaaaucagg 180
   aaggucaaac caaaucaagc uggcguggau ucccccaccu gagggaugaa gcgcgagauc 240
   uaauucaggu uagccauucg uuagcguguc gguucgcagg cgguggugaa auuaaagauc 300
   gacuaagcau guaguaccaa agaugaaugg uuuucggacg gggguucaac uccccccagc 360
   uccacca 367
<210> 129
   <211> 364
   <212> DNA
   <213> Yersinia pestis
<400> 129
   ggggctgatt ctggattcga cgggattcgc gaaacccaag gtgcatgccg aggtgcggtg 60
   gcctcgtaaa aaaccgcaaa aaaaatagtt gcaaacgacg aaaactacgc actagcagct 120
   taataacctg cttagagccc tctctgccta gcctccgctc ttaggacggg gatcaagaga 180
   ggtcaaacct aaaagagctc gtgtggaaac cttgcctggg gtggaagcat taaaactaat 240
   caggatagtt tgtcagtagc gtgtccatcc gcagctggcc ggcgaatgta atgattggac 300
   taagcatgta gtgccgacgg tgtagtaatt tcggacgggg gttcaaatcc ccccagctcc 360
   acca 364
<210> 130
   <211> 364
   <212> RNA
   <213> Yersinia pestis
<400> 130
   ggggcugauu cuggauucga cgggauucgc gaaacccaag gugcaugccg aggugcggug 60
   gccucguaaa aaaccgcaaa aaaaauaguu gcaaacgacg aaaacuacgc acuagcagcu 120
   uaauaaccug cuuagagccc ucucugccua gccuccgcuc uuaggacggg gaucaagaga 180
   ggucaaaccu aaaagagcuc guguggaaac cuugccuggg guggaagcau uaaaacuaau 240
   caggauaguu ugucaguagc guguccaucc gcagcuggcc ggcgaaugua augauuggac 300
   uaagcaugua gugccgacgg uguaguaauu ucggacgggg guucaaaucc ccccagcucc 360
   acca 364
<210> 131
   <211> 309
   <212> DNA
   <213> Campylobacter fetus
<400> 131
   aggagtaagt ctgcttagat ggcatgtcgc tttgggcaaa gcgtaaaaag cccaaataaa 60
   attaaacgca aacaacgtta aattcgctcc tgcttacgct aaagctgcgt aagttcagtt 120
   gagcctgaaa tttaagtcat actatctagc ttaattttcg gtcatctttg atagtgtagc 180
   cttgcgtttg acaagcgttg aggtgaaata aagtcttagc cttgcttttg agttttggaa 240
   gatgagcgaa gtagggtgaa gtagtcatct ttgctaagca tgtagaggtc tttgtgggat 300
   tatttttgg 309
<210> 132
   <211> 309
   <212> RNA
   <213> Campylobacter fetus
<400> 132
   aggaguaagu cugcuuagau ggcaugucgc uuugggcaaa gcguaaaaag cccaaauaaa 60
   auuaaacgca aacaacguua aauucgcucc ugcuuacgcu aaagcugcgu aaguucaguu 120
   gagccugaaa uuuaagucau acuaucuagc uuaauuuucg gucaucuuug auaguguagc 180
   cuugcguuug acaagcguug aggugaaaua aagucuuagc cuugcuuuug aguuuuggaa 240
   gaugagcgaa guagggugaa guagucaucu uugcuaagca uguagagguc uuugugggau 300
   uauuuuugg 309
<210> 133
   <211> 309
   <212> DNA
   <213> Campylobacter coli (BM2509)
<400> 133
   aggagtaagt ctgcttagat ggcatgtcgc tttggacaaa gcgtaaaaag tccaaattaa 60
   aattaaacgc aaataacgtt aaatttgctc ctgcttacgc taaagctgcg taagttcagt 120
   tgagcccgaa actcaagtga tgctatctag cttgaatttt ggtcatcttt gatagtgtag 180
   attgaaaatt gacaactttt aatcgaagtt aaagtcttag tctagcttga aattttggaa 240
   ggtgagttta gccagatgaa gttttcacct ttgctaaaca tgtagaagtc tttgtggggt 300
   tatttttgg 309
<210>134
   <211> 309
   <212> RNA
   <213> Campylobacter coli (BM2509)
<400>134
   aggaguaagu cugcuuagau ggcaugucgc uuuggacaaa gcguaaaaag uccaaauuaa 60
   aauuaaacgc aaauaacguu aaauuugcuc cugcuuacgc uaaagcugcg uaaguucagu 120
   ugagcccgaa acucaaguga ugcuaucuag cuugaauuuu ggucaucuuu gauaguguag 180
   auugaaaauu gacaacuuuu aaucgaaguu aaagucuuag ucuagcuuga aauuuuggaa 240
   ggugaguuua gccagaugaa guuuucaccu uugcuaaaca uguagaaguc uuuguggggu 300
   uauuuuugg 309
<210> 135
   <211> 311
   <212> DNA
   <213> Unknown
<220>
   <223> Description of Unknown Organism:Campylobacter chicken isolate
<400> 135
   acaggagtaa gtctgcttag atggcatgtc gctttgggca aagcgtaaaa agcccaaata 60
   aaattaaacg caaacaacgt taaattcgct cctgcttacg ctaaagctgc gtaagttcag 120
   ttgagcctga aatttaagtc atactatcta gcttaatttt cggtcatttt tgatagtgta 180
   gccttgcgtt tgacaagcgt tgaggtgaaa taaggtctta gccttgcttt tgagttttgg 240
   aagatgagcg aagtagggtg aagtagtcat ctttgctaag catgtagagg tctttgtggg 300
   attatttttg g 311
<210> 136
   <211> 311
   <212> RNA
   <213> Unknown
<220>
   <223> Description of Unknown Organism:Campylobacter chicken isolate
<400> 136
   acaggaguaa gucugcuuag auggcauguc gcuuugggca aagcguaaaa agcccaaaua 60
   aaauuaaacg caaacaacgu uaaauucgcu ccugcuuacg cuaaagcugc guaaguucag 120
   uugagccuga aauuuaaguc auacuaucua gcuuaauuuu cggucauuuu ugauagugua 180
   gccuugcguu ugacaagcgu ugaggugaaa uaaggucuua gccuugcuuu ugaguuuugg 240
   aagaugagcg aaguagggug aaguagucau cuuugcuaag cauguagagg ucuuuguggg 300
   auuauuuuug g 311
<210> 137
   <211> 313
   <212> DNA
   <213> Clostridium perfringens
<400> 137
   acgggggtag gatgggtttg ataagcgagt cgagggaagc atggtgcctc gataataaag 60
   tatgcattaa agataaacgc acgagataat tttgcattag cagcttaagt tagcgctgct 120
   catccttcct caattgccca cggttgagag taagggtgtc atttaaaagt ggggaaccga 180
   gcctagcaaa gctttgagct aggaacggaa tttatgaagc ttaccaaaga ggaagtttgt 240
   ctgtggacgt tctctgaggg aattttaaaa cacaagacta cactcgtaga aagtcttact 300
   ggtctgcttt cgg                                                    313
<210> 138
   <211> 313
   <212> RNA
   <213> Clostridium perfringens
<400> 138
   acggggguag gauggguuug auaagcgagu cgagggaagc auggugccuc gauaauaaag 60
   uaugcauuaa agauaaacgc acgagauaau uuugcauuag cagcuuaagu uagcgcugcu 120
   cauccuuccu caauugccca cgguugagag uaaggguguc auuuaaaagu ggggaaccga 180
   gccuagcaaa gcuuugagcu aggaacggaa uuuaugaagc uuaccaaaga ggaaguuugu 240
   cuguggacgu ucucugaggg aauuuuaaaa cacaagacua cacucguaga aagucuuacu 300
   ggucugcuuu cgg                                                    313
<210> 139
   <211> 331
   <212> DNA
   <213> Haemophilus ducreyi (NCTC 10945)
<400> 139
   acgggattag cgaagtccaa ggtgcacgtc gaggtgcggt aggcctcgta acaaaccgca 60
   aaaaaatagt cgcaaacgac gaacaatacg ctttagcagc ttaataacct gcatttagcc 120
   ttcgcgccct agctttcgct cgtaagacgg ggagcacgcg gagtcaaacc aaaacgagat 180
   cgtgtggacg cttccgcttg tagatgaaac actaaattga atcaagctag tttatttctt 240
   gcgtgtctgt ccgctggaga taagcgaaat taaagaccag actaaacgtg tagtactgaa 300
   gatagagtaa tttcggaccc gggttcgact c                                331
<210> 140
   <211> 331
   <212> RNA
   <213> Haemophilus ducreyi (NCTC 10945)
<400> 140
   acgggauuag cgaaguccaa ggugcacguc gaggugcggu aggccucgua acaaaccgca 60
   aaaaaauagu cgcaaacgac gaacaauacg cuuuagcagc uuaauaaccu gcauuuagcc 120
   uucgcgcccu agcuuucgcu cguaagacgg ggagcacgcg gagucaaacc aaaacgagau 180
   cguguggacg cuuccgcuug uagaugaaac acuaaauuga aucaagcuag uuuauuucuu 240
   gcgugucugu ccgcuggaga uaagcgaaau uaaagaccag acuaaacgug uaguacugaa 300
   gauagaguaa uuucggaccc ggguucgacu c                                331
<210> 141
   <211> 232
   <212> DNA
   <213> Listeria innocua (food isolate #1)
<400> 141
   ggcaaagaaa aacaaaacct agctttcgct gcctaataac cagtagcata gctgatcctc 60
   cgtgcatcgc ccatgtgcta cggtaagggt ctcactctaa gtgggctaca ctagttaatc 120
   tccgtctgag gttaaataga agagcttaat cagactagct gaatggaagc ctgttaccgg 180
   gctgatgttt atgcgaaatg ctaatacggt gactacgctc gtagatattc aa 232
<210> 142
   <211> 232
   <212> RNA
   <213> Listeria innocua (food isolate #1)
<400> 142
   ggcaaagaaa aacaaaaccu agcuuucgcu gccuaauaac caguagcaua gcugauccuc 60
   cgugcaucgc ccaugugcua cgguaagggu cucacucuaa gugggcuaca cuaguuaauc 120
   uccgucugag guuaaauaga agagcuuaau cagacuagcu gaauggaagc cuguuaccgg 180
   gcugauguuu augcgaaaug cuaauacggu gacuacgcuc guagauauuc aa 232
<210> 143
   <211> 232
   <212> DNA
   <213> Listeria innocua (food isolate #2)
<400> 143
   ggcaaagaaa aacaaaacct agctttcgct gcctaataag cagtagcata gctgatcctc 60
   cgtgcatcgc ccatgtgcta cggtaagggt ctcactctaa gtgggctaca ctagttaatc 120
   tccgtctgag gttaaataga agagcttaat cagactagct gaatggaagc ctgttaccgg 180
   gccgatgttt atgcgaaatg ctaatacggt gactacgctc gtagatattt aa 232
<210> 144
   <211> 232
   <212> RNA
   <213> Listeria innocua (food isolate #2)
<400> 144
   ggcaaagaaa aacaaaaccu agcuuucgcu gccuaauaag caguagcaua gcugauccuc 60
   cgugcaucgc ccaugugcua cgguaagggu cucacucuaa gugggcuaca cuaguuaauc 120
   uccgucugag guuaaauaga agagcuuaau cagacuagcu gaauggaagc cuguuaccgg 180
   gccgauguuu augcgaaaug cuaauacggu gacuacgcuc guagauauuu aa 232
<210> 145
   <211> 232
   <212> DNA
   <213> Listeria innocua (food isolate #3)
<400> 145
   ggcaaagaaa aacaaaacct agctttcgct gcctaataag cagtagaata gctgatcctc 60
   cgtgcatcgc ccatgtgcta cggtaagggt ctcactctaa gtgggctaca ctagttaatc 120
   tccgtctgag gttaaataga agagcttaat cggactagct gaatggaagc ctgttaccgg 180
   gccgatgttt atgcgaaatg ctaatacggt gactacgctc gtagatattt aa 232
<210> 146
   <211> 232
   <212> RNA
   <213> Listeria innocua (food isolate #3)
<400> 146
   ggcaaagaaa aacaaaaccu agcuuucgcu gccuaauaag caguagaaua gcugauccuc 60
   cgugcaucgc ccaugugcua cgguaagggu cucacucuaa gugggcuaca cuaguuaauc 120
   uccgucugag guuaaauaga agagcuuaau cggacuagcu gaauggaagc cuguuaccgg 180
   gccgauguuu augcgaaaug cuaauacggu gacuacgcuc guagauauuu aa 232
<210> 147
   <211> 232
   <212> DNA
   <213> Listeria innocua (ATCC 12210)
<400> 147
   ggcaaagaaa aacaaaacct agctttcgct gcctaataag cagtagcata gctgatcctc 60
   cgtgcatcgc ccatgtgcta cggtaagggt ctcactctaa gtgggctaca ctagttaatc 120
   tccgtctggg gttaaataga agagcttaat cagactagct gaatggaagc ctgttactgg 180
   gccgatgttt atgcgaaatg ctaatacggt gactacgctc gtagatattt aa 232
<210> 148
   <211> 232
   <212> RNA
   <213> Listeria innocua (ATCC 12210)
<400> 148
   ggcaaagaaa aacaaaaccu agcuuucgcu gccuaauaag caguagcaua gcugauccuc 60
   cgugcaucgc ccaugugcua cgguaagggu cucacucuaa gugggcuaca cuaguuaauc 120
   uccgucuggg guuaaauaga agagcuuaau cagacuagcu gaauggaagc cuguuacugg 180
   gccgauguuu augcgaaaug cuaauacggu gacuacgcuc guagauauuu aa 232
<210> 149
   <211> 322
   <212> DNA
   <213> Listeria ivanovii (NCTC 11846)
<400> 149
   acagggatag ttcgagcttg agttgcgagt cggggggatc gtcctcgtta ttaacgtcaa 60
   agccaataat aactggcaaa gaaaaacaaa acctagcttt cgctgcctaa taagcagtag 120
   catagctgat cctccgtgca tcgcccatgt gctacggtaa gggtctcact ttaagtgggc 180
   tacactaaat aatctccgtc tggggttagt tagaagagct taatcagact agctgaatgg 240
   aagcctgtta ccgggctgat gtttatgcga aatgctaata cggtgactac gctcgtagat 300
   atttaagtgc cgatatttct gg 322
<210> 150
   <211> 321
   <212> RNA
   <213> Listeria ivanovii (NCTC 11846)
<400> 150
   acagggauag uucgagcuug aguugcgagu cggggggauc guccucguua uuaacgucaa 60
   agccaauaau aacuggcaaa gaaaaacaaa accuagcuuu cgcugccuaa uaagcaguag 120
   cauagcugau ccuccgugca ucgcccaugu gcuacgguaa gggucucacu uuaagugggc 180
   uacacuaaau aaucuccguc ugggguuagu uagaagagcu uaaucagacu agcugaaugg 240
   aagccuguua ccgggcugau guuuaugcga aaugcuaaua cggugacucg cucguagaua 300
   uuuaagugcc gauauuucug g 321
<210> 151
   <211> 322
   <212> DNA
   <213> Listeria seeligeri (NCTC 11856)
<400> 151
   acagggatag ttcgagcttg agttgcgagt cggggggatc gtcctcgtta tcaacgtcaa 60
   agccaataat aactggcaaa gaaaaacaaa acctagcttt cgctgcctaa taagcagtag 120
   catagctgat cctccgtgca tcgcccatgt gctacggaaa gggtctcact ttaagtgggc 180
   tacactaaat aatctccgtc tggggttagt tagaagagct taatcagact agctgaatgg 240
   aagcctgtta ccgggctgat gtttatgcga aatactaata cggtgactac gctcgtagat 300
   atttaagtgc ccatatttct gg 322
<210> 152
   <211> 322
   <212> RNA
   <213> Listeria seeligeri (NCTC 11856)
<400> 152
   acagggauag uucgagcuug aguugcgagu cggggggauc guccucguua ucaacgucaa 60
   agccaauaau aacuggcaaa gaaaaacaaa accuagcuuu cgcugccuaa uaagcaguag 120
   cauagcugau ccuccgugca ucgcccaugu gcuacggaaa gggucucacu uuaagugggc 180
   uacacuaaau aaucuccguc ugggguuagu uagaagagcu uaaucagacu agcugaaugg 240
   aagccuguua ccgggcugau guuuaugcga aauacuaaua cggugacuac gcucguagau 300
   auuuaagugc ccauauuucu gg 322
<210> 153
   <211> 314
   <212> DNA
   <213> Salmonella enteritidis
<400> 153
   acgggatttg cgaaacccaa ggtgcatgcc gaggggcggt tggcctcgta aaaagccgca 60
   aaaaaatagt cgcaaacgac gaaacctacg ctttagcagc ttaataacct gcttagagcc 120
   ctctctccct agcctccgct cttaggacgg ggatcaagag aggtcaaacc caaaagagat 180
   cgcgtggatg ccctgcctgg ggttgaagcg ttaaaacgaa tcaggctagt ctggtagtgg 240
   cgtgtccgtc cgcaggtgcc aggcgaatgt aaagactgac taagcatgta gtaccgagga 300
   tgtaggaatt tcgg                                                   314
<210> 154
   <211> 314
   <212> RNA
   <213> Salmonella enteritidis
<400> 154
   acgggauuug cgaaacccaa ggugcaugcc gaggggcggu uggccucgua aaaagccgca 60
   aaaaaauagu cgcaaacgac gaaaccuacg cuuuagcagc uuaauaaccu gcuuagagcc 120
   cucucucccu agccuccgcu cuuaggacgg ggaucaagag aggucaaacc caaaagagau 180
   cgcguggaug cccugccugg gguugaagcg uuaaaacgaa ucaggcuagu cugguagugg 240
   cguguccguc cgcaggugcc aggcgaaugu aaagacugac uaagcaugua guaccgagga 300
   uguaggaauu ucgg                                                   314
<210> 155
   <211> 313
   <212> DNA
   <213> Staphylococcus epidermidis (NCTC 11047)
<400> 155
   acaggggtcc cccgagctta ttaagcgtgt cggagggttg gctccgtcat caacacattt 60
   cggttaaata taactgacaa atcaaacaat aatttcgcag tagctgcgta atagccactg 120
   catcgcctaa cagcatctcc tacgtgctgt taacgcgatt caaccctagt aggatatgct 180
   aaacactgcc gcttgaagtc tgtttagatg aaatataatc aagctagtat catgttggtt 240
   gtttattgct tagcatgatg cgaaaattat caataaacta cacacgtaga aagatttgta 300
   tcaggacctc tgg                                                    313
<210> 156
   <211> 313
   <212> RNA
   <213> Staphylococcus epidermidis (NCTC 11047)
<400> 156
   acaggggucc cccgagcuua uuaagcgugu cggaggguug gcuccgucau caacacauuu 60
   cgguuaaaua uaacugacaa aucaaacaau aauuucgcag uagcugcgua auagccacug 120
   caucgccuaa cagcaucucc uacgugcugu uaacgcgauu caacccuagu aggauaugcu 180
   aaacacugcc gcuugaaguc uguuuagaug aaauauaauc aagcuaguau cauguugguu 240
   guuuauugcu uagcaugaug cgaaaauuau caauaaacua cacacguaga aagauuugua 300
   ucaggaccuc ugg                                                    313
<210> 157
   <211> 302
   <212> DNA
   <213> Streptococcus agalactiae (NCTC 8181)
<400> 157
   acaggcatta tgaggtatat tttgcgactc atcggcagat gtaaaatgcc agttaaatat 60
   aactgcaaaa aatacaaatt cttacgcatt agctgcctaa aaaacagcct gcgtgatctt 120
   cacaagattg tttgcgtttt gctagaaggt cttatttatc agcaaactac gtttggctac 180
   tgtctagtta gttaaaaaga gatttataga ctcgctatgt gagggcttga gttatgtgtc 240
   atcacctagt taaatcaata cataacctat agttgtagac aaatatatta gcagatgttt 300
   gg                                                                302
<210> 158
   <211> 302
   <212> RNA
   <213> Streptococcus agalactiae (NCTC 8181)
<400> 158
   acaggcauua ugagguauau uuugcgacuc aucggcagau guaaaaugcc aguuaaauau 60
   aacugcaaaa aauacaaauu cuuacgcauu agcugccuaa aaaacagccu gcgugaucuu 120
   cacaagauug uuugcguuuu gcuagaaggu cuuauuuauc agcaaacuac guuuggcuac 180
   ugucuaguua guuaaaaaga gauuuauaga cucgcuaugu gagggcuuga guuauguguc 240
   aucaccuagu uaaaucaaua cauaaccuau aguuguagac aaauauauua gcagauguuu 300
   gg                                                                302
<210> 159
   <211> 168
   <212> DNA
   <213> Bordetella bronchiseptica
<400>159
   ggggccgatc cggattcgac gtgggtcatg aaacagctca aggcatgccg agcaccagta 60
   agctcgttaa tccactggaa cactacaaac gccaacgacg agcgtttcgc tctcgccgct 120
   taagcggtga gccgctgcac tgatctgtcc ttgggtcacg cgggggaa              168
<210> 160
   <211> 168
   <212> RNA
   <213> Bordetella bronchiseptica
<400> 160
   ggggccgauc cggauucgac gugggucaug aaacagcuca aggcaugccg agcaccagua 60
   agcucguuaa uccacuggaa cacuacaaac gccaacgacg agcguuucgc ucucgccgcu 120
   uaagcgguga gccgcugcac ugaucugucc uugggucacg cgggggaa              168
<210> 161
   <211> 426
   <212> DNA
   <213> Chlamydia pneumoniae (CWL029)
<400> 161
   gggggtgtat aggtttcgac ttgaaaatga agtgttaatt gcatgcggag ggcgttggct 60
   ggcctcctaa aaagccaaca aaacaataaa tgccgaacct aaggctgaat gcgaaattat 120
   tagcttgttt gactcagtag aggaaagact agctgcttaa ttagcaaaag ttgttagcta 180
   gataatctct aggtaacccg gtatctgcga gctccaccag aggcttgcaa aataccgtca 240
   tttatctggt tggaacttac tttctctaat tctcaaggaa gttcgttcga gatttttgag 300
   agtcattggc tgctatagag gcttctagct aagggagtcc aatgtaaaca attctagaag 360
   ataagcatgt agaggttagc agggagtttg tcaaggacga gagttcgagt ctctccacct 420
   ccacca 426
<210> 162
   <211> 426
   <212> RNA
   <213> Chlamydia pneumoniae (CWL029)
<400> 162
   ggggguguau agguuucgac uugaaaauga aguguuaauu gcaugcggag ggcguuggcu 60
   ggccuccuaa aaagccaaca aaacaauaaa ugccgaaccu aaggcugaau gcgaaauuau 120
   uagcuuguuu gacucaguag aggaaagacu agcugcuuaa uuagcaaaag uuguuagcua 180
   gauaaucucu agguaacccg guaucugcga gcuccaccag aggcuugcaa aauaccguca 240
   uuuaucuggu uggaacuuac uuucucuaau ucucaaggaa guucguucga gauuuuugag 300
   agucauuggc ugcuauagag gcuucuagcu aagggagucc aauguaaaca auucuagaag 360
   auaagcaugu agagguuagc agggaguuug ucaaggacga gaguucgagu cucuccaccu 420
   ccacca 426
<210> 163
   <211> 421
   <212> DNA
   <213> Francisella tularensis
<400> 163
   gggggcgaat atggtttcga catgaatgtc aaaatctaag gtgcatgccg aggaagtacc 60
   gtaacctcgt taataacagt acaaatgcca ataataactg gcaacaaaaa agcaaaccgc 120
   gtagcggcta acgacagcaa ctttgctgct gttgctaaag ctgcctagtc tagcttaata 180
   atctagatgc gcacggatat gatagtcttt cttatgacac tatctataca tccgttcata 240
   ttccgcataa gacggtcttt gctttttgtc tgggagttaa ggctgtattt aacagactcg 300
   ctaactatta ccctggctaa ttggggaata gtcaagctaa actcaaatag attagcctaa 360
   gcatgtagat ccaaagatct agagtttgtg gacgcgggtt caaatcccgc cgcctccacc 420
   a 421
<210> 164
   <211> 421
   <212> RNA
   <213> Francisella tularensis
<900> 164
<210> 165
   <211> 330
   <212> DNA
   <213> Guillardia theta (plastid)
<400> 165
   ggggctgatt tggattcgac atataaattt gcgtgtttca ttatgaagca agtcaagttt 60
   aatgatcttg taaaaaacat taaagtacaa ataaatgcaa gcaatatagt ttcatttagt 120
   tcaaaacgtt tagtctcttt tgcataagca aaatgtgtta ataactttct tagtagaaat 180
   tggagaagtt tactaagatt tatatttact ccataattat tttaaagatg gtaaaaaggt 240
   gattcatcat ttgtatgttt ctaaactttg tgaaagaata gtgggctcca tttataatga 300
   acgtgggttc aaatcccacc agctccacca 330
<210> 166
   <211> 330
   <212> RNA
   <213> Guillardia theta (plastid)
<400> 166
   ggggcugauu uggauucgac auauaaauuu gcguguuuca uuaugaagca agucaaguuu 60
   aaugaucuug uaaaaaacau uaaaguacaa auaaaugcaa gcaauauagu uucauuuagu 120
   ucaaaacguu uagucucuuu ugcauaagca aaauguguua auaacuuucu uaguagaaau 180
   uggagaaguu uacuaagauu uauauuuacu ccauaauuau uuuaaagaug guaaaaaggu 240
   gauucaucau uuguauguuu cuaaacuuug ugaaagaaua gugggcucca uuuauaauga 300
   acguggguuc aaaucccacc agcuccacca 330
<210> 167
   <211> 348
   <212> DNA
   <213> Thalassiosira Weissflogii (plastid)
<400> 167
   ggggctgatt tggtttcgac atttaaaact tctttctatg tgtcaggtca aagtttgtat 60
   tctttgtaaa aaaatactaa aatactaata aatgctaata atataatacc gtttattttt 120
   aaagcagtaa aaacaaaaaa agaagcaatg gctttaaatt ttgctgtata gttcattaac 180
   ttaggttatt aaatattttt tcattataac tggacttttc tctagtttat agtttagaat 240
   aaatttaaat tttgcaaaac tcgttcgaaa attttcgggc taaacctgta aacgcaaata 300
   ctaagaaatt ttagatggac atgggttcaa ttcccatcag ttccacca 348
<210> 168
   <211> 348
   <212> RNA
   <213> Thalassiosira Weissflogii (plastid)
<900> 168
<210> 169
   <211> 19
   <212> DNA
   <213> Artificial Sequence
<220>
   <223> Description of Artificial Sequence:V. cholerae tmRNA specific probe
<400> 169
   aacgaatggc taacctgaa 19
<210> 170
   <211> 17
   <212> DNA
   <213> Artificial Sequence
<220>
   <223> Description of Artificial Sequence:Universal ssrA/tmRNA 5' in vitro amplification primer
<400> 170
   gggmytacgg wttcgac 17
<210> 171
   <211> 18
   <212> DNA
   <213> Artificial Sequence
<220>
   <223> Description of Artificial Sequence:Universal ssrA/tmRNA 3' in vitro amplification primer
<400> 171
   gggartcgaa ccrsgtcc 18
<210> 172
   <211> 18
   <212> DNA
   <213> Artificial Sequence
<220>
   <223> Description of Artificial Sequence:5' Listeria genus specific PCR amplification primer
<400> 172
   aaagccaata ataactgg 18
<210> 173
   <211> 18
   <212> DNA
   <213> Artificial Sequence
<220>
   <223> Description of Artificial Sequence:3' Listeria genus specific amplification primer
<400> 173
   ccagaaatat cggcactt 18
<210> 174
   <211> 18
   <212> DNA
   <213> Artificial Sequence
<220>
   <223> Description of Artificial Sequence:Listeria genus specific hybridisation probe
<400> 174
   gtgagaccct taccgtag 18
<210> 175
   <211> 18
   <212> DNA
   <213> Artificial Sequence
<220>
   <223> Description of Artificial Sequence:Listeria monocytogenes species specific hybridisation probe
<400> 175
   tctatttaac cccagacg 18
<210> 176
   <211> 348
   <212> DNA
   <213> Helicobacter pylori
<400> 176
   tggggatgtt acggtttcga caggggtagt tcgagcttag gtggcgagtc gaggggatcg 60
   gcctcgttaa aacgtcaaag cctataactg gcaaacaaca aaacaacttc gctttagcag 120
   cttaataagc tcttagcggt tcctccctcc atcgcccatg tggtagggta agggactcaa 180
   attaagtggg ctacgctgga ttccaccgtc tgaggatgaa agaagagaac aaccagacta 240
   gctacccgga cgcccgtcga taggcagatg gagtagcgaa tcgcgaatat atcgactaca 300
   ctcgtagaag cttaagtgcc gatattcttg gacgtgggtt cgactccc             348
<210> 177
   <211> 348
   <212> RNA
   <213> Helicobacter pylori
<400> 177
   uggggauguu acgguuucga cagggguagu ucgagcuuag guggcgaguc gaggggaucg 60
   gccucguuaa aacgucaaag ccuauaacug gcaaacaaca aaacaacuuc gcuuuagcag 120
   cuuaauaagc ucuuagcggu uccucccucc aucgcccaug ugguagggua agggacucaa 180
   auuaaguggg cuacgcugga uuccaccguc ugaggaugaa agaagagaac aaccagacua 240
   gcuacccgga cgcccgucga uaggcagaug gaguagcgaa ucgcgaauau aucgacuaca 300
   cucguagaag cuuaagugcc gauauucuug gacguggguu cgacuccc             348
<210> 178
   <211> 344
   <212> DNA
   <213> Helicobacter pylori
<400> 178
   tggggacgtt acggtttcga cagggatagt tcgagcttag gttgcgagtc gaggggatcg 60
   gcctcgttaa aacgtcaaag cctataattg gcaaacaaaa caatctttct ttagctgctt 120
   aattgcacta aaggttcctc cctccatcgt ccatgtggta gggtaaggga ctcaaactaa 180
   gtggactacg ccggagttcg ccgtctgagg acaaaggaag agaacaacca gactagcaac 240
   ttggaagcct gtcgataggc cgaagagttc gcgaaatgct aatatatcga ctacactcgt 300
   agaagcttaa gtgccgatat ttttggacgt gggttcgatt ccct                  344
<210> 179
   <211> 344
   <212> RNA
   <213> Helicobacter pylori
<400> 179
   uggggacguu acgguuucga cagggauagu ucgagcuuag guugcgaguc gaggggaucg 60
   gccucguuaa aacgucaaag ccuauaauug gcaaacaaaa caaucuuucu uuagcugcuu 120
   aauugcacua aagguuccuc ccuccaucgu ccauguggua ggguaaggga cucaaacuaa 180
   guggacuacg ccggaguucg ccgucugagg acaaaggaag agaacaacca gacuagcaac 240
   uuggaagccu gucgauaggc cgaagaguuc gcgaaaugcu aauauaucga cuacacucgu 300
   agaagcuuaa gugccgauau uuuuggacgu ggguucgauu cccu                  344
<210> 180
   <211> 322
   <212> DNA
   <213> Listeria seeligeri (NCTC 11856)
<400> 180
   acagggatag ttcgagcttg agttgcgagt cggggggatc gtcctcgtta tcaacgtcaa 60
   agccaataat aactggcaaa gaaaaacaaa acctagcttt cgctgcctaa taagcagtag 120
   catagctgat cctccgtgca tcgcccatgt gctacggaaa gggtctcact ttaagtgggc 180
   tacactaaat aatctccgtc tggggttagt tagaagagct taatcagact agctgaatgg 240
   aagcctgtta ccgggctgat gtttatgcga aatactaata cggtgactac gctcgtagat 300
   atttaagtgc ccatatttct gg                                          322
<210> 181
   <211> 322
   <212> RNA
   <213> Listeria seeligeri (NCTC 11856)
<400> 181
   acagggauag uucgagcuug aguugcgagu cggggggauc guccucguua ucaacgucaa 60
   agccaauaau aacuggcaaa gaaaaacaaa accuagcuuu cgcugccuaa uaagcaguag 120
   cauagcugau ccuccgugca ucgcccaugu gcuacggaaa gggucucacu uuaagugggc 180
   uacacuaaau aaucuccguc ugggguuagu uagaagagcu uaaucagacu agcugaaugg 240
   aagccuguua ccgggcugau guuuaugcga aauacuaaua cggugacuac gcucguagau 300
   auuuaagugc ccauauuucu gg                                          322
<210> 182
   <211> 322
   <212> DNA
   <213> Listeria ivanovii (NCTC 11846)
<400> 182
   acagggatag ttcgagcttg agttgcgagt cggggggatc gtcctcgtta ttaacgtcaa 60
   agccaataat aactggcaaa gaaaaacaaa acctagcttt cgctgcctaa taagcagtag 120
   catagctgat cctccgtgca tcgcccatgt gctacggtaa gggtctcact ttaagtgggc 180
   tacactaaat aatctccgtc tggggttagt tagaagagct taatcagact agctgaatgg 240
   aagcctgtta ccgggctgat gtttatgcga aatgctaata cggtgactac gctcgtagat 300
   atttaagtgc cgatatttct gg                                          322
<210> 183
   <211> 321
   <212> RNA
   <213> Listeria ivanovii (NCTC 11846)
<400> 183
   acagggauag uucgagcuug aguugcgagu cggggggauc guccucguua uuaacgucaa 60
   agccaauaau aacuggcaaa gaaaaacaaa accuagcuuu cgcugccuaa uaagcaguag 120
   cauagcugau ccuccgugca ucgcccaugu gcuacgguaa gggucucacu uuaagugggc 180
   uacacuaaau aaucuccguc ugggguuagu uagaagagcu uaaucagacu agcugaaugg 240
   aagccuguua ccgggcugau guuuaugcga aaugcuaaua cggugacucg cucguagaua 300
   uuuaagugcc gauauuucug g                                           321
<210> 184
   <211> 319
   <212> DNA
   <213> Mycobacterium africanum
<400> 184
   acttcgcgca tcgaatcaag ggaagcgtgc cggtgcaggc aagagaccac cgtaagcgtc 60
   gttgcgacca aataagcgcc gattcacatc agcgcgacta cgctctcgct gcctaagcga 120
   cggctagtct gtcagaccgg gaacgccctc ggcccggacc ctggcatcag ctagagggat 180
   ccaccgatga gtccggtcgc gggactcctc gggacaacca cagcgactgg gatcgtcatc 240
   tcggctagtt cgcgtgaccg ggagatccga gcagaggcat agcgaactgc gcacggagaa 300
   gccttgaggg aatgccgta                                              319
<210> 185
   <211> 319
   <212> RNA
   <213> Mycobacterium africanum
<400> 185
   acuucgcgca ucgaaucaag ggaagcgugc cggugcaggc aagagaccac cguaagcguc 60
   guugcgacca aauaagcgcc gauucacauc agcgcgacua cgcucucgcu gccuaagcga 120
   cggcuagucu gucagaccgg gaacgcccuc ggcccggacc cuggcaucag cuagagggau 180
   ccaccgauga guccggucgc gggacuccuc gggacaacca cagcgacugg gaucgucauc 240
   ucggcuaguu cgcgugaccg ggagauccga gcagaggcau agcgaacugc gcacggagaa 300
   gccuugaggg aaugccgua                                              319
<210> 186
   <211> 319
   <212> DNA
   <213> Mycobacterium gordonae
<400> 186
   acttcgcgca tcgaatcaag ggaagcgtgc cggtgcaggc aagagaccac cgtaagcgtc 60
   gttgcaacca tataagcgcc gattcacatc agcgcgacta cgctctcgct gcctaagcga 120
   cggctagtct gtcggaccgg gaacgccctc gccccggacc ccggcatcag ctagagggat 180
   caaccgatga gttcggtcgc gggactcatc gggacaccaa cagcgactgg gatcgtcatc 240
   ctggctagtc cgtgtgacca ggagatccga gcagagacat agcggactgc gcacggagaa 300
   gccttgaggg aatgccgta 319
<210> 187
   <211> 319
   <212> RNA
   <213> Mycobacterium gordonae
<400> 187
   acuucgcgca ucgaaucaag ggaagcgugc cggugcaggc aagagaccac cguaagcguc 60
   guugcaacca uauaagcgcc gauucacauc agcgcgacua cgcucucgcu gccuaagcga 120
   cggcuagucu gucggaccgg gaacgcccuc gccccggacc ccggcaucag cuagagggau 180
   caaccgauga guucggucgc gggacucauc gggacaccaa cagcgacugg gaucgucauc 240
   cuggcuaguc cgugugacca ggagauccga gcagagacau agcggacugc gcacggagaa 300
   gccuugaggg aaugccgua 319
<210> 188
   <211> 319
   <212> DNA
   <213> Mycobacterium kansasii
<400> 188
   acttcgcgca tcgaatcaag ggaagcgtgc cggtgcaggc aagagaccac cgtaagcgtc 60
   gttgcaacca aataagcgcc gattcacatc agcgcgacta cgctctcgct gcctaagcga 120
   cggctagtct gtcagaccgg gaccgccctc gacccggact ctggcatcag ctagagggat 180
   caaccgatga gttcggtcgc gggactcgtc gggacaccaa cagcgactgg gatcgtcatc 240
   ctggctagtt cgcgtgacca ggagatccga gcagaggcat agcgaactgc gcacggagaa 300
   gccttgaggg aatgccgta 319
<210> 189
   <211> 319
   <212> RNA
   <213> Mycobacterium kansasii
<400> 189
   acuucgcgca ucgaaucaag ggaagcgugc cggugcaggc aagagaccac cguaagcguc 60
   guugcaacca aauaagcgcc gauucacauc agcgcgacua cgcucucgcu gccuaagcga 120
   cggcuagucu gucagaccgg gaccgcccuc gacccggacu cuggcaucag cuagagggau 180
   caaccgauga guucggucgc gggacucguc gggacaccaa cagcgacugg gaucgucauc 240
   cuggcuaguu cgcgugacca ggagauccga gcagaggcau agcgaacugc gcacggagaa 300
   gccuugaggg aaugccgua 319
<210> 190
   <211> 320
   <212> DNA
   <213> Mycobacterium chelonae
<400> 190
   acagcgagtc tcgacttaag ggaagcgtgc cggtgcaggc aagagaccac cgtaagcgtc 60
   attgcaacca attaagcgcc gattctcatc agcgcgacta cgcactcgct gcctaagcga 120
   ctgcgtgtct gtcagaccgg gagcgccctc agcccggacc ctggcatcag ctagagggac 180
   aaactacggg ttcggtcgcg ggacccgtag ggacatcaaa cagcgactgg gatcgtcatc 240
   tcggcttgtt cgcgggaccg agagatccaa gtagaggcat agcgaactgc gcacggagaa 300
   gccttaatga acggccgttg 320
<210> 191
   <211> 320
   <212> RNA
   <213> Mycobacterium chelonae
<400> 191
   acagcgaguc ucgacuuaag ggaagcgugc cggugcaggc aagagaccac cguaagcguc 60
   auugcaacca auuaagcgcc gauucucauc agcgcgacua cgcacucgcu gccuaagcga 120
   cugcgugucu gucagaccgg gagcgcccuc agcccggacc cuggcaucag cuagagggac 180
   aaacuacggg uucggucgcg ggacccguag ggacaucaaa cagcgacugg gaucgucauc 240
   ucggcuuguu cgcgggaccg agagauccaa guagaggcau agcgaacugc gcacggagaa 300
   gccuuaauga acggccguug 320
<210> 192
   <211> 320
   <212> DNA
   <213> Mycobacterium szulgai
<400> 192
   acttcgcgca tcgaatcaag ggaagcgtgc cggtgcaggc aagagaccac cgtaagcgtc 60
   gttgcaacca attaagcgcc gagaacactc agcgcgactt cgctctcgct gcctaagcga 120
   cagcaagtcc gtcagaccgg gaaagccctc gacccggacc ctggcgtcat ctagagggat 180
   ccaccggtga gttcggtcgc gggactcatc gggacaccaa cagcgactgg gatcgtcatc 240
   ctggctagtt cgcgtgacca ggagatccga gtagagacat agcgaactgc gcacggagaa 300
   gccttgaggg aatgccgtag 320
<210> 193
   <211> 320
   <212> RNA
   <213> Mycobacterium szulgai
<400> 193
   acuucgcgca ucgaaucaag ggaagcgugc cggugcaggc aagagaccac cguaagcguc 60
   guugcaacca auuaagcgcc gagaacacuc agcgcgacuu cgcucucgcu gccuaagcga 120
   cagcaagucc gucagaccgg gaaagcccuc gacccggacc cuggcgucau cuagagggau 180
   ccaccgguga guucggucgc gggacucauc gggacaccaa cagcgacugg gaucgucauc 240
   cuggcuaguu cgcgugacca ggagauccga guagagacau agcgaacugc gcacggagaa 300
   gccuugaggg aaugccguag                                             320
<210> 194
   <211> 320
   <212> DNA
   <213> Mycobacterium malmoense
<400> 194
   acttcgcgca tcgaatcaag ggaagcgtgc cggtgcaggc aagagaccac cgtaagcgtc 60
   gttgcaacca tataagcgcc gtttcaacac agcgcgacta cgctctcgct gcctaagcga 120
   cagctagtcc gtcagaccgg gaacgccctc gacccggagc ctggcgtcag ctggagggat 180
   ccaccggtga gtccggtcgc gggactcatc gggacataca cagcgactgg gatcgtcatc 240
   ctggctggtt cgcgtgaccg ggagatccga gcagaggcat agcgaactgc gcacggagaa 300
   gccttgaggg aatgccgtag                                             320
<210> 195
   <211> 320
   <212> RNA
   <213> Mycobacterium malmoense
<400> 195
   acuucgcgca ucgaaucaag ggaagcgugc cggugcaggc aagagaccac cguaagcguc 60
   guugcaacca uauaagcgcc guuucaacac agcgcgacua cgcucucgcu gccuaagcga 120
   cagcuagucc gucagaccgg gaacgcccuc gacccggagc cuggcgucag cuggagggau 180
   ccaccgguga guccggucgc gggacucauc gggacauaca cagcgacugg gaucgucauc 240
   cuggcugguu cgcgugaccg ggagauccga gcagaggcau agcgaacugc gcacggagaa 300
   gccuugaggg aaugccguag                                             320
<210> 196
   <211> 321
   <212> DNA
   <213> Mycobacterium flavescens
<400> 196
   acttcgagcg tcgaatcaag ggaagcgtgc cggtgcaggc aagagaccac cgtaagcgtc 60
   gttgcaacca attaagcgcc gattccaatc agcgcgacta cgcactcgct gcctaagcga 120
   ctgcgtgtct gtcagcccgg gagagccctc gacccggtgt ctggcatcag ctagagggat 180
   aaaccggtgg gtccggtcgc gggactcatc gggacatcaa acagcgactg ggatcgtcat 240
   cctgacttgt tcgcgtgatc aggagatccg agtagagaca tagcgaactg cgcacggaga 300
   agccttgagg gaacgccgta g 321
<210> 197
   <211> 321
   <212> RNA
   <213> Mycobacterium flavescens
<400> 197
   acuucgagcg ucgaaucaag ggaagcgugc cggugcaggc aagagaccac cguaagcguc 60
   guugcaacca auuaagcgcc gauuccaauc agcgcgacua cgcacucgcu gccuaagcga 120
   cugcgugucu gucagcccgg gagagcccuc gacccggugu cuggcaucag cuagagggau 180
   aaaccggugg guccggucgc gggacucauc gggacaucaa acagcgacug ggaucgucau 240
   ccugacuugu ucgcgugauc aggagauccg aguagagaca uagcgaacug cgcacggaga 300
   agccuugagg gaacgccgua g 321
<210> 198
   <211> 320
   <212> DNA
   <213> Mycobacterium marinum
<400> 198
   acttcgcgca tcgaatcaag ggaagcgtgc cggtgcaggc aagagaccac cgtaagcgtc 60
   gatgcaacta gataagcgcc gattcacatc agcgcgacta cgctctcgct gcctaagcga 120
   cggctagtct gtcggaccgg gaacgccctc gccccggacc ccggcatcag ctagagggat 180
   caaccgatga gttcggtcgc ggggctcatc gggacatcaa cagcgactgg gatcgtcatc 240
   ctggctagtt cgcgtgacca ggagatccga gcagagacct agcggactgc gcacggagaa 300
   gccttgaggg aatgccgtag 320
<210> 199
   <211> 320
   <212> RNA
   <213> Mycobacterium marinum
<400> 199
   acuucgcgca ucgaaucaag ggaagcgugc cggugcaggc aagagaccac cguaagcguc 60
   gaugcaacua gauaagcgcc gauucacauc agcgcgacua cgcucucgcu gccuaagcga 120
   cggcuagucu gucggaccgg gaacgcccuc gccccggacc ccggcaucag cuagagggau 180
   caaccgauga guucggucgc ggggcucauc gggacaucaa cagcgacugg gaucgucauc 240
   cuggcuaguu cgcgugacca ggagauccga gcagagaccu agcggacugc gcacggagaa 300
   gccuugaggg aaugccguag 320
<210> 200
   <211> 319
   <212> DNA
   <213> Mycobacterium microti
<400> 200
   acttcgcgca tcgaatcaag ggaagcgtgc cggtgcaggc aagagaccac cgtaagcgtc 60
   gttgcgacca aataagcgcc gattcacatc agcgcgacta cgctctcgct gcctaagcga 120
   cggctagtct gtcagaccgg gaacgccctc ggcccggacc ctggcatcag ctagagggat 180
   ccaccgatga gtccggtcgc gggactcctc gggacagcca cagcgactgg gatcgtcatc 240
   tcggctagtt cgcgtgaccg ggagatccga gcagaggcat agcgaactgc gcacggagaa 300
   gccttgaggg aatgccgta                                              319
<210> 201
   <211> 319
   <212> RNA
   <213> Mycobacterium microti
<400> 201
   acuucgcgca ucgaaucaag ggaagcgugc cggugcaggc aagagaccac cguaagcguc 60
   guugcgacca aauaagcgcc gauucacauc agcgcgacua cgcucucgcu gccuaagcga 120
   cggcuagucu gucagaccgg gaacgcccuc ggcccggacc cuggcaucag cuagagggau 180
   ccaccgauga guccggucgc gggacuccuc gggacagcca cagcgacugg gaucgucauc 240
   ucggcuaguu cgcgugaccg ggagauccga gcagaggcau agcgaacugc gcacggagaa 300
   gccuugaggg aaugccgua                                              319
<210> 202
   <211> 321
   <212> DNA
   <213> Mycobacterium smegmatis
<400> 202
   acttcgagca tcgaatccag ggaagcgtgc cggtgcaggc aagagaccac cgtaagcgtc 60
   gttgcaacca attaagcgcc gattccaatc agcgcgacta cgccctcgct gcctaagcga 120
   cggctggtct gtcagaccgg gagtgccctc ggcccggatc ctggcatcag ctagagggac 180
   ccacccacgg gttcggtcgc gggacctgtg gggacatcaa acagcgactg ggatcgtcat 240
   ctcggcttgt tcgtgtgacc gggagatccg agtagagaca tagcgaactg cgcacggaga 300
   agcctcgagg acatgccgta g                                           321
<210> 203
   <211> 321
   <212> RNA
   <213> Mycobacterium smegmatis
<400> 203
   acuucgagca ucgaauccag ggaagcgugc cggugcaggc aagagaccac cguaagcguc 60
   guugcaacca auuaagcgcc gauuccaauc agcgcgacua cgcccucgcu gccuaagcga 120
   cggcuggucu gucagaccgg gagugcccuc ggcccggauc cuggcaucag cuagagggac 180
   ccacccacgg guucggucgc gggaccugug gggacaucaa acagcgacug ggaucgucau 240
   cucggcuugu ucgugugacc gggagauccg aguagagaca uagcgaacug cgcacggaga 300
   agccucgagg acaugccgua g                                           321
<210> 204
   <211> 320
   <212> DNA
   <213> Mycobacterium xenopi
<400> 204
   acttcgcgca tcgaatcaag ggaagcgtgc cggtgcaggc aagagaccac cgtaagcgtc 60
   gttgcaacta aataagcgcc gattcacatc agcgcgacta cgctctcgct gcctaagcga 120
   cagctagtcc gtcaggccgg gagttccctc gacccggatc ctggcgtcag ctagagggat 180
   ccaccgatgg gttcggtcgc gggacccatc gggacaccac acagcgactg ggatcgccgt 240
   cccggctagt tcgcgagacc gggagatccg agtaagggca aagcgaactg cgcacggaga 300
   agccttgagg gtatgccgta 320
<210> 205
   <211> 320
   <212> RNA
   <213> Mycobacterium xenopi
<400> 205
   acuucgcgca ucgaaucaag ggaagcgugc cggugcaggc aagagaccac cguaagcguc 60
   guugcaacua aauaagcgcc gauucacauc agcgcgacua cgcucucgcu gccuaagcga 120
   cagcuagucc gucaggccgg gaguucccuc gacccggauc cuggcgucag cuagagggau 180
   ccaccgaugg guucggucgc gggacccauc gggacaccac acagcgacug ggaucgccgu 240
   cccggcuagu ucgcgagacc gggagauccg aguaagggca aagcgaacug cgcacggaga 300
   agccuugagg guaugccgua 320
<210> 206
   <211> 320
   <212> DNA
   <213> Mycobacterium intracellulare
<400> 206
   acttcgcgca tcgaatcaag ggaagcgtgc cggtgcaggc aaccgaccac cgtaagcgtc 60
   gttgcaaaca gataagcgcc gattcacatc agcgcgacta cgctctcgct gcctaagcga 120
   cagctagtcc gtcagaccgg gaacgccctc gacccggagc ctggcgtcag ctagagggat 180
   ccaccgatga gtccggtcgc gggacttatc gggacaccaa cagcgactgg gatcgtcatc 240
   tcggcttgtt cgcgtgaccg ggagatccga gtagaggcat agcgaactgc gcacggagaa 300
   gtcttgaggg aatgccgtag 320
<210> 207
   <211> 320
   <212> RNA
   <213> Mycobacterium intracellulare
<400> 207
   acuucgcgca ucgaaucaag ggaagcgugc cggugcaggc aaccgaccac cguaagcguc 60
   guugcaaaca gauaagcgcc gauucacauc agcgcgacua cgcucucgcu gccuaagcga 120
   cagcuagucc gucagaccgg gaacgcccuc gacccggagc cuggcgucag cuagagggau 180
   ccaccgauga guccggucgc gggacuuauc gggacaccaa cagcgacugg gaucgucauc 240
   ucggcuuguu cgcgugaccg ggagauccga guagaggcau agcgaacugc gcacggagaa 300
   gucuugaggg aaugccguag 320
<210> 208
   <211> 320
   <212> DNA
   <213> Mycobacterium scrofulaceum
<400> 208
   acatcgcgca tcgaatcaag ggaagcgtgc cggtgcaggc aagagaccac cgtaagcgtc 60
   gttgcaacca attaagcgcc gattcacatc agcgcgacta cgctctcgct gcctaagcga 120
   cagctagtcc gtcagaccgg gaaagccctc gacccggagc ctggcgtcag ctagagggat 180
   caaccgatga gttcggtcgc gggactcatc gggacaccaa cagcgactgg gatcgtcatc 240
   ctggctagtc cgcgtgacca ggagatccga gcagaggcat agcggactgc gcacggagaa 300
   gtcttgaggg aatgccgttg 320
<210> 209
   <211> 320
   <212> RNA
   <213> Mycobacterium scrofulaceum
<400> 209.
   acaucgcgca ucgaaucaag ggaagcgugc cggugcaggc aagagaccac cguaagcguc 60
   guugcaacca auuaagcgcc gauucacauc agcgcgacua cgcucucgcu gccuaagcga 120
   cagcuagucc gucagaccgg gaaagcccuc gacccggagc cuggcgucag cuagagggau 180
   caaccgauga guucggucgc gggacucauc gggacaccaa cagcgacugg gaucgucauc 240
   cuggcuaguc cgcgugacca ggagauccga gcagaggcau agcggacugc gcacggagaa 300
   gucuugaggg aaugccguug 320
<210> 210
   <211> 326
   <212> DNA
   <213> Nocardia asteroides
<400> 210
   actgtgtgcg ccgaggtagg ggaagcgtgt cggtgcaggc tggagaccac cgttaagcgt 60
   cgcggcaacc aattaagcgc cgattccaat cagcgcgact acgccctcgc tgcctgatca 120
   gcgacggcta gctgtcggcc cgggttgtgt tcccgaaccc ggatgccggc atcatctcag 180
   ggaactcacc gtgttcgccg gtcgcggacg gacacgggac agcaaacagc gactgggatc 240
   gtcatctcgg cttgttcgcg tgaccgggag atccaagtag agacatagcg gactgcacac 300
   ggagaagccc tactgactcg acacag 326
<210> 211
   <211> 325
   <212> RNA
   <213> Nocardia asteroides
<400> 211
   acugugugcg ccgagguagg ggaagcgugu cggugcaggc uggagaccac cguuaagcgu 60
   cgcggcaacc aauuaagcgc cgauuccaau cagcgcgacu acgcccucgc ugccugauca 120
   gcgacggcua gcugucggcc cggguugugu ucccgaaccc ggaugccggc aucaucucag 180
   ggaacucacc guguucgccg gucgcggacg gacacgggac agcaaacagc gacugggauc 240
   gucaucucgg cuuguucgcg ugaccgggag auccaaguag agacauagcg gcugcacacg 300
   gagaagcccu acugacucga cacag                                      325
<210> 212
   <211> 314
   <212> DNA
   <213> Salmonella enteritidis
<400> 212
   acgggatttg cgaaacccaa ggtgcatgcc gaggggcggt tggcctcgta aaaagccgca 60
   aaaaaatagt cgcaaacgac gaaacctacg ctttagcagc ttaataacct gcttagagcc 120
   ctctctccct agcctccgct cttaggacgg ggatcaagag aggtcaaacc caaaagagat 180
   cgcgtggatg ccctgcctgg ggttgaagcg ttaaaacgaa tcaggctagt ctggtagtgg 240
   cgtgtccgtc cgcaggtgcc aggcgaatgt aaagactgac taagcatgta gtaccgagga 300
   tgtaggaatt tcgg                                                  314
<210> 213
   <211> 314
   <212> RNA
   <213> Salmonella enteritidis
<900> 213
<210> 214
   <211> 313
   <212> DNA
   <213> Staphylococcus epidermidis (NCTC 11047)
<400> 214
   acaggggtcc cccgagctta ttaagcgtgt cggagggttg gctccgtcat caacacattt 60
   cggttaaata taactgacaa atcaaacaat aatttcgcag tagctgcgta atagccactg 120
   catcgcctaa cagcatctcc tacgtgctgt taacgcgatt caaccctagt aggatatgct 180
   aaacactgcc gcttgaagtc tgtttagatg aaatataatc aagctagtat catgttggtt 240
   gtttattgct tagcatgatg cgaaaattat caataaacta cacacgtaga aagatttgta 300
   tcaggacctc tgg                                                   313
<210> 215
   <211> 313
   <212> RNA
   <213> Staphylococcus epidermidis (NCTC 11047)
<400> 215
   acaggggucc cccgagcuua uuaagcgugu cggaggguug gcuccgucau caacacauuu 60
   cgguuaaaua uaacugacaa aucaaacaau aauuucgcag uagcugcgua auagccacug 120
   caucgccuaa cagcaucucc uacgugcugu uaacgcgauu caacccuagu aggauaugcu 180
   aaacacugcc gcuugaaguc uguuuagaug aaauauaauc aagcuaguau cauguugguu 240
   guuuauugcu uagcaugaug cgaaaauuau caauaaacua cacacguaga aagauuugua 300
   ucaggaccuc ugg                                                    313
<210> 216
   <211> 302
   <212> DNA
   <213> Streptococcus agalactiae (NCTC 8181)
<900> 216
<210> 217
   <211> 302
   <212> RNA
   <213> Streptococcus agalactiae (NCTC 8181)
<400> 217
   acaggcauua ugagguauau uuugcgacuc aucggcagau guaaaaugcc aguuaaauau 60
   aacugcaaaa aauacaaauu cuuacgcauu agcugccuaa aaaacagccu gcgugaucuu 120
   cacaagauug uuugcguuuu gcuagaaggu cuuauuuauc agcaaacuac guuuggcuac 180
   ugucuaguua guuaaaaaga gauuuauaga cucgcuaugu gagggcuuga guuauguguc 240
   aucaccuagu uaaaucaaua cauaaccuau aguuguagac aaauauauua gcagauguuu 300
   gg                                                                302
<210> 218
   <211> 21
   <212> DNA
   <213> Artificial Sequence
<220>
   <223> Description of Artificial Sequence: Salmonella genus specificic probe
<400> 218
   cgaatcaggc tagtctggta g 21
<210> 219
   <211> 23
   <212> DNA
   <213> Artificial Sequence
<220>
   <223> Description of Artificial Sequence: Oligonucleotide probe for detection of tuberculosis complex
<400> 219
   actcctcggg acarccacag cga 23
<210> 220
   <211> 20
   <212> DNA
   <213> Artificial Sequence
<220>
   <223> Description of Artificial Sequence: Oligonucleotide probes for detection of M.avium and M. paratuberculosis sequences
<400> 220
   gttgcaaata gataagcgcc 20
<210> 221
   <211> 20
   <212> DNA
   <213> Artificial Sequence
<220>
   <223> Description of Artificial Sequence: Oligonucleotide probe for detection of M. avium and M. paratuberculosis sequences
<400> 221
   tccgtcagcc cgggaacqcc 20
<210> 222
   <211> 18
   <212> DNA
   <213> Artificial Sequence
<220>
   <223> Description of Artificial Sequence: Oligonucleotide probe used in determination of tmRNA integrity after heat killing treatment of Listeria cells
<400> 222
   ttttgttttt ctttgcca 18
<210> 223
   <211> 18
   <212> DNA
   <213> Artificial Sequence
<220>
   <223> Description of Artificial Sequence: Oligonucleotide probe used in determination of tmRNA integrity after heat killing treatment of Escherichia coli cells
<400> 223
   agttttcgtc gtttgcga 18
<210> 224
   <211> 20
   <212> DNA
   <213> Artificial Sequence
<220>
   <223> Description of Artificial Sequence: Degenerative oligonucleotide primer for amplification of all mycobacterial sequences
<400> 224
   caggcaashg accaccgtaa 20
<210> 225
   <211> 23
   <212> DNA
   <213> Artificial Sequence
<220>
   <223> Description of Artificial Sequence: Degenerative oligonucleotide primers for amplification of all mycobacterial sequences
<400> 225
   ggatctccyg rtcwcrcgra cwa 23
<210> 226
   <211> 18
   <212> DNA
   <213> Artificial Sequence
<220>
   <223> Description of Artificial Sequence: Oligonucleotide primer for amplification of M. avium and M. paratuberculosis sequences
<400> 226
   tgccggtgca ggcaactg 18
<210> 227
   <211> 18
   <212> DNA
   <213> Artificial Sequence
<220>
   <223> Description of Artificial Sequence: Oligonucleotide primer for amplification of M. avium and M. paratuberculosis sequences
<400> 227
   cacgcgaaca agccagga 18
<210> 228
   <211> 23
   <212> DNA
   <213> Artificial Sequence
<220>
   <223> Description of Artificial Sequence: Oligonucleotide probe for the detection of Listeria ssrA gene sequences
<400> 228
   cattaaactt tagcaaggaa gtg 23

## Description

### Technical Field

This invention relates to the identification of target sequences for use in nucleic acid assays for the detection and identification of prokaryotic and/or eukaryotic organisms.

### Background Art

The ssrA gene, which encodes a small stable high copy number RNA transcript (tmRNA), is found in all bacteria and has recently been identified in chloroplasts and diatoms. It has a dual function both as a tRNA and as an mRNA molecule and is involved in rescuing truncated mRNAs which have lost stop codons, facilitating trans-translation of truncated peptides prior to protease degradation (Keiler, K.C. et al. (1996), Science, 271, 990-993). The unique function of tmRNAs has directed researchers to analyse the relationship of the secondary structure of these molecules with their function. These studies have focussed on the conservation of the secondary structure of tmRNAs from different microorganisms, and on the evolutionary significance and functional_relevance of such structural conservation. Studies were carried out by Matveeva, O *et al* (1998), Vol. 16, No. 13, 1374-1375 to investigate oligonucleotide binding to RNA molecules using tmRNA as a model of RNA containing secondary structure. The studies did not have as their objective the identification of sites in tmRNA with the goal of designing antisense oligonucleotide for therapeutic purposes.

The number of nucleic acid targets/probes for bacterial diagnostics is currently limited. As such, the need to identify and characterise novel DNA and RNA targets for diagnostic purposes is now seen as a priority. Target nucleic acid sequences for the development of probes can be for example, plasmids, ribosomal RNA genes, intergenic regions, genes encoding virulence factors or random genomic DNA fragments. In addition, a number of RNA molecules have been described which are used as targets for RNA-based detection for example, ribosomal RNA and RNase P.

The basis of any nucleic acid-based probe assay is the requirement for well characterised nucleic acid sequences which are present in all prokaryotes and eukaryotes under study. For reliable detection of a prokaryotic or eukaryotic organism, the nucleic acid probes used should be highly specific (i.e. not cross-react with nucleic acids from other organisms) and highly sensitive (i.e. most or all strains of the organism to be detected should react with the probe). Therefore, preferred target sequences would be present in all strains of the organism concerned. Such sequences would have significant sequence variability to allow differentiation of the species concerned from other closely related species but, on the other hand, have sufficient sequence conservation to allow the detection of all strains of the species concerned. In general, the precise identification of a nucleic acid sequence, which could form the basis of a specific nucleic acid probe assay, is tedious, difficult and uncertain. To date there are few general approaches which would facilitate the development of nucleic acid probes for a wide variety of microorganisms. The nucleic acid sequences which have been identified as potentially useful targets for probe development are, for example, rRNA genes and RNA, and the rRNA 16S/23S intergenic region.

The majority of nucleic acid probe/target assays centre on the high copy number ribosomal RNAs (rRNA) and rRNA 16S/23S spacer regions (European Patent No. 0 395 292) of the bacterial cell for the purposes of detection and identification. A number of successful commercial bacterial diagnostic kits have been marketed based on these rRNA probes/targets for the detection of a variety of microrganisms. These include a range of commercial probe kits based on the 16S rRNA gene marketed by Genprobe Inc. San Diego California, and DNA probes based on the 16S/23S spacer region marketed by Innogenetics N.V. Ghent, Belgium. However, many of these diagnostic kits have limitations, including lack of sensitivity due to low copy-number target sequences and lack of specificity due to sequence identity between closely related organisms in many cases.

Nucleic acid-based methods that could be applied directly to samples to give an indication of the viability of any microbes present therein would be of enormous significance for food, industrial, environmental and medical applications.

A disadvantage of DNA-based methods is that they do not distinguish between living and dead organisms. Some studies have focussed on using rRNA and mRNA as indicators of cell viability (Sheridan, G.E.C. et al. (1998) Applied and Environmental Microbiology, 64, 1313-1318). However, these sequences are not satisfactory targets as rRNA and mRNA can be present in bacterial cells up to 48 hours after cell death. EP 0915170 and WO 9810101 describe assays for the detection of chlamydia using a cryptic plasmid and a region of 165 RNA.

With the advent of nucleic acid based microarray-like formatting, incorporating simultaneous monitoring of multiple nucleic acid targets, there is now a clear requirement to identify and characterise novel nucleic acid sequences for use as probes and/or target regions to detect and identify viable prokaryotic and eukaryotic cells.

### Disclosure of Invention

The invention is only limited by the claims.

The invention provides use of the ssrA gene or a fragment thereof as a target region in a nucleic acid probe assay for a prokaryotic or eukaryotic organism.

Thus, the invention has application in relation to all organisms other than viruses.

No other nucleic acid probe assay has been reported which uses regions of the ssrA gene as a target region to detect and identify species of prokaryotes and eukaryotes with the attendant advantages.

According to one embodiment of the invention a fragment of the ssrA gene molecule corresponding to a region of high homology from the 5' end of the DNA molecule can be used as a universal target region.

In an alternative embodiment of the invention a fragment of the ssrA gene molecule corresponding to a region of high homology from the 3' end of the DNA molecule can be used as a universal target region.

In a further embodiment of the invention a fragment of the ssrA gene molecule corresponding to a region of low homology can be used as a target region in a nucleic acid probe assay to distinguish between species.

In a still further embodiment of the invention a fragment of the ssrA gene molecule corresponding to a region of low homology can be used as a target region for the generation of a genus specific probe.

As hereinafter described nucleotide sequence alignments of ssrA gene sequences from different organisms show that the 5' and 3' regions of these molecules demonstrate a high degree of homology and are therefore useful as universal target regions. The ssrA genes also demonstrate a more significant degree of nucleotide sequence variability between closely related organisms than any other bacterial high copy number RNA. These variable regions are ideal targets for nucleic acid assays to distinguish between species.

The invention also provides use of tmRNA, an RNA transcript of the ssrA gene, or a fragment thereof as a target region in a nucleic acid probe assay for a prokaryotic or eukaryotic organism.

According to one embodiment of this aspect of the invention a fragment of a tmRNA molecule corresponding to a region of high homology from the 5' end of the tmRNA molecule can be used as a universal target region.

Alternatively, a fragment of a tmRNA molecule corresponding to a region of high homology from the 3' end of the tmRNA molecule can be used as a universal target region.

According to a further embodiment of this aspect of the invention a fragment of a tmRNA molecule corresponding to a region of low homology can be used as a target region in a nucleic acid probe assay to distinguish between species.

According to a still further embodiment a fragment of a tmRNA molecule corresponding to a region of low homology can be used as a target region for the generation of a genus specific probe.

The nucleic acid probe (DNA or RNA) in accordance with the invention typically consists of at least 10 nucleotides of the ssrA gene and /or tmRNA transcript or their complementary sequence and is used in a nucleic acid probe hybridisation assay for a prokaryotic or eukaryotic organism. Probe hybridisation to its complementary sequence is typically revealed by labelling the nucleic acid probe with a radioactive or nonradioactive (e.g. colorimetric or fluorimetric) label.

In preferred embodiments said ssrA gene fragment or said tmRNA fragment can be used as the basis of a primer to be used in an amplification procedure.

Universal oligonucleotide primers directed to the 5' and 3' regions of either the ssrA gene or the tmRNA sequence can be used in accordance with the invention to amplify the ssrA gene or its encoding tmRNA from a wide variety of bacteria, facilitating amplification of a wide range of organisms simultaneously, whilst also enabling specific nucleic acid probe hybridisation and detection.

Preferably, the product of the amplification procedure is used as a target region in a nucleic probe assay.

Further, preferably, a cDNA transcript of a tmRNA molecule is used as a probe in a nucleic acid hybridisation assay.

Such assays can be carried out *in vitro* or *in situ.*

The target region as defined herein can be used as the basis of an assay for distinguishing between living and dead prokaryotic or eukaryotic organisms.

In contrast to rRNA and mRNA which can be present in bacterial cells following cell death, tmRNA is rapidly degraded in dead organisms. Thus, tmRNA can be a useful target for distinguishing between living and dead prokaryotic or eukaryotic organisms either directly by nucleic acid probe hybridisation to isolated bacterial RNA, or by combined RNA amplification and nucleic acid probe hybridisation to the amplified product.

Preferably, the target region is used in a multiple probe format for broad scale detection and/or identification of prokaryotic or eukaryotic organisms.

An ssrA gene probe or a tmRNA transcript probe in accordance with the invention can be linked to a microarray gene chip system for the broad scale high throughput detection and identification of prokaryotic or eukaryotic organisms.

A target region in accordance with the invention can also be used as a probe in an assay to detect prokaryotic or eukaryotic organisms in a sample of matter.

Such a sample of matter can include biological samples such as samples of tissue from the respiratory tract, the uro-genital tract or the gastrointestinal tract, or body fluids such as blood and blood fractions, sputum or cerebrospinal fluid.

An assay in accordance with the invention can also be carried out on food samples, environmental samples including air, water, marine and soil samples, and plant and animal derived samples.

According to the invention a fragment of the ssrA gene or the tmRNA transcript can also be used in an assay to obtain a DNA profile of a prokaryotic or eukaryotic organism and, thereby, distinguish between strains of the same species.

Nucleic acid sequence alignments have shown that sequence variation occurs in the ssrA gene and the tmRNA transcript within individual species. This intra-species sequence variation can be used to distinguish between strains of the same species for epidemiology, tracing of infectious agents for example, in outbreaks, or for population studies.

Other applications of the invention include the use of the ssrA gene, the tmRNA transcript or a DNA sequence complementary thereto, or a fragment thereof, to design an agent directed against infectious prokaryotic or eukaryotic organisms for therapeutic purposes.

Such agents can include antisense mRNA or oligonucleotides, ribozymes, and antagonistic peptides and are suitable for use in any kind of medical condition.

Thus, the invention can be used for the detection of viable organisms only in biological samples using the tmRNA target. Thus, during and following any anti-infectious agent drug treatment, the tmRNA target can be used to monitor the efficacy of the therapy on those specific infectious agents (e.g. antimicrobial and/or anti-parasitic treatments).

In one embodiment, the target region is used to monitor the efficacy of drug therapies against infectious agents.

In another embodiment, the target region is used to monitor the viability and level of health-promoting organisms in the gastrointestinal tract.

This aspect of the invention relates, for example, to the introduction into the gut flora of health-promoting (probiotic) organisms contained in for example yoghurt or other food to improve health. There is an interest and need to continuously monitor the presence and levels of these organisms to ensure their continued function in promoting health. The tmRNA region can be used as a target to detect viable organisms, for example in faeces, so as to monitor the presence of the health promoting organisms.

In a further embodiment, the assay is used for the quantification of prokaryotic or eukaryotic organisms.

When using probe hybridisation and/or *in vitro* amplification to detect organisms in a sample it is possible to determine the number of organisms present, based on the signal intensity. Real-time methods of *in vitro* amplification can also be used to enable the quantification of organisms in a sample. Thus, the ability to quantify the number of organisms in a sample can be important in clinical situations for treatment purposes, for example for antibiotic or other treatments or for monitoring treatment efficacy.

A still further application of the invention is the use of a database of ssrA gene sequences to identify a prokaryotic or eukaryotic organism.

The invention provides a variety of probes for the 5' and 3' homologous regions and the variable regions of the ssrA gene and tmRNA sequences, the probes being derived from these sequences or sequences complementary thereto. Representative sequences are as follows:

### Actinobacillus actinomycetemcomitans ssrA

### Actinobacillus actinomycetemcomitans tmRNA

### Aeromonas salmonicida ssrA, internal partial

### Aeromonas salmonicida tmRNA, internal partial

### Alcaligenes eutrophus ssrA

### Alcaligenes eutrophus tmRNA

### Aquifex aeolicus ssrA

### Aquifex aeolicus tmRNA

### Bacillus megaterium ssrA, internal partial

### Bacillus megaterium tmRNA, internal partial

### Bacillus subtilis ssrA

### Bacillus subtilis tmRNA

### Bordetella pertussis ssrA

### Bordetella pertussis tmRNA

### Borrelia burgdorferi ssrA

### Borrelia burgdorferi tmRNA

### Campylobacter jejuni ssrA

### Campylobacter jejuni tmRNA

### Chlamydia trachomatis (D/UW-3/CX) ssrA

### Chlamydia trachomatis (D/UW-3/CX) tmRNA

### Chlamydia trachomatis (mouse pneumonitis) ssrA

### Chlamydia trachomatis (mouse pneumonitis) tmRNA

### Chlorobium tepidum ssrA

### Chlorobium tepidum tmRNA

### Cyanophora paradoxa (alga) cyanelle ssrA

### Cyanophora paradoxa (alga) cyanelle tmRNA

### Clostridium acetobutylicum ssrA, 3' partial

### Clostridium acetobutylicum tmRNA, 3' partial

### Deinococcus radiodurans ssrA

### Deinococcus radiodurans tmRNA

### Desulfovibrio desulfuricans ssrA, internal partial

### Desulfovibrio desulfuricans tmRNA, internal partial

### Dichelobacter nodosus ssrA, 3' partial

### Dichelobacter nodosus tmRNA, 3' partial

### Enterococcus faecalis ssrA

### Enterococcus faecalis tmRNA

### Escherichia coli ssrA

### Escherichia coli tmRNA

### Haemophilus influenzae ssrA

### Haemophilus influenzae tmRNA

### Helicobacter pylori (ATCC 43504) ssrA, internal partial

### Helicobacter pylori (ATCC 43504) tmRNA, internal partial

### Helicobacter pylori (strain 26695) ssrA

### Helicobacter pylori (strain 26695) tmRNA

### Klebsiella aerogenes (NCTC 9528)ssrA, internal partial

### Klebsiella aerogenes (NCTC 9528) tmRNA, internal partial

### Lactobacillus lactis (NCTC 662)ssrA, internal partial

### Lactobacillus lactis (NCTC 662) tmRNA, internal partial

### Legionella pneumophila ssrA, internal partial

### Legionella pneumophila tmRNA, internal partial

### Listeria grayi ssrA, internal partial

### Listeria grayi tmRNA, internal partial

### Listeria innocua ssrA, internal partial

### Listeria innocua tmRNA, internal partial

### Listeria monocytogenes (NCTC 7973)ssrA, internal partial

### Listeria monocytogenes (NCTC 7973)tmRNA, internal partial

### Listeria monocytogenes (NCTC 11994) ssrA, internal partial

### Listeria monocytogenes (NCTC 11994) tmRNA, internal partial

### Listeria murrayi ssrA, internal partial

### Listeria murrayi tmRNA, internal partial

### Listeria welshimeri ssrA, internal partial

### Listeria welshimeri tmRNA, internal partial

### Marinobacter hydrocarbonoclasticus ssrA, internal partial

### Marinobacter hydrocarbonoclasticus tmRNA, internal partial

### Mycobacterium avium ssrA, internal partial

### Mycobacterium avium tmRNA, internal partial

### Mycobacterium bovis ssrA, internal partial

### Mycobacterium bovis tmRNA, internal partial

### Mycobacterium leprae ssrA

### Mycobacterium leprae tmRNA

### Mycobacterium paratuberculosis ssrA, internal partial

### Mycobacterium paratuberculosis tmRNA, internal partial

### Mycobacterium tuberculosis ssrA

### Mycobacterium tuberculosis tmRNA

### Mycoplasma capricolum ssrA

### Mycoplasma capricolum tmRNA

### Mycoplasma genitalium (ATTC 33530, # 1) ssrA

### Mycoplasma genitalium (ATTC 33530, #1) tmRNA

### Mycoplasma genitalium (ATTC 33530, #2) tmRNA, internal partial

### Mycoplasma genitalium (ATTC 33530, #2) tmRNA, internal partial

### Mycoplasma pneumophila ssrA

### Mycoplasma pneumophila tmRNA

### Neisseria gonorrhoeae (ATCC 19424) ssrA, internal partial

### Neisseria gonorrhoeae (ATCC 19424) tmRNA, internal partial

### Neisseria gonorrhoeae (FA 1090) ssrA

### Neisseria gonorrhoeae (FA 1090) tmRNA

### Neisseria meningitidis ssrA

### Neisseria meningitidis tmRNA

### Nostoc muscorum PCC7120 ssrA

### Nostoc muscorum PCC7120 tmRNA

### Odontella sinensis (diatom) chloroplast ssrA

### Odontella sinensis (diatom) chloroplast tmRNA

### Porphyra purpureum (red alga) chloroplast ssrA

### Porphyra purpureum (red alga) chloroplast tmRNA

### Porphyromonas gingivalis ssrA

### Porphyromonas gingivalis tmRNA

### Proteus rettgeri ssrA (NCTC 10975), internal partial

### Proteus rettgeri tmRNA (NCTC 10975), internal partial

### Pseudoalteromonas haloplanktoni ssrA, internal partial

### Pseudoalteromonas haloplanktoni tmRNA, internal partial

### Pseudomonas aeruginosa ssrA

### Pseudomonas aeruginosa tmRNA

### Salmonella typhimurium ssrA

### Salmonella typhimurium tmRNA

### Shewanella putrefaciens ssrA

### Shewanella putrefaciens tmRNA

### Staphylococcus aureus ssrA

### Staphylococcus aureus tmRNA

### Streptococcus gordonii ssrA

### Streptococcus gordonii tmRNA

### Streptococcus mutans ssrA

### Streptococcus mutans tmRNA

### Streptococcus pneumoniae ssrA

### Streptococcus pneumoniae tmRNA

### Streptococcus pyogenes ssrA

### Streptococcus pyogenes tmRNA

### Synechococcus sp. PCC6301 ssrA

### Synechococcus sp. PCC6301 tmRNA

### Synechocystis sp. PCC6803 ssrA

### Synechocystis sp. PCC6803 tmRNA

### Thermotoga maritima ssrA

### Thermotoga maritima tmRNA

### Thermus thermophilus ssrA

### Thermus thermophilus tmRNA

### Treponema pallidum ssrA

### Treponema pallidum tmRNA

### Vibrio cholerae ssrA

### Vibrio cholerae tmRNA

### Yersinia pestis ssrA

### Yersinia pestis tmRNA

### Campylobacter fetus ssrA, internal partial

### Campylobacter fetus tmRNA, internal partial

### Campylobacter coli (BM2509) ssrA, internal partial

### Campylobacter coli (BM2509) tmRNA, internal partial

### Camplyobacter chicken isolate ssrA, internal partial

### Camplyobacter chicken isolate tmRNA, internal partial

### Clostridium perfringens ssrA, internal partial

### Clostridium perfringens tmRNA, internal partial

### Haemophilus ducreyi (NCTC 10945) ssrA, internal partial

### Haemophilus ducreyi (NCTC 10945) tmRNA, internal partial

### Listeria innocua (food isolate # 1) ssrA, internal partial

### Listeria innocua (food isolate #2) ssrA, internal partial

### Listeria innocua (food isolate #2) tmRNA, internal partial

### Listeria innocua (food isolate #3) ssrA, internal partial

### Listeria innocua (food isolate #3) tmRNA, internal partial

### Listeria innocua (ATCC 12210) ssrA, internal partial

### Listeria innocua (ATCC 12210) tmRNA, internal partial

### Listeria ivanovii (NCTC 11846) ssrA, internal partial

### Listeria ivanovii (NCTC 11846) tmRNA, internal partial

### Listeria seeligeri (NCTC 11856) ssrA, internal partial

### Listeria seeligeri (NCTC 11856) tmRNA, internal partial

### Salmonella enteritidis ssrA, internal partial

### Salmonella enteritidis tmRNA, internal partial

### Staphylococcus epidermidis (NCTC 11047) ssrA, internal partial

### Staphylococcus epidermidis (NCTC 11047) tmRNA, internal partial

### Streptococcus agalactiae (NCTC 8181) ssrA, internal partial

### Streptococcus agalactiae (NCTC 8181) tmRNA, internal partial

### Bordetella bronchiseptica ssrA

### Bordetella bronchiseptica tmRNA

### Chlamydia pneumoniae (C WL029), ssrA

### Chlamydia pneumoniae (C WL029) tmRNA

### Francisella tularensis ssrA

### Francisella tularensis tmRNA

### Guillardia theta (plastid) ssrA

### Guillardia theta (plastid) tmRNA

### Thalassiosira Weissflogii (plastid) ssrA

### Thalassiosira Weissflogii (plastid) tmRNA

### Helicobacter pylori ssrA, (clinical isolate 1), internal partial

### Helicobacter pylori tmRNA, (clinical isolate 1), internal partial

### Helicobacter pylori ssrA, (clinical isolate 2), internal partial

### Helicobacter pylori tmRNA, (clinical isolate 2), internal partial

### Listeria seeligeri (NCTC 11856) ssrA, internal partial

### Listeria seeligeri (NCTC 11856) tmRNA, internal partial

### Listeria ivanovii (NCTC 11846) ssrA, internal partial

### Listeria ivanovii (NCTC 11846) tmRNA, internal partial

### Mycobacterium africanum (clinical isolate) ssrA, internal partial

### Mycobacterium africanum (clinical isolate) tmRNA, internal partial

### Mycobacterium gordonae(clinical isolate)ssrA, internal partial

### Mycobacterium gordonae(clinical isolate) tmRNA, internal partial

### Mycobacterium kansasii (clinical isolate) ssrA, internal partial

### Mycobacterium kansasii (clinical isolate) tmRNA, internal partial

### Mycobacterium chelonae ssrA, internal partial

### Mycobacterium chelonae tmRNA, internal partial

### Mycobacterium szulgai (ATCC 35 799) ssrA, internal partial

### Mycobacterium szulgai (A TCC 35799) tmRNA, internal partial

### Mycobacterium malmoense (clinical isolate) ssrA, internal partial

### Mycobacterium malmoense (clinical isolate) tmRNA, internal partial

### Mycobacterium flavescens ssrA, internal partial

### Mycobacterium flavescens tmRNA, internal partial

### Mycobacterium marinum ssrA, internal partial

### Mycobacterium marinum tmRNA, internal partial

### Mycobacterium microti (environmental isolate) ssrA, internal partial

### Mycobacterium microti (environmental isolate) tmRNA, internal partial

### Mycobacterium smegmatis (ATCC 10143) ssrA, internal partial

### Mycobacterium smegmatis (ATCC 10143) ssrA, internal partial

### Mycobacterium xenopi (clinical isolate) ssrA, internal partial

### Mycobacterium xenopi (clinical isolate) tmRNA, internal partial

### Mycobacterium intracellulare (NCTC 10425) ssrA, internal partial

### Mycobacterium intracellulare (NCTC 10425) tmRNA, internal partial

### Mycobacterium scrofulaceum (NCTC 10803) ssrA, internal partial

### Mycobacterium scrofulaceum (NCTC 10803) tmRNA, internal partial

### Nocardia asteroides ssrA, internal partial

### Nocardia asteroides tmRNA, internal partial

### Salmonella enteritidis ssrA, internal partial

### Salmonella enteritidis tmRNA, internal partial

### Staphylococcus epidermidis (NCTC 11047) ssrA, internal partial

### Staphylococcus epidermidis (NCTC 11047) tmRNA, internal partial

### Streptococcus agalactiae (NCTC 8181) ssrA, internal partial

### Streptococcus agalactiae (NCTC 8181) tmRNA, internal partial

Of the above sequences SEQ ID NOs 47 to 62, 65 to 68, 71 and 72, 98 and 99, 159 to 168 and 176-217 are novel sequences.

The above mentioned sequences can be used to form a database of ssrA gene sequences which can be used to identify a bacterial species, or for the generation of nucleic acid diagnostic assays.

Representative probes identified in accordance with the invention are as follows:

### Salmonella:

### 1) Genius specific probe:

5'-CGAATCAGGCTAGTCTGGTAG-3' SEQ ID NO: 218

### Mycobacteria:

### 2) Oligonucleotide probe for detection of tuberculosis complex

TB01 5'-ACTCCTCGGACA (A/G) CCACAGCGA-3' SEQ ID NO: 219

### 3) Oligonucleotide probes for detection of M. avium and M. paratuberculosis sequences

Probe 1: PAV1 - 5' - GTTGCAAATAGATAAGCGCC-3' SEQ ID NO: 220

Probe 2: PAV2 - 5' - TCCGTCAGCCCGGGAACGCC-3' SEQ ID NO: 221

### Listeria:

### 4) Oligonucleotide probe used in the determination of tmRNA integrity after heat killing treatment of cells:

LVtm: 5' - TTTTGTTTTTCTTTGCCA - 3' SEQ ID NO: 222

### Escherichia coli:

### 5) Oligonucleotide probe used in the determination of tmRNA integrity after heat killing treatment of cells:

Evtm: 5' - AGTTTTCGTCGTTTGCGA - 3' SEQ ID NO: 223

Further representative primers identified in accordance with the invention are as follows:

### Mycobacteria:

### 1) Degenerative oligonucleotide primers for the amplification of all mycobacterial sequences

5' Primer
   10SAAM3 - 5' - CAGGCAA (G/C) (A/T/C) GACCACCGTAA-3' SEQ ID NO: 224
3' Primer

### 2) Oligonucleotide primers for the amplification of M. avium and M. paratuberculosis

5' Primer: AP for- 5'-TGCCGGTGCAGGCAACTG-3' SEQ ID NO: 226
3' Primer: AP2rev - 5' - CACGCGAACAAGCCAGGA-3' SEQ ID NO: 227

### Brief Description of the Drawings

In the accompanying drawings:
Fig. 1 is a clustal alignment of *E*. *coli* and *V cholerae* ssrA gene sequences;
Fig. 2 is a photograph of an agarose gel of total cellular RNA prepared from *E*. *coli* and *V. cholerae* cells;
Fig. 3 is a photograph of an autoradiogram of hybridisation of a *V*. *cholerae* oligonucleotide probe to tmRNA transcripts of *E*. *coli* and *V. cholerae*;
Fig. 4 is a photograph of an agarose gel of the amplified products of universal ssrA gene amplification primers from a panel of organisms;
Fig. 5 is a clustal alignment of the ssrA gene sequences from the Listeria species;
Fig. 6 is a clustal alignment of the *L. monocytogenes* and *B. subtilus* ssrA/tmRNA gene sequences;
Fig. 7 is a photograph of an agarose gel of the amplified products of Listeria genus specific PCR amplification primers from a panel of organisms;
Fig. 8 is a photograph of an autoradiogram of hybridised Listeria genus specific oligonucleotide probe to a panel of organisms as prepared in Example 4;
Fig. 9 is a photograph of an autoradiogram of hybridised *L*. *monocytogenes* species specific probe to a panel of organisms as prepared in Example 7;
Fig. 10 is a computer scanned image of a nylon membrane strip used in the multiple colorimetric probe detection of Listeria ssrA gene sequences as described in Example 6.
Fig. 11 is a clustal alignment of ssrA gene sequences from *C*. *trachomatis* strains;
Fig. 12 is a clustal alignment of ssrA gene sequences from *H. pylori* strains;

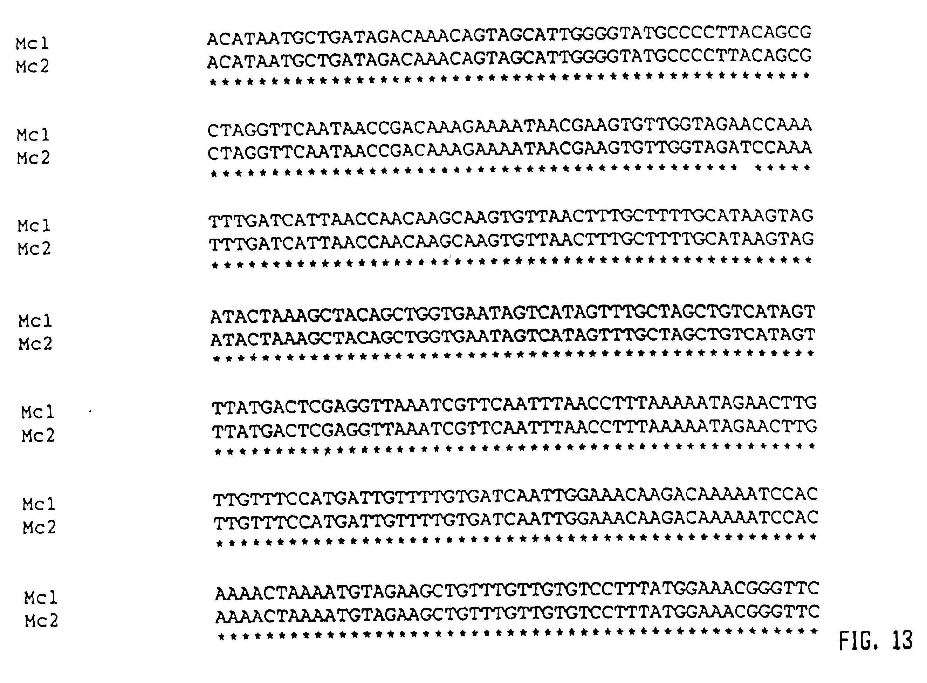

Fig. 13 is a clustal alignment of ssrA gene sequences from *M*. *genitalium* strains;

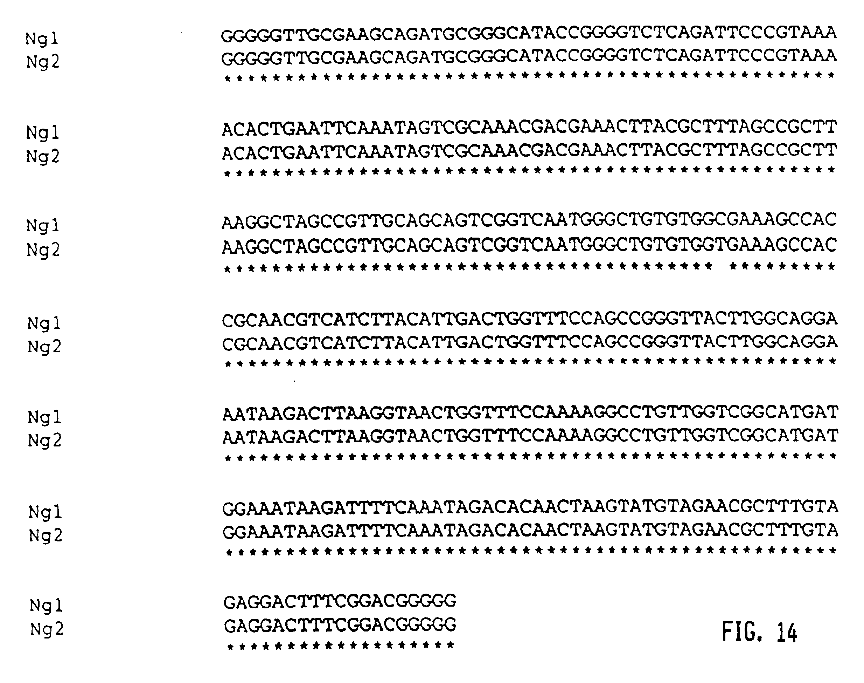

Fig. 14 is a clustal alignment of ssrA gene sequences from *N*. *gonorrhoeae* strains;
Fig. 15 is a clustal alignment of ssrA gene sequences from *L*. *monocytogenes* strains;

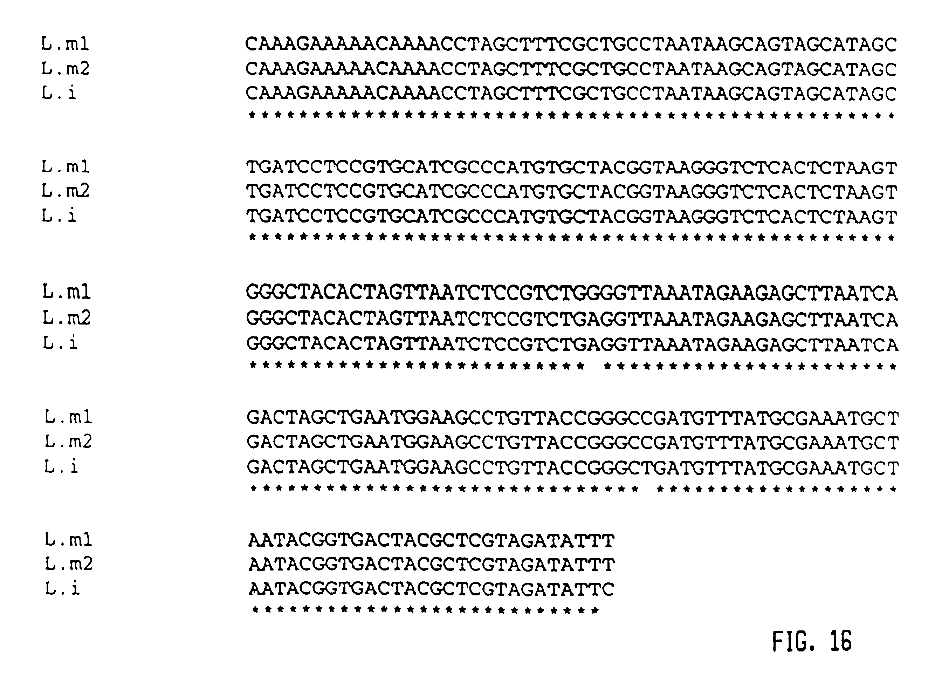

Fig. 16 is a clustal alignment of ssrA gene sequences from *L*. *monocytogenes* strains and the *L. innocua* strain;
Fig. 17 is a photograph of an autoradiogram hybridised Listeria oligonucleotide probe (Evtm) to total RNA samples isolated after medium heat treatment of *E*. *coli* cells;
Fig. 18 is a photograph of an autoradiogram hybridised Listeria oligonucleotide probe (Evtm) to total RNA samples isolated after extreme heat treatment of *E*. *coli* cells;
Fig. 19 is a photograph of an autoradiogram hybridised Listeria oligonucleotide probe (Lvtm) to total RNA samples isolated after medium heat treatment of *L. monocytogenes* cells;
Fig. 20 is a photograph of an autoradiogram hybridised Listeria oligonucleotide probe (Lvtm) to total RNA samples isolated after extreme heat treatment of *L. monocytogenes* cells; and
Fig. 21 is a photograph of an agarose gel of RT-PCR generated tmRNA products at various time points post heat treatment.

The invention will be further illustrated by the following Examples.

### Modes for carrying out the Invention

### Example 1

### Examination of the primary nucleotide sequences of available tmRNA sequences.

A comparative primary nucleotide sequence alignment of available tmRNA sequences using the Clustal W nucleic acid alignment programme demonstrated that tmRNA sequences from prokaryotes show a more significant degree of nucleotide sequence variability and non-homology than other bacterial high copy number RNA, as demonstrated in Table 1.

**Table 1**

| Percentage nucleotide sequence homology between RNA molecules from different bacteria. | | |
|---|---|---|
| | *Escherichia coli vs. Vibrio cholerae* | *Bacillus subtilus vs. Mycobacterium tuberculosis* |
| rRNA % homology | 88 | 66 |
| tmRNA % homology | 68 | 25 |

These regions of non-homology between tmRNA sequences from different bacteria are located in the middle of the molecule, and the extent of nucleotide sequence non-homology within the tmRNA molecule indicated that genus as well as species specific probes could be generated to distinguish between and/or detect bacteria.

Nucleotide sequence alignments had previously shown that the 5' and 3' flanking regions of the tmRNA molecules share a high degree of homology both within species and within genus. This observation indicated that universal oligonucleotide primers could be generated to amplify the ssrA gene or its encoding tmRNA from a wide variety of bacteria.

We have now demonstrated that these regions of homology and non-homology within the nucleotide sequence of tmRNA molecules from different organisms can be used as the basis of identifying and detecting organisms at the molecular level.

### Example 2

### Development of a V. cholerae tmRNA specific probe.

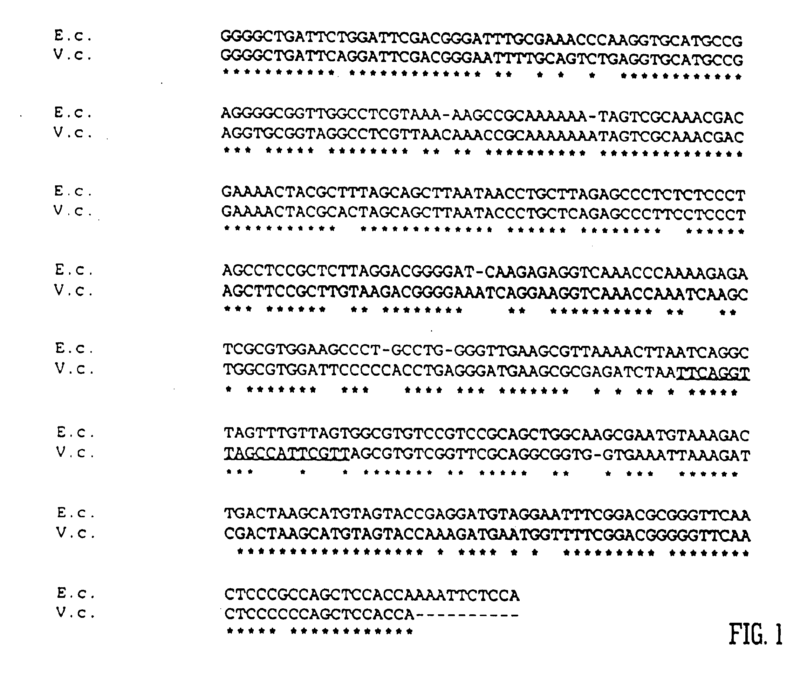

A nucleotide sequence alignment of the *E. coli* (SEQ ID NO. 37) and *V. cholerae* (SEQ ID NO. 127) *ssrA* sequences as depicted in Fig. 1, shows that these two bacterial species are phylogenetically closely related. There are however, regions of non-homology between the sequences as evidenced by the absence of asterix marks. An oligonucleotide probe, complementary to the variable region of the *V. cholerae ssr*A nucleotide sequence underlined in Fig. 1, was synthesised.

The sequence of the *V. cholerae* tmRNA specific probe is
5'- AACGAATGGCTAACCTGAA-3' SEQ ID NO. 169

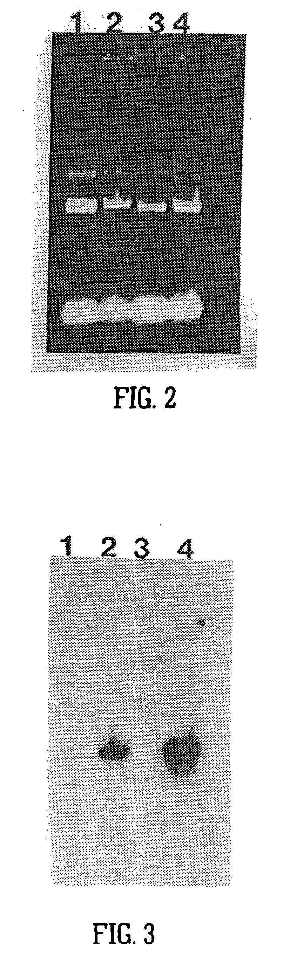

Total RNA was isolated from liquid cultures of *E*. *coli* and *V*. *cholerae* at the mid-exponential phase and the stationary phase of growth. Equivalent amounts of the isolated total RNA were electrophoresed on a denaturing formaldehyde agarose gel and blotted onto HYBOND-N nylon membrane as shown in Fig. 2 in which the Lanes 1-4 represent the following:
- Lane 1:: Total *E*. *coli* RNA mid-log phase
- Lane 2:: Total *V cholerae* RNA mid-log phase
- Lane 3:: Total *E*. *coli* RNA stationary phase
- Lane 4:: Total *V. cholerae* RNA stationary phase

The resulting Northern blot was then hybridised with the *V. cholerae* tmRNA specific probe end-labelled with γP³². The results of the hybridisation experiment shown in Fig. 3 demonstrate the specificity of the probe as only *V. cholerae* tmRNAs were detected. Moreover, a greater degree of hybridisation signal intensity was observed with the *V. cholerae* tmRNA isolated from cultures during the stationary phase of growth, indicating that a higher copy number of the tmRNA molecule is present in *V. cholerae* cells during this phase.

### Example 3

### Generation of universal ssrA/tmRNA oligonucleotide amplification primers for the characterisation of unknown ssrA gene and tmRNA sequences.

Clustal W alignment of all available ssrA gene and tmRNA sequences indicated that degenerate oligonucleotide primers could be designed to amplify ssrA gene and tmRNA nucleotide sequences for a wide variety of organisms.

Degenerate oligonucleotide primers were synthesised to PCR amplify ssrA gene sequences from total genomic DNA preparations from a broad range of bacteria.

The sequences of the synthesised degenerate oligonucleotides are as follows:

| | | |
|---|---|---|
| (a) | tmU5': | 5' *in vitro* PCR amplification primer |
| | | 5'- GGG(A/C)(ClT)TACGG(A/T)TTCGAC- 3' SEQ ID NO: 170 |
| | | |
| (b) | tmU3': | 3' *in vitro* PCR amplification primer |
| | | 5'- GGGA(A/G)TCGAACC(A/G)(C/G)GTCC- 3' SEQ ID NO: 171 |
| | | |
| Degenerate base positions are in parentheses. | | |

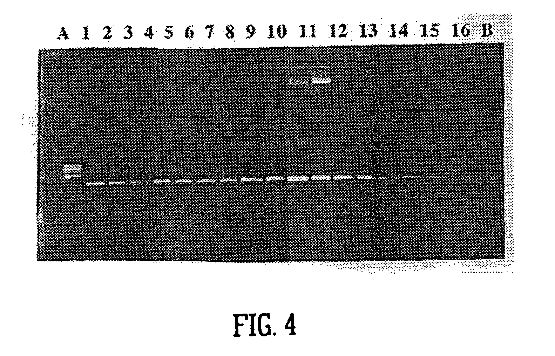

The products of PCR reactions were electrophoresed on an agarose gel and a 350 base pair (approx.) PCR product was amplified in all cases, as shown in Fig. 4, demonstrating the "universality" of the degenerate tmRNA primers.

In Fig. 4 the lanes represent the following:
- Lane A:: Molecular weight marker V
- Lane 1:: *Escherichia coli*
- Lane 2:: *Salmonella poona*
- Lane 3:: *Klebsiella aerogenes*
- Lane 4:: *Proteus mirabilis*
- Lane 5:: *Proteus rettgeri*
- Lane 6:: *Aeromonas hydrophilia*
- Lane 7:: *Staphyloccus aureus*
- Lane 8:: *Enterococcus faecalis*
- Lane 9:: *Lactobacillus lactis*
- Lane 10:: *Bacillus subtilus*
- Lane 11:: *Listeria monocytogenes*
- Lane 12:: *Listeria innocua*
- Lane 13:: *Listeria murrayi*
- Lane 14:: *Listeria welshimeri*
- Lane 15:: *Listeria grayi*
- Lane 16:: *Mycobacterium bovis*
- Lane B:: Molecular weight marker V

The universal primers amplified the ssrA gene from both Gram positive and Gram negative bacteria, as shown in Table 2.

**Table 2**

| Bacterial species tested with universal amplification primers. | | |
|---|---|---|
| | | PCR Product |
| | *Escherichia coli* | + |
| | *Salmonella poona* | + |
| | *Klebsiella aerogenes* | + |
| Gram negative bacteria | *Proteus mirabilis* | + |
| | *Proteus rettgeri* | + |
| | *Aeromonas hydrophilia* | + |
| | *Staphyloccus aureus* | + |
| | *Enterococcus faecalis* | + |
| | *Lactobacillus lactis* | + |
| | *Bacillus subtilus* | + |
| | *Listeria monocytogenes* | + |
| Gram positive bacteria | *Listeria innocua* | + |
| | *Listeria murrayi* | + |
| | *Listeria welshimeri* | + |
| | *Listeria grayi* | + |
| | *Mycobacterium bovis* | + |

### Example 4

### Isolation and characterisation of previously unknown bacterial ssrA/tmRNA nucleotide sequences.

The PCR products amplified from genomic DNA from the Listeria species of bacteria and that from the *M bovis* bacterium, from Example 2, were subcloned into a T-tailed plasmid vector for the purposes of DNA sequencing. Three recombinant clones were selected for each species and sequenced by the di-deoxy sequencing method. The sequence of both DNA strands for each subclone was determined.

The nucleotide sequence determined for the *M bovis* ssrA gene shared 100% homology with the *Mycobacterium tuberculosis* ssrA gene sequence.

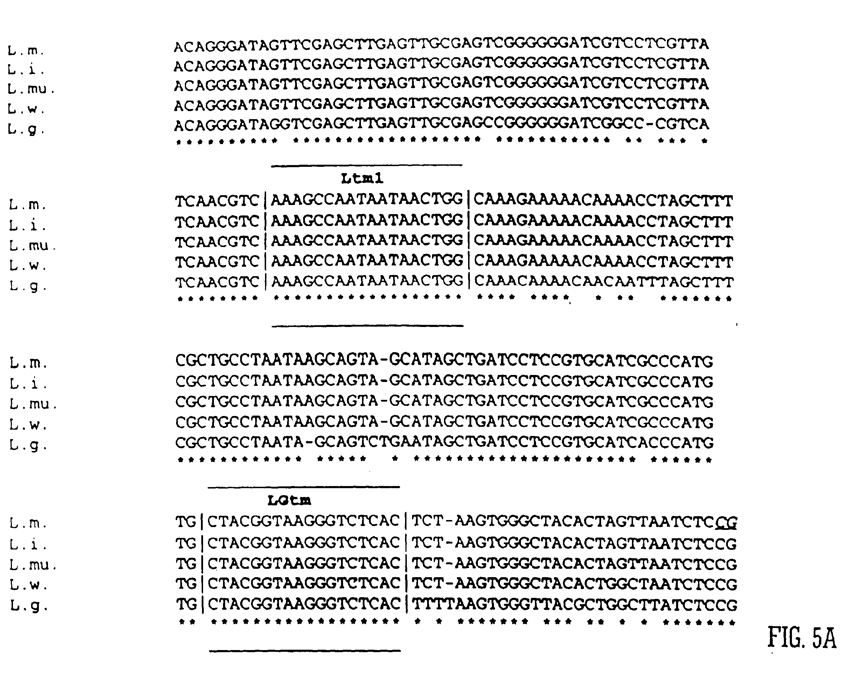

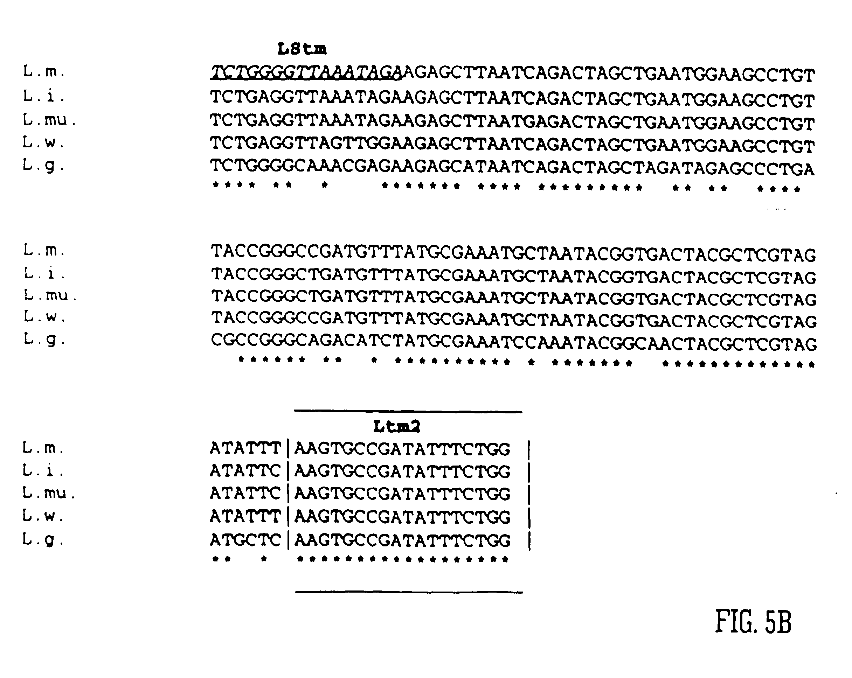

A clustal W alignment of the novel ssrA gene sequences obtained for the Listeria species (SEQ ID NOS 51, 53, 55, 59 and 61) is shown in Fig. 5. This analysis indicated that genus-specific probes and oligonucleotide amplification primers can be generated for Listeria bacteria. Furthermore, the alignment also indicated that a species specific oligonucleotide probe can be generated which will distinguish *L. monocytogenes* from the other Listeria species.

In Fig. 5 the proposed genus specific oligonucleotide primers, Ltm 1 and Ltm 2, are boxed, as is the genus specific Listeria oligonucleotide probe, LGtm. The proposed *L. monocytogenes* species specific oligonucleotide probe sequence, LStm, is underlined and italicised.

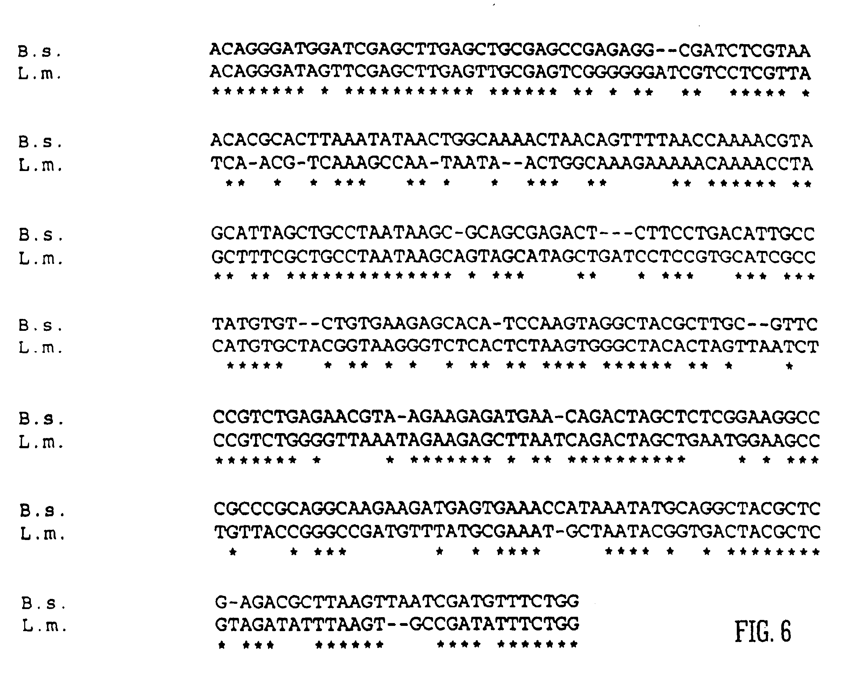

To further illustrate that the ssrA gene/tmRNA nucleic acid target is a suitable target for bacterial diagnostics, a comparative alignment of the *L*. *monocytogenes* ssrA gene nucleotide sequence (SEQ ID NO. 55) with the available *B. subtilis* ssrA gene nucleotide sequence (SEQ ID NO. 11) (a phylogenetically closely related bacteria to Listeria) was carried out as shown in Fig. 6. Analysis of the sequence alignment showed a percentage nucleotide sequence homology of 41 %, whereas the corresponding 16S rRNA alignment exhibits a nucleotide sequence percentage homology of 87%, (data not shown).

### Example 5

### Generation and application of ssrA gene/tmRNA genus-specific amplification primers, genus-specific and species-specific probes for the Listeria bacterial species.

Using the Listeria genus ssrA gene/tmRNA nucleotide sequence alignment of Example 4, regions of the ssrA gene/tmRNA nucleotide sequence were analysed to determine their suitability for the generation of genus-specific amplification primers, and genus-specific and species-specific oligonucleotide probes. In this analysis , regions which demonstrated the greatest sequence differences to *B. subtilis,* were selected in the design of these amplification primers and probes.

The sequences of the synthesised oligonucleotides are as follows:

| | | |
|---|---|---|
| (a) | Ltm1 : | 5' Listeria genus specific amplification primer |
| | | 5' -AAAGCCAATAATAACTGG- 3' SEQ ID NO: 172 |
| | | |
| (b) | Ltm2: | 3' Listeria genus specific amplification primer |
| | | 5' -CCAGAAATATCGGCACTT- 3' SEQ ID NO: 173 |
| | | |
| (c) | LGtm: | Listeria genus specific hybridisation probe |
| | | 5' -GTGAGACCCTTACCGTAG- 3' SEQ ID NO: 174 |
| | | |
| (d) | LStm: | *L. monocytogenes* species specific hybridisation probe |
| | | 5' -TCTATTTAACCCCAGACG- 3' SEQ ID NO: 175 |

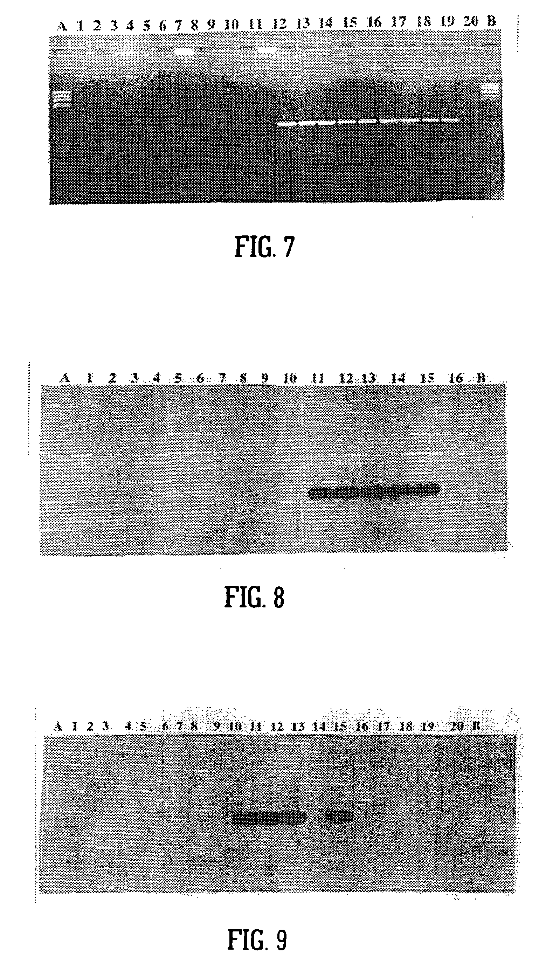

The genus specific amplification primers Ltm1 and Ltm2 were used in a series of PCR reactions with total genomic DNA from twenty different strains as the template in each case. Only ssrA gene sequences from the Listeria species were amplified (260 base pair product) with these primers (Fig. 7 and Table 3) demonstrating that the ssrA gene/tmRNA is a suitable target for specific *in vitro* amplification of a bacterial genus. No amplification products were observed for any other bacterial species tested, although PCR products were obtained from the DNA from these bacterial species using the universal primers (tmU5' and tmU3') described in Example 2.

In Fig. 7 the lanes represent the following:
- Lane A:: Molecular weight marker V
- Lane 1:: *E. coli*
- Lane 2:: *S. poona*
- Lane 3:: *K. aerogenes*
- Lane 4:: *P. mirabilis*
- Lane 5:: *P. rettgeri*
- Lane 6:: *A. hydrophilia*
- Lane 7:: *S. aureus*
- Lane 8:: *E. faecalis*
- Lane 9:: *L. lactis*
- Lane 10:: *B. subtilus*
- Lane 11:: *L. monocytogenes* strain I
- Lane 12:: *L. monocytogenes* strain 2
- Lane 13:: *L. monocytogenes* strain 3
- Lane 14:: *L. monocytogenes* strain 4
- Lane 15:: *L. monocytogenes* clinical isolate
- Lane 16:: *L. innocua*
- Lane 17:: *L. murrayi*
- Lane 18:: *L. welshimeri*
- Lane 19:: *L. grayi*
- Lane 20:: *M bovis*
- Lane B:: Molecular weight marker V

**Table 3**

| Bacterial species tested with Listeria specific amplification primers. | | |
|---|---|---|
| | | PCR Product |
| | *Escherichia coli* | *-* |
| | *Salmonella poona* | *-* |
| | *Klebsiella aerogenes* | - |
| Gram negative bacteria | *Proteus mirabilis* | *-* |
| | *Proteus rettgeri* | *-* |
| | *Aeromonas hydrophilia* | *-* |
| | *Staphyloccus aureus* | *-* |
| | *Entrococcus faecalis* | *-* |
| | *Lactobacillus lactis* | *-* |
| | *Bacillus subtilus* | *-* |
| | *Listeria monocytogenes* strain 1 | + |
| | *Listeria monocytogenes* strain 2 | + |
| | *Listeria monocytogenes* strain 3 | + |
| Gram positive bacteria | *Listeria monocytogenes* strain 4 | + |
| | *Listeria monocytogenes* clinical isolate | + |
| | *Listeria innocua* | *+* |
| | *Listeria murrayi* | *+* |
| | *Listeria welshimeri* | *+* |
| | *Listeria grayi* | *+* |
| | *Mycobacterium bovis* | - |

The Listeria genus specific oligonucleotide probe, LGtm, was hybridised to the Southern blot depicted in Fig. 4. Positive hybridisation signals were observed only with Listeria species as shown in Fig. 8 and Table 4, demonstrating the utility of the tmRNA sequence as a target in detecting a specific genus.

In Fig. 8 the lanes represent the following:
- Lane A:: Molecular weight marker V
- Lane 1:: *Escherichia coli*
- Lane 2:: *Salmonella poona*
- Lane 3:: *Klebsiella aerogenes*
- Lane 4:: *Proteus mirabilis*
- Lane 5:: *Proteus rettgeri*
- Lane 6:: *Aeromonas hydrophilia*
- Lane 7:: *Staphyloccus aureus*
- Lane 8:: *Enterococcus faecalis*
- Lane 9:: *Lactobacillus lactis*
- Lane 10:: *Bacillus subtilus*
- Lane 11:: *Listeria monocytogenes*
- Lane 12:: *Listeria innocua*
- Lane 13:: *Listeria murrayi*
- Lane 14:: *Listeria welshimeri*
- Lane 15:: *Listeria grayi*
- Lane 16:: *Mycobacterium bovis*
- Lane B:: Molecular weight marker V

The PCR products generated using the genus-specific amplification described in this Example, and shown in Fig. 7, were Southern blotted and hybridised to the *L. monocytogenes* species-specific oligonucleotide probe. A positive hybridisation signal was observed with three of the four typed strains and the clinical isolate of *L. monocytogenes* as shown in Fig. 9 and Table 4.

In Fig. 9 the lanes represent the following:
- Lane A:: Molecular weight marker V
- Lane 1:: *E. coli*
- Lane 2:: *S. poona*
- Lane 3:: *K. aerogenes*
- Lane 4:: *P. mirabilis*
- Lane 5:: *P. rettgeri*
- Lane 6:: *A. hydrophilia*
- Lane 7:: *S. aureus*
- Lane 8:: *E. faecalis*
- Lane 9:: *L. lactis*
- Lane 10:: *B. subtilus*
- Lane 11:: *L. monocytogenes* strain 1
- Lane 12:: *L. monocytogenes* strain 2
- Lane 13:: *L. monocytogenes* strain 3
- Lane 14:: *L. monocytogenes* strain 4
- Lane 15:: *L. monocytogenes* clinical isolate
- Lane 16:: *L. innocua*
- Lane 17:: *L. murrayi*
- Lane 18:: *L. welshimeri*
- Lane 19:: *L. grayi*
- Lane 20:: *M bovis*
- Lane B:: Molecular weight marker V

**Table 4**

| Specificity of the Listeria genus-specific probe and the *L. monocytogenes* species-specific probe. | | | |
|---|---|---|---|
| | | LGtm Genus-specific probe | LStm Species-specific probe |
| | *Escherichia coli* | - | - |
| | *Salmonella poona* | - | - |
| | *Klebsiella aerogenes* | - | - |
| Gram negative bacteria | *Proteus mirabilis* | - | - |
| | *Proteus rettgeri* | - | - |
| | *Aeromonas hydrophilia* | - | - |
| | *Staphyloccus aureus* | - | - |
| | *Entrococcus faecalis* | - | - |
| | *Lactobacillus lactis* | - | - |
| | *Bacillus subtilus* | - | - |
| | *Listeria monocytogenes* strain 1 | + | + |
| | *Listeria monocytogenes* strain 2 | + | + |
| Gram positive bacteria | *Listeria monocytogenes* strain 3 | + | + |
| | *Listeria monocytogenes* strain 4 | + | - |
| | *Listeria monocytogenes clinical* isolate | + | + |
| | *Listeria innocua* | + | - |
| | *Listeria murrayi* | + | - |
| | *Listeria welshimeri* | + | - |
| | *Listeria grayi* | + | - |
| | *Mycobacterium bovis* | - | - |

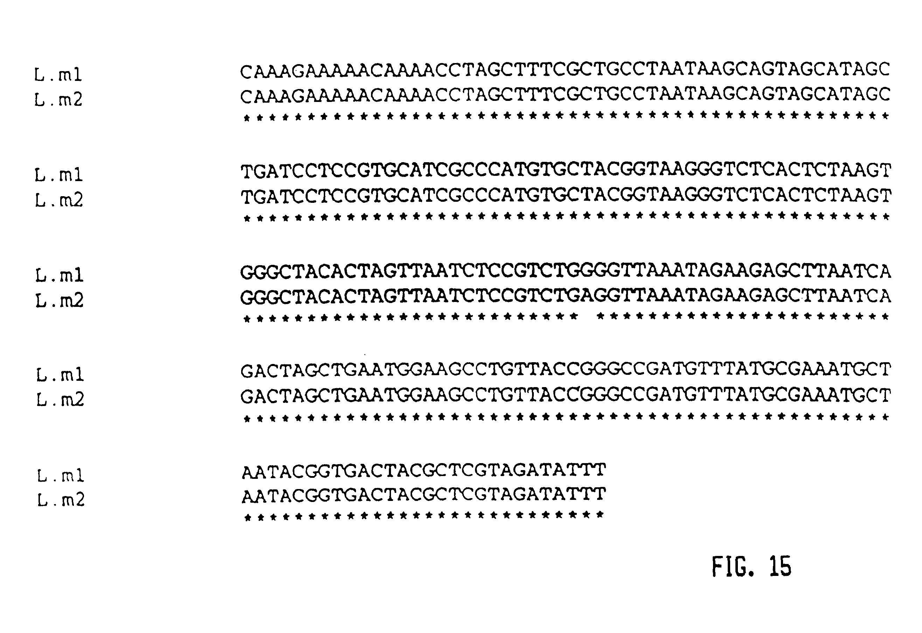

One of the typed *L. monocytogenes* strains, strain 4, failed to generate a positive signal with this probe. DNA sequencing of the PCR amplified ssrA gene from this strain demonstrated that it contained a probe target region identical to *L. innocua.* It should be noted however that the ssrA gene from this strain contains other regions where the sequence is identical to the previously characterised *L. monocytogenes* strain and that these sequences are different to the *L. innocua* sequence, as shown in Fig. 15. Therefore a species specific oligonucleotide directed to one of these variable regions can be synthesised which would recognise each strain type (isolate) within the species, for example *L. monocytogenes.*

### Example 6

### Multiple colorimetric probe detection of Listeria ssrA gene sequences.

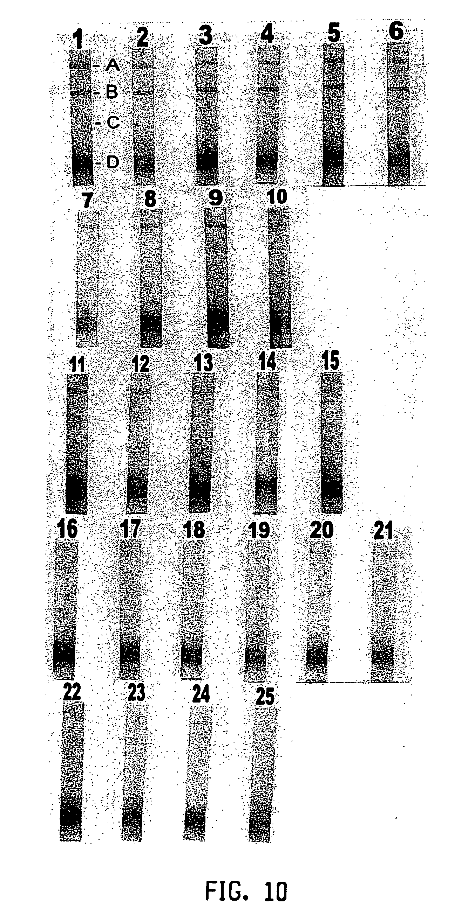

LGTm (A), LStm (B) and a *Campylobacter upsaliensis* 16S - 23S rRNA spacer (C - 5' CATTAAACTTTAGCAAGGAAGTG 3') SEQ ID NO: 228 oligonucleotide probe were irreversibly bound to nylon membrane strips and hybridised to with amplified *ssrA* PCR product, using the genus specific primers Ltm1 and Ltm2 ( Ltm1 was labelled with biotin at the 5' end), from *L. monocytogenes* (1-6), *L. innocua* (7-10), *L. ivanovii* (11), *L. murrayi* (12), *L. seeligeri* (13), *L. welshmeri* (14) and *L. grayii* (15). The *ssr*A amplified PCR products, using tmU5' and tmU3' (tmU5' was labelled with biotin at the 5' end), were also hybridised to the nylon membrane strips from the Gram-positive bacteria, *B. subtilus, L. lactis, S. aureus, S.* epidermis, E. faecalis, C. perfringins (16-21) and the Gram-negative bacteria E. coli, S. enteritidis, P. Rettgeri, K. aerogenes (22-25). As shown in Fig. 10 after hybridisation, development of the colorimetric assay to biotin revealed the following: Strips 1-6 demonstrates that the ssrA amplified PCR product originated from L. monocytogenes combined with the confirmation that the PCR product amplified is from the genus Listeria - A and B give colour detection; Strips 7 - 15 demonstrate that these PCR products originated from the genus Listeria - only A gives colour detection; and Strips 16 - 25 demonstrate that the PCR products are not from the genus Listeria - no colour detection. C is a negative oligonucleotide control probe and D is a positive control colorimetric detection assay for all samples.

### Example 7

### Use of ssrA / tmRNA sequences to distinguish between species of organisms.

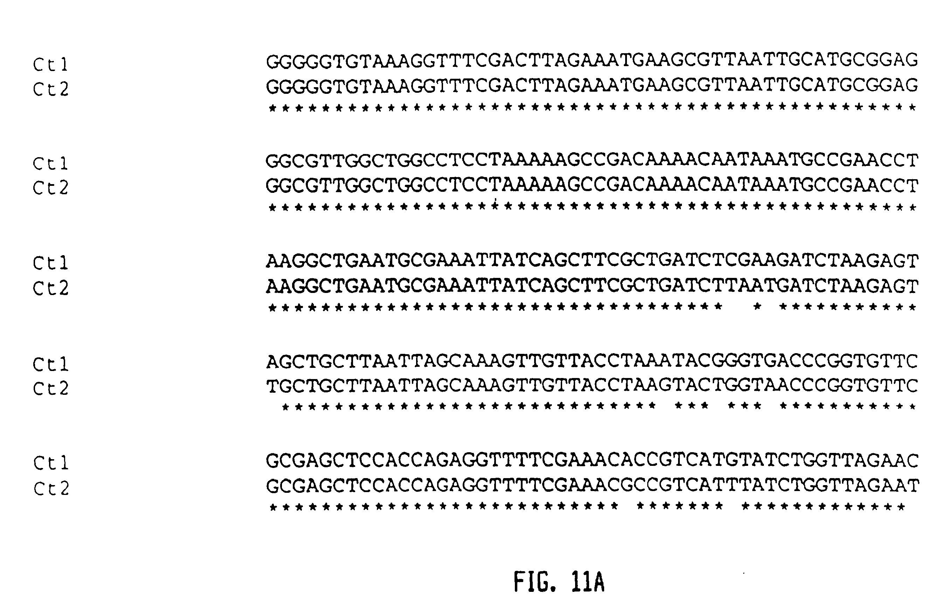

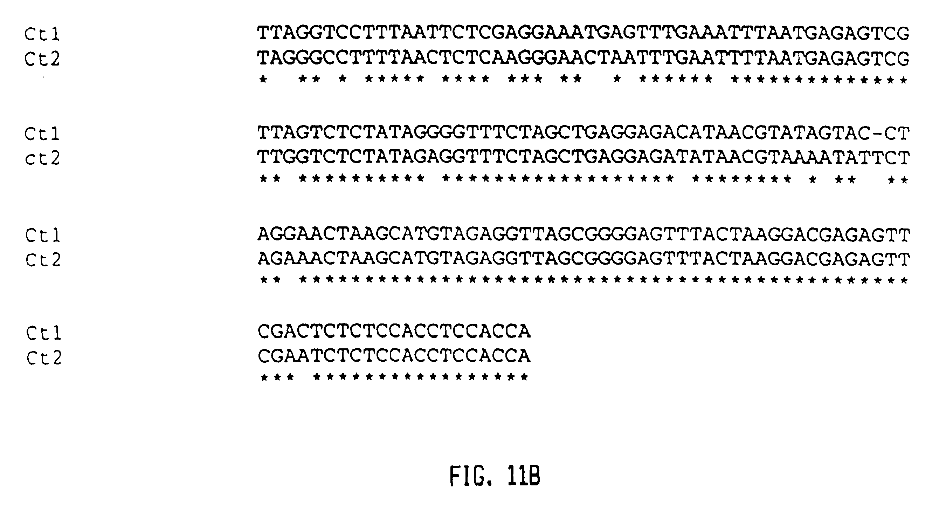

Clustal W alignments as shown in Figs. 11 (SEQ ID NOS. 19 and 21), 12 (SEQ ID NOS. 41 and 43), 13 (SEQ ID NOS. 77 and 79), 14 (SEQ ID NOS. 83 and 85), 15 and 16 (SEQ ID NO. 53, 55 and 57), indicate that there are nucleotide differences within the ssrA/tmRNA sequences of different strains of the same bacteria. This suggests that the ssrA/tmRNA sequences could potentially be used to discriminate between individual and/or groups of strains within a bacterial species. This may have useful applications in epidemiology and bacterial population analysis.

### Example 8

### tmRNA integrity analysis after medium and extreme heat treatment of bacterial cells.

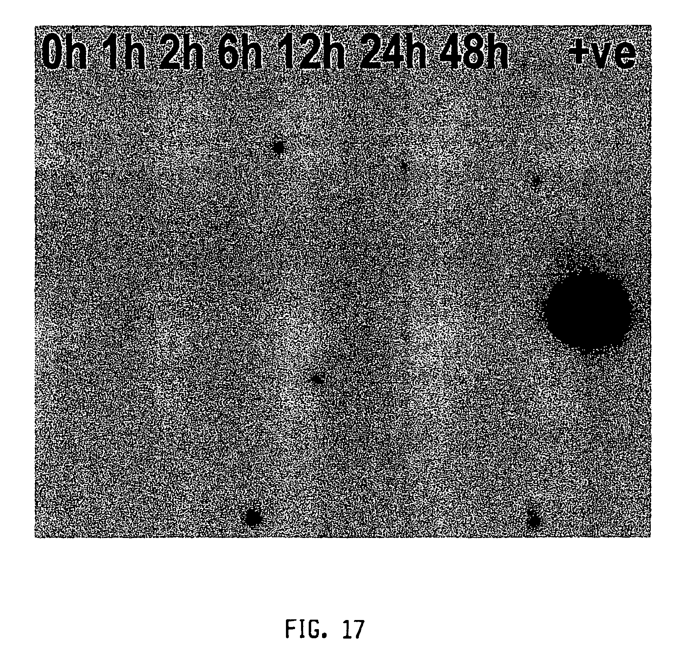

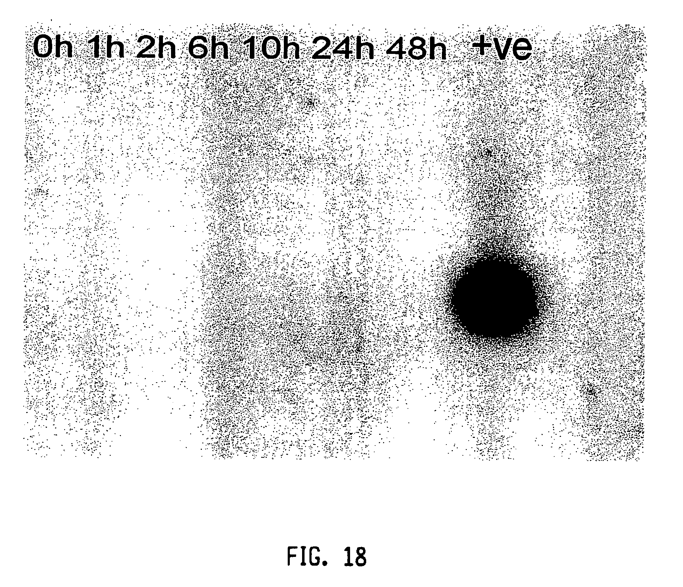

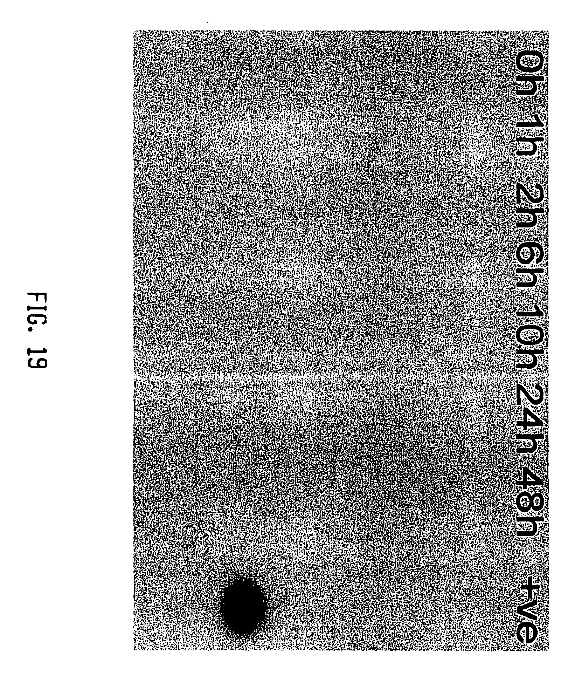

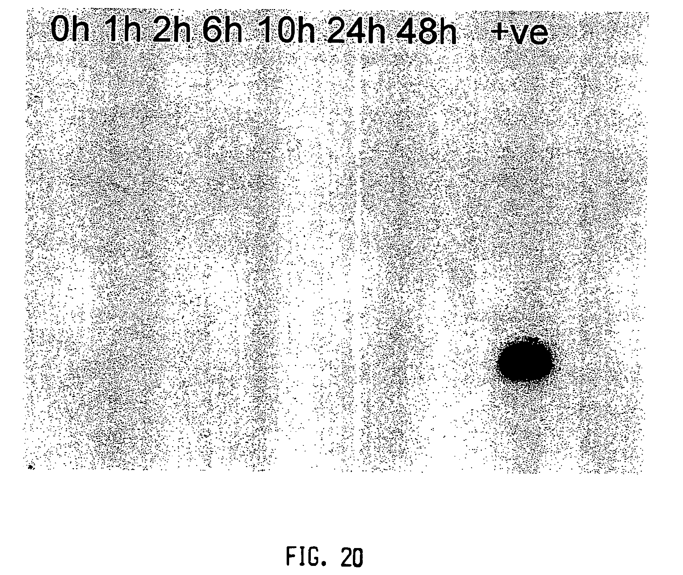

*E. coli* and *L. monocytogenes* cultures were heat treated at 80°C , for 20 min. in the case of *E*. *coli* and 40 min. in the case of *L. monocytogenes* and at 120°C for 15 min. (autoclaving) after overnight growth and tested for viability at 0h, 1h, 2h, 6h, 12h, 24h and 48h after heat treatment. No viability was-observed at each time period tested. Total RNA was also isolated at these time periods and electrophoresed on denaturing 1.2% agarose gels and Northern blotted. Each blot was hybridised to, in the case *of E. coli* (Figs. 17 and 18) with a radioactively labelled oligonucleotide probe Evtm and in the case of *L. monocytogenes* (Figs. 19 and 20) with a radiolabelled LVtm. No tmRNA transcript was detected with each sample tested, demonstrating that tmRNA transcript is degraded after heat treatment. The lanes represented with the notation +ve is a positive control total RNA sample.

### Example 9

### Use of the tmRNA transcript in distinguishing between viable and non-viable bacteria.

A 100 ml culture of *L. monocytogenes* was grown overnight in liquid culture. After growth, serial dilutions of the cells were carried out and viability was determined by spread plating on nutrient agar plates.
Simultaneously, total RNA was isolated from a 1 ml aliquot of these cells. The remainder of the cells were heated at 65°C for 20 min. Cells were then removed for both viability analysis and total RNA isolation. Samples were taken for viability and RNA isolation at time periods of 0 h, 2 h, 6 h and 24 h after treatment.

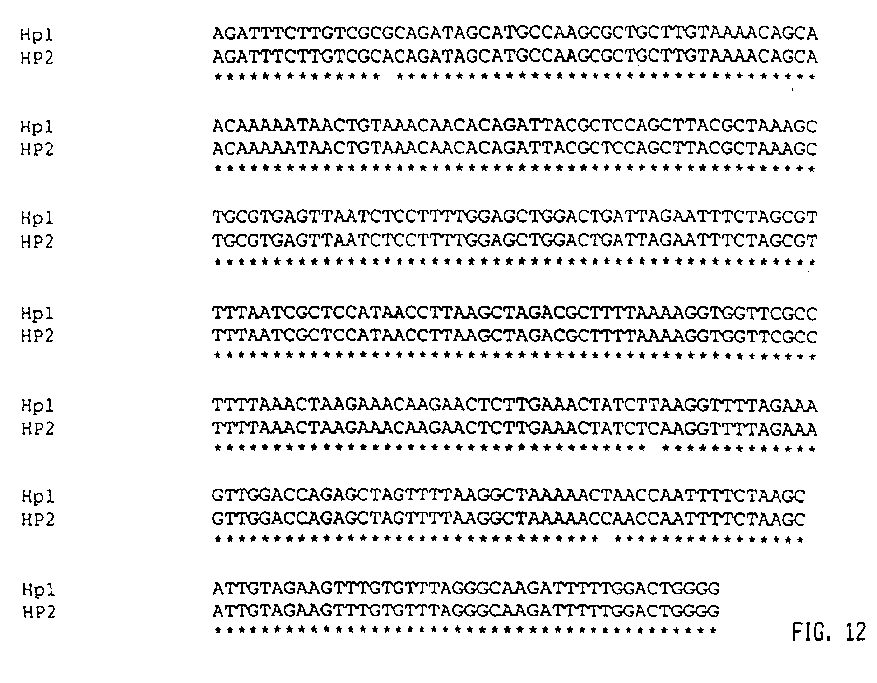

Spread plating on nutrient agar plates indicated that heat treatment killed *L. monocytogenes* cells, with no viable colony forming units observed. Each RNA sample isolated was then treated with DNase to remove any contaminating DNA and total RNA samples (100 ng) were subjected to Reverse Transcriptase-PCR amplification using the Listeria genus specific ssrA/tmRNA oligonucleotide primers Ltm1 and Ltm2. Negative control amplification reactions included primers, target, and Taq polymerase, but no Reverse Transcriptase. The results of the amplification reactions are shown in Fig. 12.

Amplified tmRNA RT-PCR products were only observed with the RNA sample which was not heat treated. All other samples gave no RT-PCR product indicating that the tmRNA molecules in these samples may have been degraded in the non-viable heat treated cells.

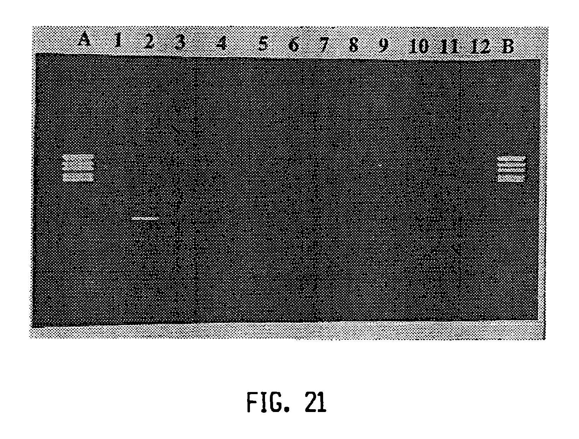

In Fig. 21 the lanes represent the following:
- Lane A:: Molecular weight marker V;
- Lane 1:: PCR amplification of RNA (no heat treatment of cells) - Reverse Transcriptase (RT), + Taq polymerase (TP);
- Lane 2:: RT-PCR of RNA (no heat treatment of cells), + RT, +TP;
- Lane 3:: PCR amplification of RNA (at 0 time after heat treatment), -RT, +TP;
- Lane 4:: RT-PCR of RNA (at 0 time after heat treatment), +RT, +TP;
- Lane 5:: PCR amplification of RNA (at 1 h time after heat treatment), -RT, +TP;
- Lane 6:: RT-PCR of RNA (at 1 h time after heat treatment), +RT, +TP;
- Lane 7:: PCR amplification of RNA (at 2 h time after heat treatment), -RT, +TP;
- Lane 8:: RT-PCR of RNA (at 2 h time after heat treatment), +RT, +TP;
- Lane 9:: PCR amplification of RNA (at 6 h time after heat treatment), -RT, +TP;
- Lane 10:: RT-PCR of RNA (at 6 h time after heat treatment), +RT, +TP;
- Lane 11:: PCR amplification of RNA (at 24 h time after heat treatment), -RT, +TP;
- Lane 12:: RT-PCR of RNA (at 24 h time after heat treatment), +RT, +TP;
- Lane B:: Molecular weight marker V.

## Claims

1. Use of the ssrA gene or a fragment thereof consisting of at least ten nucleotides as a target region in a nucleic acid probe assay for the detection and identification of Chlamydia trachomatis in vitro, wherein the sequence of the ssrA gene is selected from a group consisting of SEQ ID NO:19 and SEQ ID NO:21.

2. Use of tmRNA, an RNA transcript of the ssrA gene, or a fragment thereof consisting of at least ten nucleotides as a target region in a nucleic acid probe assay for the detection and identification of Chlamydia trachomatis, wherein the sequence of the tmRNA is selected from a group consisting of SEQ ID NO:20 and SEQ ID NO:22.

3. Use according to Claim 1 or 2, wherein a fragment of the ssrA gene molecule or a fragment of a tmRNA molecule, either corresponding to a region of high homology from the 5' end or a region of high homology from the 3' end of the DNA molecule is used as a universal target region.

4. Use according to Claim 1 or 2, wherein a fragment of the ssrA gene molecule or a fragment of a tmRNA molecule, either corresponding to a region of low homology is used as a target region in a nucleic acid probe assay to distinguish between species.

5. Use according to Claim 1 or 2, wherein a fragment of the ssrA gene molecule or a fragment of a tmRNA molecule, either corresponding to a region of low homology is used as a target region for the generation of a genus specific probe.

6. Use according to any preceding claim, wherein said ssrA gene fragment or said tmRNA fragment is used as the basis of a primer to be used in an amplification procedure.

7. Use according to Claim 6, wherein the product of the amplification procedure is used as a target region in a nucleic acid probe assay.

8. Use according to any one of Claims 2 to 7, wherein a cDNA transcript of a tmRNA molecule is used as a probe in a nucleic acid hybridisation assay.

9. Use according to any preceding claim, wherein the target region is used as the basis of an assay for distinguishing between living and dead prokaryotic or eukaryotic organisms or in a multiple probe format for broad scale detection and/or identification of prokaryotic or eukaryotic organisms.

10. Use according to Claim 9 wherein an ssrA gene probe or a tmRNA transcript probe is linked to a microarray gene chip system for the broad scale high throughput detection and identification of prokaryotic or eukaryotic organisms.

11. Use according to any preceding claim, wherein the target region is used as a probe in an assay to detect prokaryotic or eukaryotic organisms in a sample of matter.

12. Use according to any preceding claim, wherein a fragment of the ssrA gene or the tmRNA transcript is used in an assay to obtain a DNA profile of a prokaryotic or eukaryotic organism and, thereby, distinguish between strains of the same species.

13. Use according to any preceding claim, wherein the ssrA gene, the tmRNA transcript or a DNA sequence complementary thereto, or a fragment thereof, is used to design an agent directed against infectious prokaryotic or eukaryotic organisms for therapeutic purposes.

14. Use according to any preceding claim wherein the target region is used to monitor the efficacy of drug therapies against infectious agents or to monitor the viability and level of health-promoting organisms in the gastrointestinal tract.

15. Use according to any preceding claim, wherein the assay is used for the quantification of prokaryotic or eukaryotic organisms.

## Patentansprüche

1. Verwendung des ssrA-Gens oder eines Fragments davon, das aus wenigstens zehn Nucleotiden besteht, als Targetbereich in einem Nucleinsäurensondenassay zum Nachweis und zur Identifizierung von *Chlamydia trachomatis* in vitro, wobei die Sequenz des ssrA-Gens aus der Gruppe ausgewählt ist, die aus SEQ ID Nr. 19 und SEQ ID Nr. 21 besteht.

2. Verwendung von tmRNA, eines RNA-Transcripts des ssrA-Gens oder eines Fragments davon, das aus wenigstens zehn Nucleotiden besteht, als Targetbereich in einem Nucleinsäurensondenassay zum Nachweis und zur Identifizierung von *Chlamydia trachomatis,* wobei die Sequenz der tmRNA aus der Gruppe ausgewählt ist, die aus SEQ ID Nr. 20 und SEQ ID Nr. 22 besteht.

3. Verwendung gemäß Anspruch 1 oder 2, wobei ein Fragment des ssrA-Genmoleküls oder ein Fragment eines tmRNA-Moleküls, das entweder einem Bereich hoher Homologie ausgehend vom 5'-Ende oder einem Bereich hoher Homologie ausgehend vom 3'-Ende des DNA-Moleküls entspricht, als universeller Targetbereich verwendet wird.

4. Verwendung gemäß Anspruch 1 oder 2, wobei ein Fragment des ssrA-Genmoleküls oder ein Fragment eines tmRNA-Moleküls, die beide einem Bereich geringer Homologie entsprechen, als Targetbereich in einem Nucleinsäurensondenassay zur Unterscheidung zwischen Spezies verwendet wird.

5. Verwendung gemäß Anspruch 1 oder 2, wobei ein Fragment des ssrA-Genmoleküls oder ein Fragment eines tmRNA-Moleküls, die beide einem Bereich geringer Homologie entsprechen, als Targetbereich zur Erzeugung einer gattungsspezifischen Sonde verwendet wird.

6. Verwendung gemäß einem der vorstehenden Ansprüche, wobei das ssrA-Gen-Fragment oder das tmRNA-Fragment als Grundlage für einen Primer zur Verwendung in einem Amplifikationsverfahren verwendet wird.

7. Verwendung gemäß Anspruch 6, wobei das Produkt des Amplifikationsverfahrens als Targetbereich in einem Nucleinsäurensondenassay verwendet wird.

8. Verwendung gemäß einem der Ansprüche 2 bis 7, wobei ein cDNA-Transcript eines tmRNA-Moleküls als Sonde in einem Nucleinsäurehybridisierungsassay verwendet wird.

9. Verwendung gemäß einem der vorstehenden Ansprüche, wobei der Targetbereich als Grundlage eines Assays zur Unterscheidung zwischen lebenden und toten prokaryontischen oder eukaryontischen Organismen oder in einem multiplen Sondenformat zum Breitbandnachweis und/oder zur Identifizierung von prokaryontischen oder eukaryontischen Organismen verwendet wird.

10. Verwendung gemäß Anspruch 9, wobei eine ssrA-Gensonde oder eine tmRNA-Transcriptsonde mit einem Mikroarray-Gen-Chipsystem für den Breitband-Hochdurchsatz-Nachweis und die Identifizierung von prokaryontischen oder eukaryontischen Organismen verknüpft ist.

11. Verwendung gemäß einem der vorstehenden Ansprüche, wobei der Targetbereich als Sonde in einem Assay zum Nachweis von prokaryontischen oder eukaryontischen Organismen in einer Stoffprobe verwendet wird.

12. Verwendung gemäß einem der vorstehenden Ansprüche, wobei ein Fragment des ssrA-Gens oder des tmRNA-Transcripts in einem Assay verwendet wird, um ein DNA-Profil eines prokaryontischen oder eukaryontischen Organismus zu erhalten und **dadurch** zwischen Stämmen derselben Spezies zu unterscheiden.

13. Verwendung gemäß einem der vorstehenden Ansprüche, wobei das ssrA-Gen, das tmRNA-Transcript oder eine dazu komplementäre DNA-Sequenz oder ein Fragment davon verwendet wird, um ein Mittel zu entwerfen, das gegen infektiöse prokaryontische oder eukaryontische Organismen gerichtet ist, für therapeutische Zwecke.

14. Verwendung gemäß einem der vorstehenden Ansprüche, wobei der Targetbereich verwendet wird, um die Wirksamkeit von Wirkstofftherapien gegen infektiöse Agentien zu überwachen oder um die Lebensfähigkeit und die Konzentration von gesundheitsfördernden Organismen im Magen-Darm-Trakt zu überwachen.

15. Verwendung gemäß einem der vorstehenden Ansprüche, wobei der Assay zur Quantifizierung von prokaryontischen oder eukaryontischen Organismen verwendet wird.

## Revendications

1. Utilisation du gène ssrA ou d'un fragment de celui-ci consistant en au moins dix nucléotides en tant que région cible dans un dosage par sonde d'acide nucléique pour la détection et l'identification de Chlamydia trachomatis *in vitro,* dans laquelle la séquence du gène ssrA est choisie dans un groupe constitué par SEQ ID N° : 19 et SEQ ID N° : 21.

2. Utilisation d'ARNtm, d'un transcrit ARN du gène ssrA, ou d'un fragment de celui-ci consistant en au moins dix nucléotides en tant que région cible dans un dosage par sonde d'acide nucléique pour la détection et l'identification de Chlamydia trachomatis, dans laquelle la séquence de l'ARNtm est choisie dans un groupe constitué par SEQ ID N° : 20 et SEQ ID N° : 22.

3. Utilisation selon la revendication 1 ou 2, dans laquelle un fragment de la molécule du gène ssrA ou un fragment d'une molécule d'ARNtm, l'un ou l'autre correspondant à une région de forte homologie depuis l'extrémité 5' ou à une région de forte homologie depuis l'extrémité 3' de la molécule d'ADN, est utilisé en tant que région cible universelle.

4. Utilisation selon la revendication 1 ou 2, dans laquelle un fragment de la molécule du gène ssrA ou un fragment d'une molécule d'ARNtm, l'un ou l'autre correspondant à une région de faible homologie, est utilisé en tant que région cible dans un dosage par sonde d'acide nucléique pour différencier des espèces.

5. Utilisation selon la revendication 1 ou 2, dans laquelle un fragment de la molécule du gène ssrA ou un fragment d'une molécule d'ARNtm, l'un ou l'autre correspondant à une région de faible homologie, est utilisé en tant que région cible pour générer une sonde spécifique d'un genre.

6. Utilisation selon l'une quelconque des revendications précédentes, dans laquelle ledit fragment du gène ssrA ou ledit fragment d'ARNtm est utilisé en tant que base d'une amorce à utiliser dans un procédé d'amplification.

7. Utilisation selon la revendication 6, dans laquelle le produit obtenu par le procédé d'amplification est utilisé en tant que région cible dans un dosage par sonde d'acide nucléique.

8. Utilisation selon l'une quelconque des revendications 2 à 7, dans laquelle un transcrit ADNc d'une molécule d'ARNtm est utilisé en tant que sonde dans un essai d'hybridation d'acides nucléiques.

9. Utilisation selon l'une quelconque des revendications précédentes, dans laquelle la région cible est utilisée en tant que base d'un essai permettant de différencier des organismes procaryotes ou eucaryotes vivants et morts ou dans un format à sondes multiples pour une détection et/ou identification à grande échelle d'organismes procaryotes ou eucaryotes.

10. Utilisation selon la revendication 9, dans laquelle une sonde de gène ssrA ou une sonde de transcrit d'ARNtm est liée à une puce à ADN pour la détection et l'identification à grande échelle et à haut débit d'organismes procaryotes ou eucaryotes.

11. Utilisation selon l'une quelconque des revendications précédentes, dans laquelle la région cible est utilisée en tant que sonde dans un essai permettant de détecter des organismes procaryotes ou eucaryotes dans un échantillon de matière.

12. Utilisation selon l'une quelconque des revendications précédentes, dans laquelle un fragment du gène ssrA ou du transcrit d'ARNtm est utilisé dans un essai permettant d'obtenir le profil ADN d'un organisme procaryote ou eucaryote et, de cette façon, de différencier les souches de la même espèce.

13. Utilisation selon l'une quelconque des revendications précédentes, dans laquelle le gène ssrA, le transcrit d'ARNtm ou une séquence d'ADN complémentaire de celui-ci, ou un fragment de celui-ci, est utilisé pour concevoir un agent dirigé contre des organismes procaryotes ou eucaryotes infectieux à des fins thérapeutiques.

14. Utilisation selon l'une quelconque des revendications précédentes, dans laquelle la région cible est utilisée pour surveiller l'efficacité de traitements médicamenteux contre des agents infectieux ou pour surveiller la viabilité et le taux d'organismes bénéfiques pour la santé dans le tractus gastro-intestinal.

15. Utilisation selon l'une quelconque des revendications précédentes, dans laquelle l'essai est utilisé pour la quantification d'organismes procaryotes ou eucaryotes.
